(19) Europäisches Patentamt
European Patent Office
Office européen des brevets

(11) Publication number: 0 286 145
A2

# (12) EUROPEAN PATENT APPLICATION

(21) Application number: 88105733.5

(22) Date of filing: 11.04.88

(51) Int. Cl.4: C07D 501/36 , A61K 31/545

Claims for the following Contracting States: ES + GR.

(30) Priority: 10.04.87 CH 1407/87
30.04.87 CH 1657/87
26.06.87 CH 2413/87

(43) Date of publication of application:
12.10.88 Bulletin 88/41

(84) Designated Contracting States:
AT BE CH DE ES FR GB GR IT LI LU NL SE

(71) Applicant: SANKEI PHARMACEUTICAL COMPANY LIMITED
2, Kanda-Mikura-cho
Chiyoda-Ku Tokyo(JP)

Applicant: NIPPON PHARMACEUTICAL DEVELOPMENT INSTITUTE COMPANY LIMITED
3-25, Hanazono-cho Mukawa-machi
Yuufutsu-gun Hokkaido(JP)

(72) Inventor: Furlenmeier, André, Dr.
119 Wettsteinallee
CH-4058 Basle(CH)
Inventor: Götschi, Erwin, Dr.
23 Landhofweg
CH-4153 Reinach(CH)
Inventor: Hebeisen, Paul, Dr.
52 Klusweg
CH-4153 Reinach(CH)
Inventor: Hofheinz, Werner, Dr.
7 Talmattweg
CH-4103 Bottmingen(CH)
Inventor: Link, Helmut, Dr.
70 Dammerkirchstrasse
CH-4056 Basle(CH)

(74) Representative: Lederer, Franz, Dr. et al
Patentanwält Dr. Franz Lederer Lucile Grahnstrasse 22
D-8000 München 80(DE)

## (54) 3-Heterocyclylthiomethyl cephalosporins.

(57) Acyl derivatives of the general formula

EP 0 286 145 A2

Xerox Copy Centre

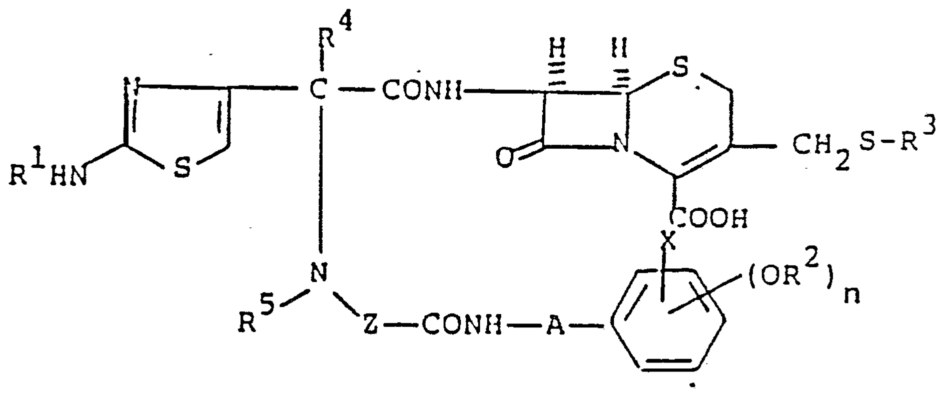

I

in which A signifies a group of the general formulae

-NHCO-, -NHCONHCO-, -NHCOCH = CH-,

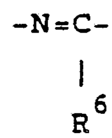

,

wherein $R^6$ represents hydrogen or lower alkyl; $R^1$ and $R^2$ in each case signify hydrogen or a protecting group, X signifies hydrogen, halogen, lower alkoxy. nitro or an additional group $-OR^2$, n signifies the number 1 or 2, $R^4$ and $R^5$ both signify hydrogen or together signify an additional bond and Z signifies a direct bond or carbonyl (where $R^4$ and $R^5$ both represent hydrogen) or a group of the formula -O-B-(where $R^4$ and $R^5$ together represent an additional bond) in which B signifies straight-chain, branched or cyclic lower alkylene; and wherein further $R^3$ signifies a substituted bicyclic group of the general formulae

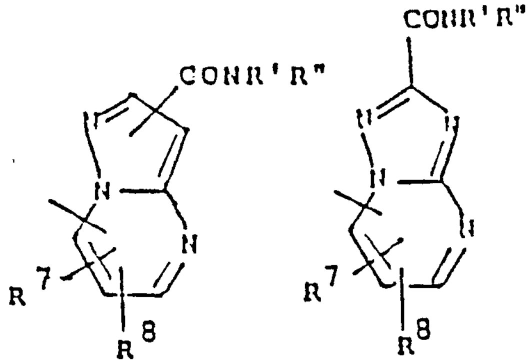

(a) (b)

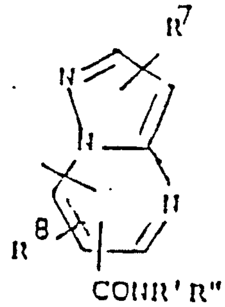

(c)

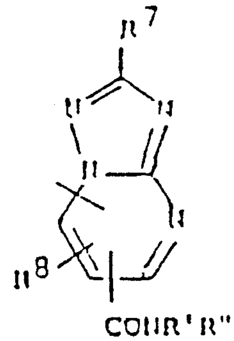

(d)

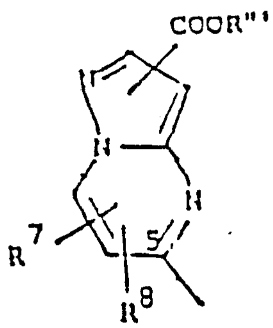

(e)

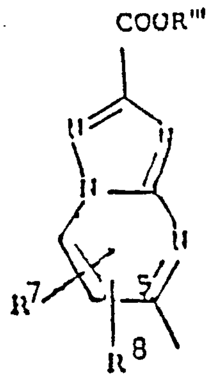

(f)

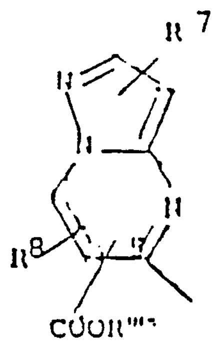

(g)

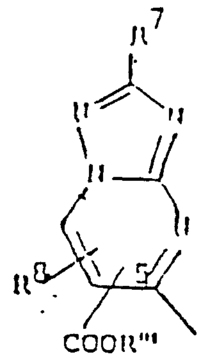

(h)

in which $R^7$ and $R^8$ each individually represent hydrogen, lower alkyl or trifluoromethyl or (in formulae (a), (b), (e) and (f)) together represent alkylene with 3 or 4 carbon atoms and R′, R″ and R‴ each individually represent hydrogen, lower alkyl or lower cycloalkyl; whereby compounds of formula I in which $R^4$ and $R^5$ together represent an additional bond are present in the syn-isomeric form or as a mixture with the anti-isomeric form in which the syn-isomeric form predominates,

as well as readily hydrolyzable esters and salts of these compounds and hydrates of the compounds of formula I or of their esters and salts.
The compounds have antibiotic activity.

## 3-Heterocyclylthiomethyl Cephalosporins

The present invention is concerned with novel acyl derivatives namely cephalosporin derivatives of the general formula

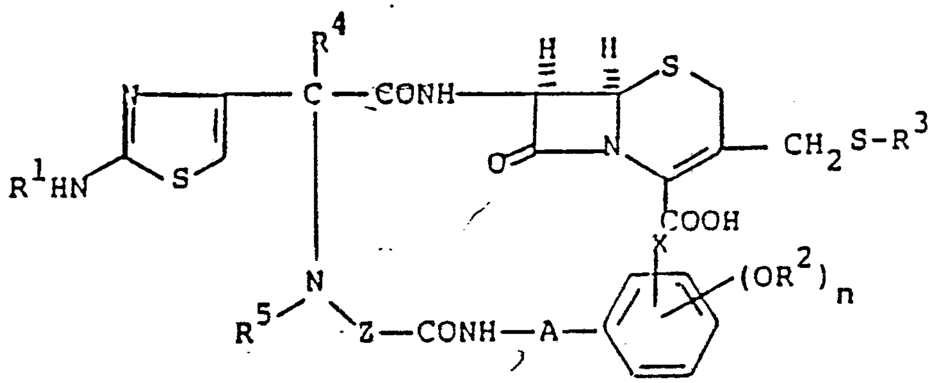

I

in which A signifies a group of the general formulae

-NHCO-, -NHCONHCO-, -NHCOCH = CH-,

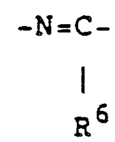

wherein $R^6$ represents hydrogen or lower alkyl; $R^1$ and $R^2$ in each case signify hydrogen or a protecting group, X signifies hydrogen, halogen, lower alkoxy nitro or an additional group $-OR^2$, n signifies the number 1 or 2, $R^4$ and $R^5$ both signify hydrogen or together signify an additional bond and Z signifies a direct bond or carbonyl (where $R^4$ and $R^5$ both represent hydrogen) or a group of the formula -O-B-(where $R^4$ and $R^5$ together represent an additional bond) in which B signifies straight-chain, branched or cyclic lower alkylene; and wherein further $R^3$ signifies a substituted bicyclic group of the general formulae

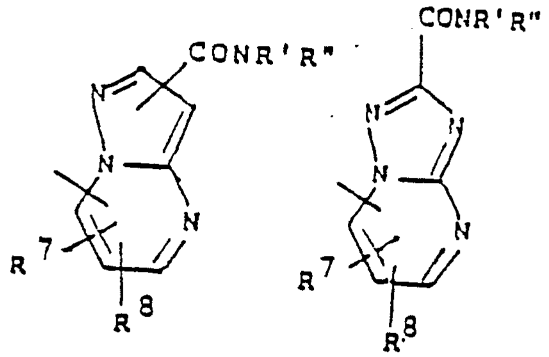

(a) (b)

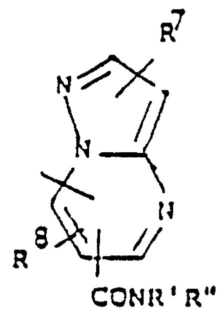

(c)

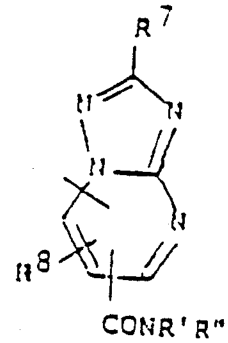

(d)

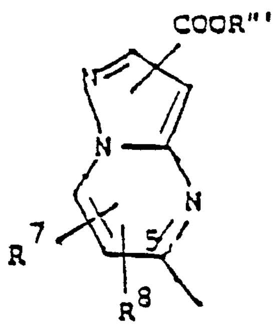

(e)

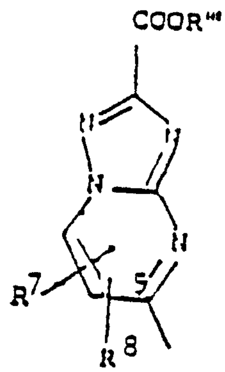

(f)

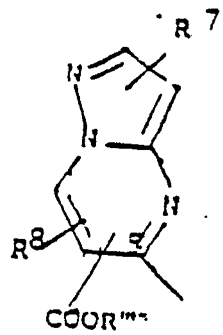

(g)

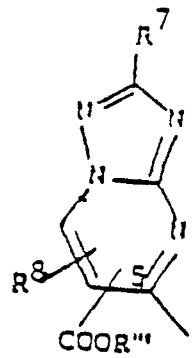

(h)

in which $R^7$ and $R^8$ each individually represent hydrogen, lower alkyl or trifluoromethyl or (in formulae (a), (b), (e) and (f)) together represent alkylene with 3 or 4 carbon atoms and R', R" and R"' each individually represent hydrogen, lower alkyl or lower cycloalkyl; whereby compounds of formula I in which $R^4$ and $R^5$ together represent an additional bond are present in the syn-isomeric form or as a mixture with the anti-isomeric form in which the syn-isomeric form predominates,

as well as readily hydrolyzable esters and salts of these compounds and hydrates of the compounds of formula I or of their esters and salts.

The group A is preferably -NHCO-; Z is preferably the group -O-B-; $R^1$ and $R^2$ are preferably hydrogen; X is preferably hydrogen or chlorine; n is preferably 2. Accordingly, a preferred sub-group of compounds in accordance with the invention comprises those of the general formula

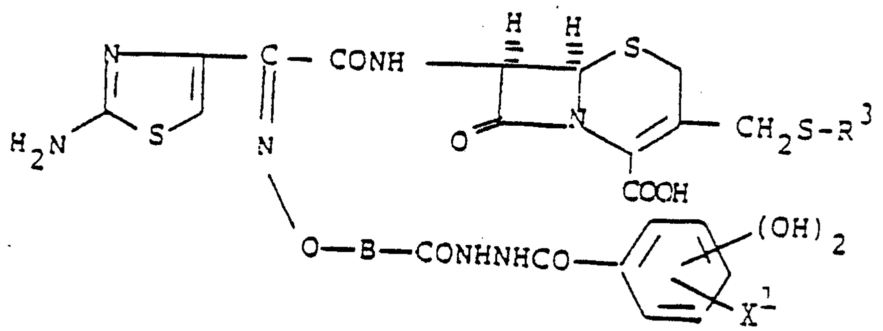

Ia

in which B has the above significance, and $X^1$ represents hydrogen or chlorine,

as well as readily hydrolyzable esters and salts of these compounds and hydrates of the compounds of formula Ia or of their esters and salts.

B is preferably the group $-C(CH_3)_2-$or $-CH_2-$. The two groups $-(OH)_2$ are preferably present in the 3,4-or 2,3-position.

Compounds of formula I with an oxyimino substituent ($R^4$ and $R^5$ together an additional bond) and the compounds of formula Ia are present in the syn-isomeric form or as mixture with the anti-isomeric form in which the syn-isomeric form predominates.

"Lower alkyl" and "lower alkenyl" signify straight-chain and branched carbon chains with 1-7, especially 1-4, carbon atoms, e.g. methyl, ethyl, n-propyl, isopropyl, t-butyl, vinyl, allyl, 2-butenyl or the like. Modified groups such as lower alkoxy (lower alkyl-O), lower alkanoyl (lower alkyl-CO-), lower alkoxycarbonyl lower alkyl-OCO-) and lower alkanoyloxy (lower alkyl--COO-) have an analogous significance, also in combinations such as lower alkanoyloxyalkyl. Lower cycloalkyl and lower cycloalkenyl have 3-7 carbon atoms such as e.g. cyclopropyl, cyclobutyl, cyclohexyl, methyl-cyclopropyl, cyclohexenyl. "Lower alkylene" is to be understood analogously to "lower alkyl" and has 1-7, especially 1-4, carbon atoms, e.g. "straight-chain alkylene" such as $-CH_2-$. $-CH_2-CH_2-$; "branched lower alkylene" has 2-7, especially 2-4, carbon atoms, e.g.

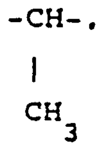 . 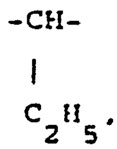 . 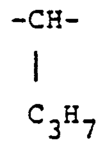 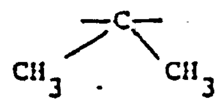 . 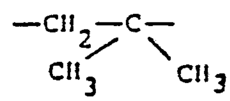 ;

"cyclic lower alkylene" has 3-7 carbon atoms, e.g.

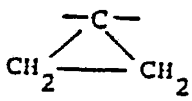 , 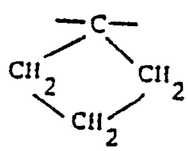 , 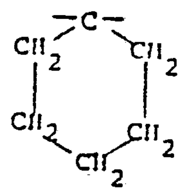 , 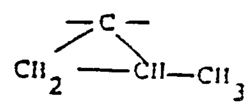

"Halogen" represents chlorine, fluorine, bromine and iodine, especially chlorine.

protecting groups $R^1$ are those which are usually found in cephalosporin chemistry, e.g. formyl, trityl, t-butoxycarbonyl, trifluoroacetyl, chloroacetyl, bromoacetyl or iodoacetyl. Protecting groups $R^2$ are likewise of the usual type, e.g. lower alkanoyl such as acetyl, lower alkoxycarbonyl such as methoxycarbonyl t-butoxycarbonyl; trimethylsilyl or tetrahydropyranyl.

The bicyclic residues (a)-(d) are preferably linked via position 5:

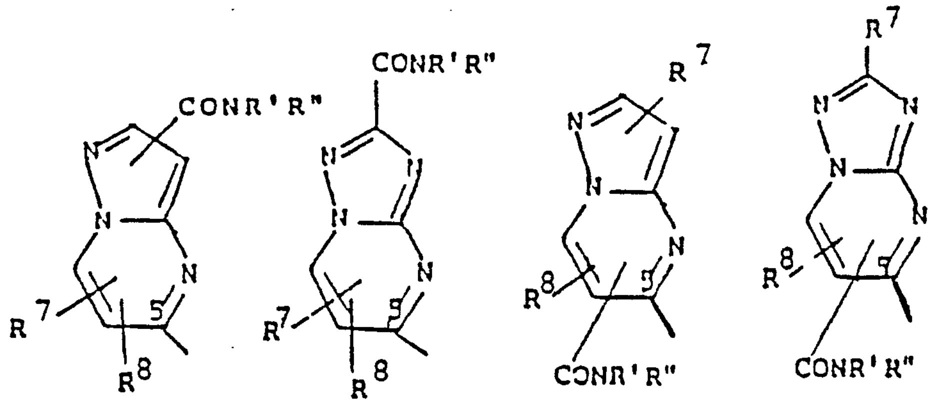

(a$^1$) (b$^1$) (c$^1$) (d$^1$)

or, especially, via position 7:

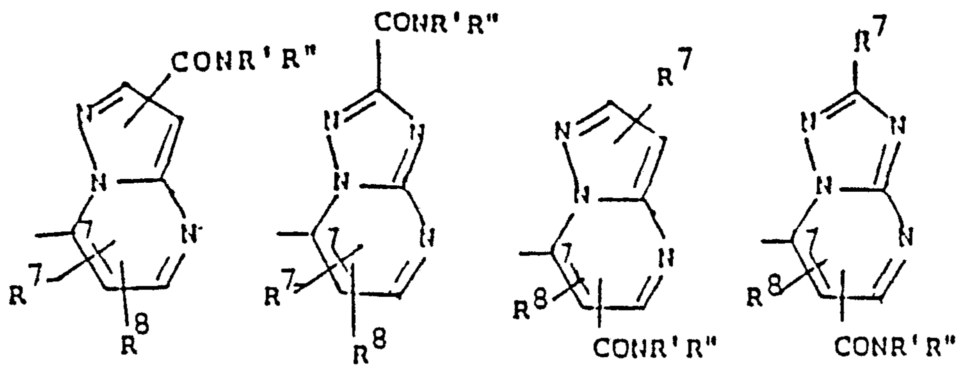

(a$^2$) (b$^2$) (c$^2$) (d$^2$)

The substituent -CONR′R″ of group (a) and the substituent -COOR‴ of group (e) are preferably situated in position 3:

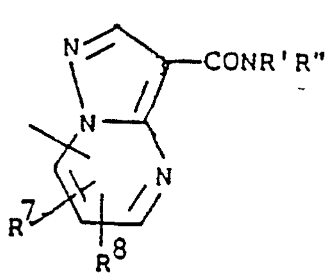

(a$^3$)

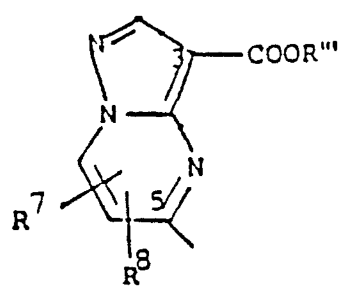

(e$^3$)

A preferred sub-group of compounds in accordance with the invention comprises those in which $R^3$ signifies a substituted bicyclic group of the general formulae

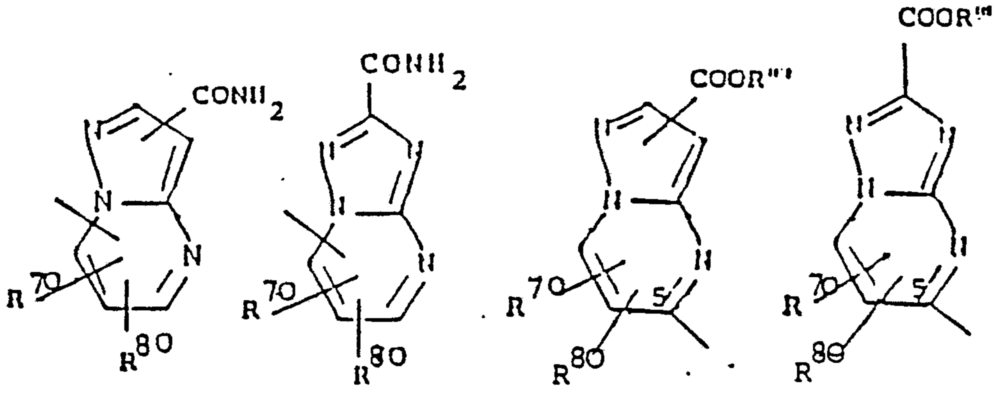

(a⁴) (b⁴) (e⁴) (f⁴)

in which $R^{70}$ and $R^{80}$ each individually represent hydrogen, methyl or trifluoromethyl or together represent alkylene with 3 or 4 carbon atoms and R‴ signifies hydrogen or methyl.

preferred groups $R^3$ are

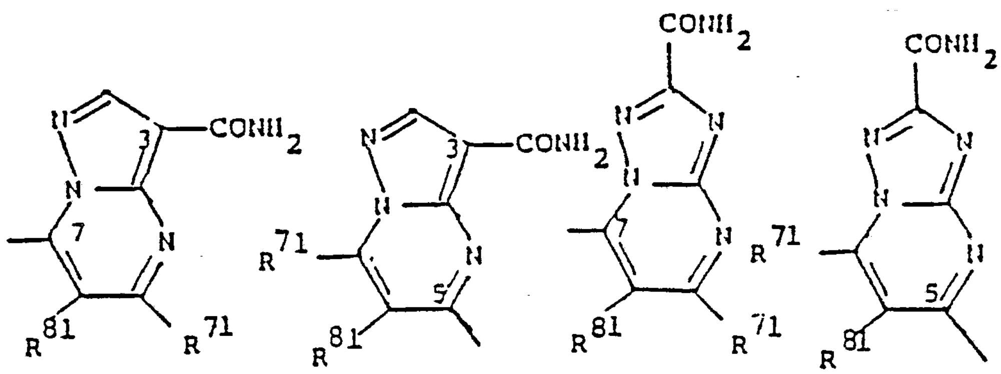

(a⁵) (a⁶) (b⁵) (b⁶)

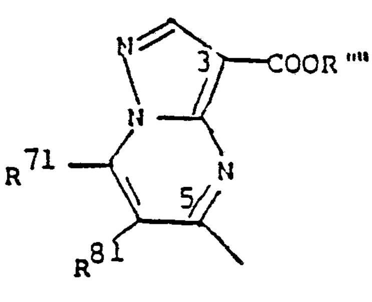

(e⁵)

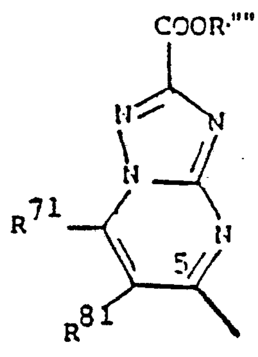

(f⁵)

wherein $R^{71}$ represents methyl or trifluoromethyl and $R^{81}$ represents hydrogen or $R^{71}$ and $R^{81}$ together represent tetramethylene and R⁗ signifies hydrogen or methyl.

Especially preferred groups $R^3$ are

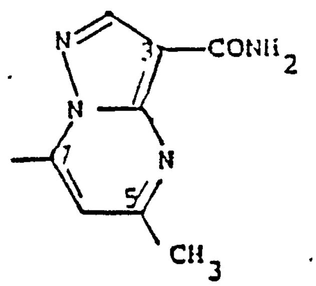 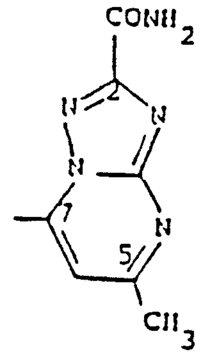

(a[7]) (b[7])

Salts of the compounds of formula I are pharmaceutically compatible salts and can be salts with bases or with acids. Salts with bases are e.g. alkali metal salts such as the sodium and potassium salt; the ammonium salt; alkaline earth metal salts such as the calcium salt; salts with organic bases such as salts with amines, e.g. salts with diisopropylamine, benzylamine, dibenzylamine, triethanolamine, triethylamine, N,N-dibenzylethylenediamine, N-methylmorpholine pyridine, piperazine, N-ethyl-piperidine, procaine. The compounds of formula I also form addition salts with organic or inorganic acids. Examples of such salts are hydrohalides, for example hydrochlorides, hydrobromides, hydroiodides, as well as other mineral acid salts such as sulphates, nitrates, phosphates and the like, alkyl-and mono-arylsulphonates such as ethanesulphonates, toluenesulphonates, benzenesulphonates and the like and also other organic acid salts such as acetates, tartrates, maleates, citrates, benzoates. salicylates, ascorbates and the like.

As readily hydrolyzable esters of the compounds of formula I there are to be understood compounds of formula I whose carboxy group is present in the form of a readily hydrolyzable ester group. Examples of such esters, which can be of the conventional type, are the lower alkanoyloxyalkyl esters, e.g. the acetoxymethyl, pivaloyloxymethyl, 1-acetoxyethyl and 1-pivaloyloxyethyl ester; the lower alkoxycarbonyloxyalkyl esters, e.g. the methoxycarbonyloxymethyl, 1-ethoxycarbonyloxyethyl and 1-isopropoxycarbonyloxyethyl ester; the lactonyl esters, e.g. the phthalidyl and thiophthalidyl ester; the lower alkoxymethyl esters, e.g. the methoxymethyl ester; and the lower alkanoylaminomethyl esters, e.g. the acetamidomethyl ester. Other esters, e.g. the benzyl and cyanomethyl ester, can also be used. Additional carboxy groups which may be present in a compound of formula I can also form the above readily hydrolyzable esters.

The compounds of formula I (including their salts and readily hydrolyzable esters) can be hydrated. The hydration can take place in the course of the manufacturing process or can occur gradually as a consequence of hygroscopic properties of an initially anhydrous product.

Especially preferred products are:

(6R,7R)-7-[(Z)-2-(2-Amino-4-thiazolyl)-2-[[1-[3-(3,4-dihydrobenzoyl)carbazoyl]-1-methylethoxy]imino]-acetamido]-3-[[(2-carbamoyl-5-methyl-s-triazolo[1,5-a]pyrimidin-7-yl)thio]methyl]-8-oxo-5-thia-1-azabicyclo-[4.2.0]oct-2-ene-2-carboxylic acid,

(6R,7R)-7-[(Z)-2-(2-Amino-4-thiazolyl)-2-[[1-[3-(3,4-dihydroxybenzoyl)carbazoyl]-1-methylethoxy]imino]-acetamido]-3-[[(3-carbamoyl-5-methylpyrazolo[1,5-a]pyrimidin-7-yl)thio]methyl]-8-oxo-5-thia-1-azabicyclo-[4.2.0]oct-2-ene-2-carboxylic acid,

(6R,7R)-7-[(Z)-2-(2-Amino-4-thiazolyl)-2-[[1-[3-(3,4-dihydroxybenzoyl)carbazoyl]-1-methylethoxy]imino]-acetamido]-3-[[(2-carbamoyl-6,7-dihydro-5H-cyclopenta[d]-s-triazolo[1,5-a]pyrimidin-8-yl)thio]methyl]-8-oxo-5-thia-1-azabicyclo[4.2.0]oct-2-ene-2-carboxylic acid,

(6R,7R)-7-[(Z)-2-(2-Amino-4-thiazolyl)-2-[[1-[3-(3,4-dihydroxybenzoyl)carbazoyl]-1-methylethoxy]]imino]-acetamido]-3-[[(2-carbamoyl-7-methyl-s-triazolo[1,5-a]pyrimidin-5-yl)thio]methyl]-8-oxo-5-thia-1-azabicyclo-[4.2.0]oct-2-ene-2-carboxylic acid,

(6R,7R)-7-[(Z)-2-(2-Amino-4-thiazolyl)-2-[[1-[3-(3,4-dihydroxybenzoyl)carbazoyl]-1-methylethoxy]imino]-acetamido]-3-[[[3-carbamoyl-7-(trifluoromethyl)pyrazolo[1,5-a]pyrimidin-5-yl]thio]methyl]-8-oxo-5-thia-1-azabicyclo[4.2.0]oct-2-ene-2-carboxylic acid,

(6R,7R)-7-[(Z)-2-(2-Amino-4-thiazolyl)-2-[[1-[3-(3,4-dihydroxybenzoyl)carbazoyl]-1-methylethoxy]imino]-acetamido]-3-[[[3-carbamoyl-5-(trifluoromethyl)pyrazolo[1,5-a]pyrimidin-7-yl]thio]methyl]-8-oxo-5-thia-1-azabicyclo[4.2.0]oct-2-ene-2-carboxylic acid,

(6R,7R)-7-[(Z)-2-(2-Amino-4-thiazolyl)-2-[[1-[3-(3-chloro-4,5-dihydroxybenzoyl)carbazoyl]-1-methylethoxy]-imino]acetamido]-3-[[(2-carbamoyl-5-methyl-s-triazolo[1,5-a]pyrimidin-7-yl)thio]methyl]-8-oxo-5-thia-1-azabicyclo[4.2.0]oct-2-ene-2-carboxylic acid, as well as their salts.

Other interesting products are:

(6R,7R)-7-[(Z)-2-(2-amino-4-thiazolyl)-2-[[1-[3-(3,4-dihydroxybenzoyl)carbazoyl]-1-methylethoxy]imino]-acetamido]-3-[[(2-carboxy-7-methyl-s-triazolo[1,5-a]pyrimidin-5-yl)thio]-methyl]-8-oxo-5-thia-1-azabicyclo-[4.2.0]oct-2-ene-2-carboxylic acid,

(6R,7R)-7-[(Z)-2-(2-amino-4-thiazolyl)-2-[[1-[3-(3,4-dihydroxybenzoyl)carbazoyl]-1-methylethoxy]imino]-acetamido]-3-[[(2-methoxycarbonyl-7-methyl-s-triazolo[1,5-a]pyrimidin-5-yl)thio]-methyl]-8-oxo-5-thia-1-azabicyclo[4.2.0]oct-2-ene-2-carboxylic acid,

(6R,7R)-7-[(Z)-2-(2-amino-4-thiazolyl)-2-[[1-[3-(2,3-dihydroxybenzoyl)carbazoyl]-1-methylethoxy]imino]-acetamido]-3-[[(2-carbamoyl-5-methyl-s-triazolo[1,5-a]pyrimidin-7-yl)-thio]methyl]-8-oxo-5-thia-1-azabicyclo-[4.2.0]oct-2-ene-2-carboxylic acid,

(6R,7R)-7-[(Z)-2-(2-amino-4-thiazolyl)-2-[[1-[3-(3,4-dihydroxybenzoyl)carbazoyl]-1-methylethoxy]imino]-acetamido]-3-[[[2-(cyclopropylcarbamoyl)-7-methyl-s-triazolo[1,5-a]pyrimidin-5-yl]thio]methyl]-8-oxo-5-thia-1-azabicyclo[4.2.0]oct-2-ene-2-carboxylic acid,

(6R,7R)-7-[(Z)-2-(2-amino-4-thiazolyl)-2-[[1-[3-(3,4-dihydroxybenzoyl)carbazoyl]-1-methylethoxy]imino]-acetamido]-3-[[[3-(methoxycarbonyl)-7-methylpyrazolo[1,5-a]pyrimidin-5-yl]thio]methyl]-8-oxo-5-thia-1-azabicyclo[4.2.0]oct-2-ene-2-carboxylic acid,

(6R,7R)-7-[[[(Z)-2-(2-amino-4-thiazolyl)-2-[1-[3-(3,4-dihydroxybenzoyl)carbazoyl]-1-methylethoxy]imino]-acetamido]-3-[[[3-carboxy-7-(trifluoromethyl)pyrazolo[1,5-a]pyrimidin-5-yl]thio]methyl]-8-oxo-5-thia-1-azabicyclo[4.2.0]oct-2-ene-2-carboylic acid,

(6R,7R)-7-[(Z)-2-(2-amino-4-thiazolyl)-2-[[1-[3-(3,4-dihydroxybenzoyl)carbazoyl]-1-methylethoxy]imino]-acetamido]-3-[[[5-methyl-2-(methylcarbamoyl)-s-triazolo[1,5-a]pyrimidin-7-yl]thio]methyl]-8-oxo-5-thia-1-azabicyclo[4.2.0]oct-2-ene-2-carboxylic acid,

(6R,7R)-7-[(Z)-2-(2-amino-4-thiazolyl)-2-[[1-[3-(3,4-dihydroxybenzoyl)carbazoyl]-1-methylethoxy]imino]-acetamido]-3-[[[2-(dimethylcarbamoyl)-5-methyl-s-triazolo[1,5-a]-pyrimidin-7-yl]thio]methyl]-8-oxo-5-thia-1-azabicyclo[4.2.0]-oct-2-ene-2-carboxylic acid,

(6R,7R)-7-[(Z)-2-(2-amino-4-thiazolyl)-2-[[1-[3-(3,4-dihydroxybenzoyl)carbazoyl]-1-methylethoxy]iomino]-acetamido]-3-[[[2-carbamoyl-5-(trifluoromethyl)-s-triazolo[1,5-a]pyrimidin-7-yl]thio]methyl]-8-oxo-5-thia-1-azabicyclo[4.2.0]oct-2-ene-2-carboxylic acid,

(6R,7R)-7-[(Z)-2-(2-amino-4-thiazolyl)-2-[[1-[3-(3,4-dihydroxybenzoyl)carbazoyl]-1-methylethoxy]imino]-acetamido]-3-[[[2-carbamoyl-7-(trifluoromethyl)-s-triazolo[1,5-a]pyrimidin-5-yl]thio]methyl]-8-oxo-5-thia-1-azabicyclo[4.2.0]oct-2-ene-2-carboxylic acid,

(6R,7R)-7-[(Z)-2-(2-amino-4-thiazolyl)-2-[[1-[3-(3,4-dihdyroxybenzoyl)carbazoyl]-1-methylethoxy]imino]-acetamido]-3-[[[2-carboxy-7,8-dihydro-6H-cyclopenta[e][1,2,4]triazolo[1,5-a]pyrimidin-5-yl]thio]methyl]-8-oxo-5-thia-1-azabicyclo[4.2.0]oct-2-ene-2-carboxylic acid,

(6R,7R)-7-[(Z)-2-(2-amino-4-thiazolyl)-2-[[1-[3-(3,4-dihydroxybenzoyl)carbazoyl]-1-methylethoxy]imino]-acetamido]-3-[[[2-carbamoyl-7,8-dihydro-6H-cyclopenta[e][1,2,4]triazolo[1,5-a]pyrimidin-5-yl]thio]methyl]-8-oxo-5-thia-1-azabicyclo[4.2.0]oct-2-ene-2-carboxylic acid,

(6R,7R)-7-[(Z)-2-(2-amino-4-thiazolyl)-2-[[1-[3-(3,4-dihydroxybenzoyl)carbazoyl]-1-methylethoxy]imino]-acetamido]-3-[[[2-carboxy-7-(trifluoromethyl)-s-triazolo[1,5-a]pyrimidin-5-yl]thio]methyl]-8-oxo-5-thia-1-azabicyclo[4.2.0]oct-2-ene-2-carboxylic acid,

(6R,7R)-7-[(Z)-2-(2-amino-4-thiazolyl)-2-[[1-[3-(3,4-diacetoxybenzoyl)carbazoyl]-1-methylethoxy]imino]-acetamido]-3-[[(3-carbamoyl-6,7-dihydro-5H-cyclopenta[d]pyrazolo[1,5-a]pyrimidin-8-yl)thio]methyl]-8-oxo-5-thia-1-azabicyclo[4.2.0]oct-2-ene-carboxylic acid,

(6R,7R)-7-[(Z)-2-(2-amino-4-thiazolyl)-2-[[1-[3-(3,4--dihydroxybenzoyl)carbazoyl]-1-methylethoxy]imino]-acetamido]-3-[[(3-carbamoyl-6,7-dihdyro-5H-cyclopenta[d]pyrazolo[1,5-a]pyrimidin-8-yl)thio]methyl]-8-oxo-5-thia-1-azabicyclo[4.2.0]oct-2-ene-2-carboxylic acid,

(6R,7R)-7-[(Z)-2-(2-amino-4-thiazolyl)-2-[[1-[3-(3,4-diacetoxybenzoyl)carbazoyl]-1-methylethoxy]imino]-acetamido]-3-[[(3-carbamoyl-5-methyl-pyrazolo[1,5-a]pyrimidin-7-yl)-thio]methyl]-8-oxo-5-thia-1-azabicyclo-[4.2.0]oct-2-ene-2-carboxylic acid,

(6R,7R)-7-[(Z)-2-(2-amino-4-thiazolyl)-2-[[1-[3-(3,4-diacetoxybenzoyl)carbazoyl]-1-methylethoxy]imino]-acetamido]-3-[[[3-(dimethylcarbamoyl)-5-methyl-pyrazolo[1,5-a]-pyrimidin-7-yl]thio]methyl]-8-oxo-5-thia-1-azabicyclo[4.2.0]-oct-2-ene-2-carboxylic acid,

(6R,7R)-7-[(Z)-2-(2-amino-4-thiazolyl)-2-[[1-[3-(3,4-dihydroxybenzoyl)carbazoyl]-1-methylethoxy]imino]-acetamido]-3-[[[3-(dimethylcarbamoyl)-5-methyl-pyrazolo[1,5-a]-pyrimidin-7-yl]thio]methyl]-8-oxo-5-thia-1-azabicyclo[4.2.0]-oct-2-ene-2-carboxylic acid as well as their salts.

The above acyl derivatives are manufactured in accordance with the invention by

(a) reacting a compound of the general formula

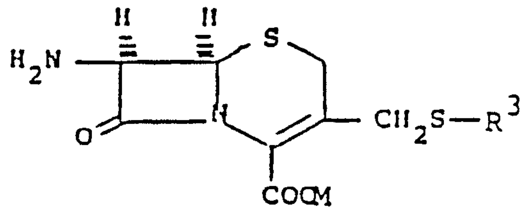

III

in which $R^3$ has the significance given above and M represents hydrogen or an ester protecting group,

with a carboxylic acid of the general formula

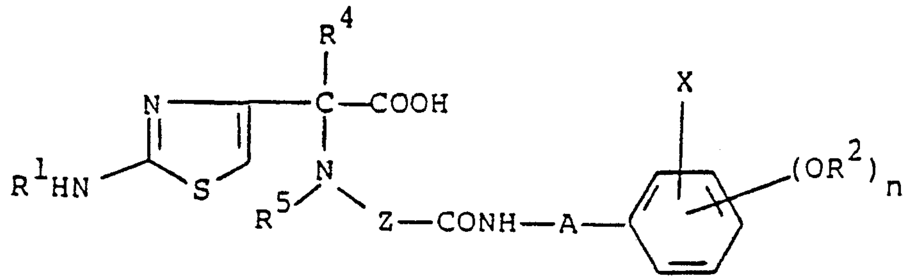

IV

in which $R^1$, $R^2$, $R^4$, $R^5$, X, Z, A and n have the significance given above,

or one of its reactive derivatives and, if desired, cleaving off any protecting groups, or

(b) reacting a compound of the general formula

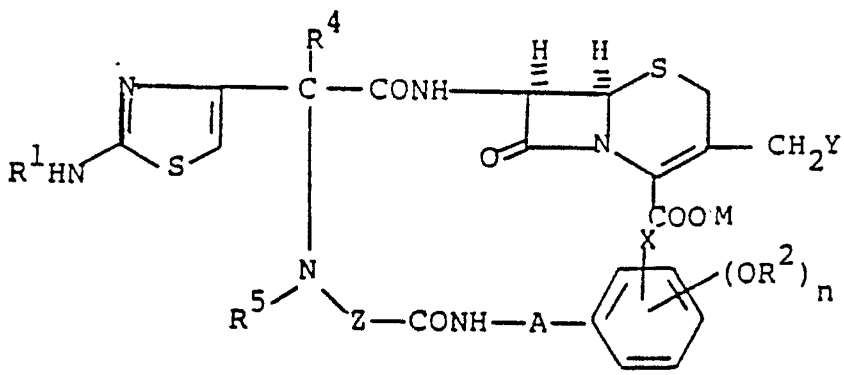

V

in which $R^1$, $R^2$, $R^4$, $R^5$, X, Z, A, M and n have the significance given above and Y represents a leaving group,

with a compound of the general formula

$HSR^3$ VI

in which $R^3$ has the significance given above.

and, if desired, cleaving off any protecting groups, or

(c) reacting a compound of the general formula

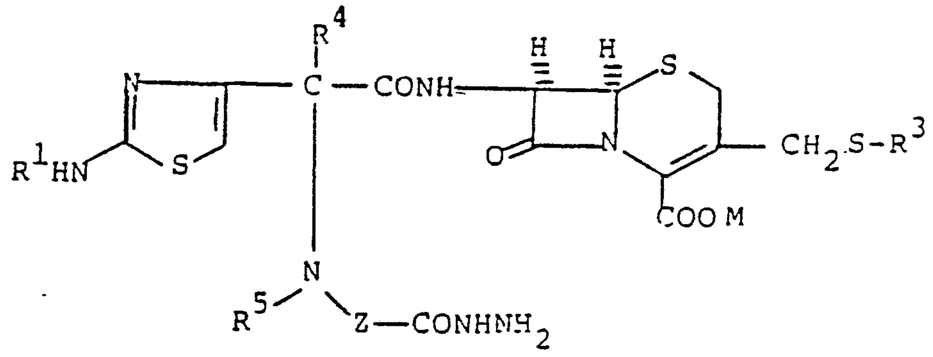

VII

in which $R^1$, $R^3$, $R^4$, $R^5$, Z and M have the above significance,

with a compound of the general formulae

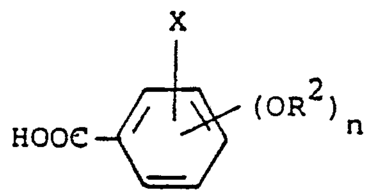

VIIIa

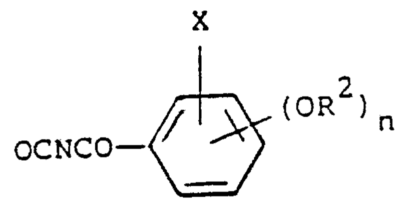

VIIIb

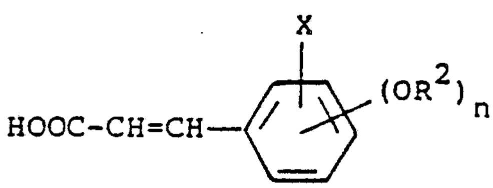

VIIIc

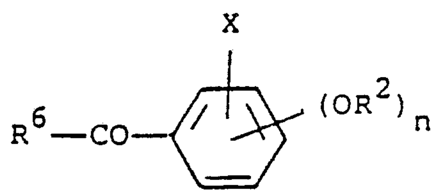

VIIId

in which $R^2$, $R^6$, X and n have the significance given above,

or a reactive derivative of one of the carboxylic acids VIIIa and VIIIc and, if desired, cleaving off any protecting groups, or

(d) for the manufacture of compounds of formula I in which $R^4$ and $R^5$ together represent an additional bond, reacting a compound of the general formula

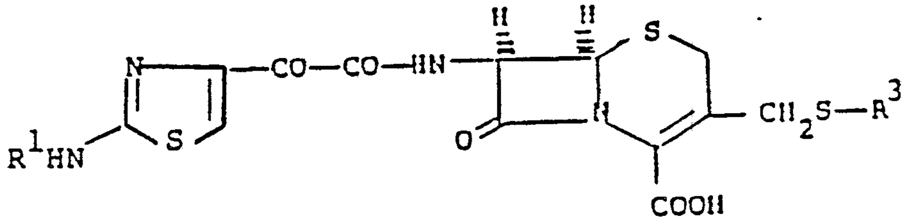

IX

wherein $R^1$ and $R^3$ have the significance given above,

with a salt of a compound of the general formula

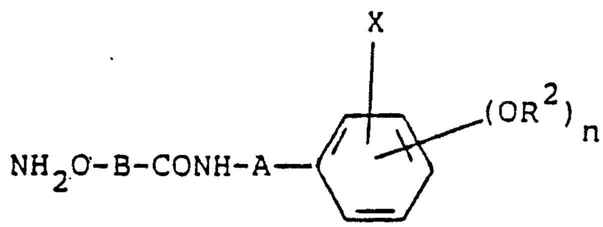

X

in which $R^2$, A, B, X and n have the above significance,

or

(e) for the manufacture of a readily hydrolyzable ester of a compound of formula I, subjecting a carboxylic acid of formula I to an appropriate esterification, or

(f) for the manufacture of salts and hydrates of a compound of formula I or of hydrates of these salts, converting a compound of formula I into a salt or hydrate or into a hydrate of this salt.

Any reactive groups in the starting compounds used above can be protected, if desired. Thus e.g. phenolic hydroxy groups can be protected by lower alkanoyl groups, such as acetyl, lower alkoxycarbonyl groups, such as methoxycarbonyl, t-butoxycarbonyl, or by trimethylsilyl or tetrahydropyranyl; and amino groups can be protected by e.g. protecting groups which are cleavable by acid, e.g. t-butoxycarbonyl, trityl or formyl, or also protecting groups which are cleavable by basic hydrolysis, such as e.g. trifluoroacetyl, or by protecting groups which are cleavable with thiourea, such as e.g. chloro-, bromo-and iodoacetyl. Carboxy groups can be protected by e.g. benzhydryl, t-butyl, allyl or trimethylsilyl groups.

In accordance with variant (a) of the process in accordance with the invention a compound of general formula III is reacted with a carboxylic acid of formula IV or with one of its reactive derivatives. As acylating agents there come into consideration: corresponding carboxylic acids of formula IV in the presence of 2-halopyridinium salts, e.g. of 2-chloro-or 2-fluoro-1-methylpyridinium chloride or tosylate, or also in the presence of N,N'-dicyclohexylcarbodiimide, preferably together with N-hydroxybenztriazole or N-hydroxysuccinimide. There can also be used corresponding reactive derivatives of the carboxylic acid such as e.g. the acid halide, acid anhydride or acid azide. Also usable are the corresponding thiolesters such as e.g. 2-benzthiazolyl thioester as well as hydroxybenztriazole esters or N-hydroxysuccinimide esters. Possible ester protecting groups in compounds III are e.g. benzhydryl, t-butyl, allyl or trimethylsilyl groups. The reaction is preferably carried out in an organic solvent or solvent mixture, optionally in admixture with water, e.g. acetone, methylene chloride, dimethylacetamide, dimethylformamide or acetonitrile, optionally in admixture with water. The temperature generally lies between -30°C and room temperature.

In accordance with variant (b) of the process in accordance with the invention a compound V is thiolated with a compound VI. As possible ester protecting groups there come in to consideration the same as given above for the compounds III. Leaving groups Y are, for example, halogens, e.g. iodine, acyloxy residues, e.g. lower alkanoyloxy residues such as acetoxy, lower alkyl-or arylsulphonyloxy residues such as mesyloxy or tosyloxy. Y in the significance acetoxy is preferably used. Thereby, the reaction can be carried out in a manner known per se, e.g. at a temperature between about 40 and 80°C, conveniently at about 60°C, in water or in a buffer solution having a pH of about 6-7. Where Y represents a halogen atom, M in formula V should represent an ester protecting group, with the reaction being preferably carried out in a polar organic solvent, e.g. in dimethylformamide ethyl acetate, acetonitrile or tetrahydrofuran. The temperature preferably lies between -30°C and room temperature.

In accordance with process variant (c) of the process in accordance with the invention a hydrazide of formula VII is reacted with a compound of formulae VIIIa, VIIIb, VIIIc or VIIId or with a reactive derivative of one of the carboxylic acids of formulae VIIIa and VIIIc. When a carboxylic acid of formula VIIIa or VIIIc is used, the reaction is carried out e.g. in the presence of a condensation agent such as N,N'-dicyclohexylcarbodiimide, N,N'-carbonyldiimidazole, cyanogen chloride, Vilsmeier reagent or the like. As reactive derivatives of the carboxylic acids VIIIa and VIIIc there are used e.g. acid halides, mixed anhydrides or active esters. The reaction temperature preferably lies in the range of -10 to +20°C. The reaction with compounds VIIIa, VIIIb and VIIIc or with corresponding, reactive derivatives is preferably effected in an inert solvent such as dimethylformamide, dimethylacetamide, dimethyl sulphoxide, methylene chloride, chloroform, acetonitrile or in a mixture of these solvents. When using compounds VIIIb and VIIId, the reaction temperature preferably lies in the range of about 0° and +50°C. When compounds VIIId are used, the reaction is preferably carried out in water or in a lower alkenol such as methanol or ethanol.

In accordance with process variant (d) of the process in accordance with the invention a

ketocephalosporin of formula IX is reacted with a salt of an O-substituted hydroxylamine of formula X. As the salt there preferably comes into consideration a mineral acid salt, e.g. the hydrochloride, or an organic sulphonate such as e.g. the p-toluenesulphonate. The salt is preferably used in about an equimolar amount up to a slight excess. The reaction is preferably carried out in a polar organic solvent, e.g. in dimethylformamide, N-methylpyrrolidine, dimethyl sulphoxide, acetonitrile, water or, especially, in dimethylacetamide. When the latter solvent is used, there are obtained especially high amounts of the syn form of the end product. The temperature preferably lies between 0°C and room temperature.

Any protecting groups in an obtained compound, e.g. on phenolic hydroxy or amino, can be cleaved off; phenolic hydroxy protecting groups e.g. as follows: acetyl with water at pH 7-8 or with ammonia, trimethylsilyl with ethanol or water, tetrahydropyranyl by acidic hydrolysis, e.g. with aqueous hydrochloric acid. Amino protecting groups can be cleaved off as follows: amino protecting groups which are cleavable by acid are preferably removed with the aid of a lower alkanecarboxylic acid which is optionally halogenated. In particular, formic acid or trifluoroacetic acid is used. As a rule, the temperature is room temperature, although slightly elevated or slightly lowered temperature can be used, e.g. in the range of about 0°C to 40°C. Protecting groups which are cleavable by alkali are generally hydrolyzed with dilute aqueous caustic alkali at 0°C to 30°C. The chloroacetyl, bromoacetyl and iodoacetyl protecting groups can be cleaved off by means of thiourea in an acidic, neutral or alkaline medium at about 0-30°C.

Any carboxy protecting groups can be cleaved off as follows: when the protecting group represents a trimethylsilyl group, this group can be removed especially readily by treatment with water or ethanol. Benzhydryl and t-butyl groups can be cleaved off with formic acid or trifluoroacetic acid in the manner given above. Allyl groups are removed e.g. by means of palladium salts and tertiary amines such as N-methylpyrrolidine or N-methylmorpholine.

For the manufacture of the readily hydrolyzable esters of the carboxylic acids of formula I in accordance with variant (e). the carboxylic acid is preferably reacted with the halide, preferably with the iodide, containing the ester group. The reaction can be accelerated with the aid of a base. e.g. an alkali metal hydroxide or carbonate or an organic amine such as triethylamine. This reaction is preferably carried out in an inert organic solvent such as dimethylacetamide, hexamethylphosphoric acid triamide, dimethyl sulphoxide or, preferably, dimethylformamide. The temperature preferably lies in the range of about 0 to 40°C.

The manufacture of the salts and hydrates of the compounds of formula I or of the hydrates of these salts can be effected in a manner known per se, e.g. by reacting the carboxylic acid of formula I with an equivalent amount of the desired base, conveniently in a solvent such as water or in an organic solvent such as ethanol, methanol, acetone or many others. Corresponding salt formation is brought about by the addition of an organic or inorganic acid. The temperature of the salt formation is not critical. It generally lies at room temperature, but can also be slightly thereover or thereunder, for example in the range of 0°C to +50°C.

The manufacture of the hydrates is effected for the most part automatically in the course of the manufacturing process or as a consequence of hygroscopic properties of an initially anhydrous product. For the planned manufacture of a hydrate, a completely or partially anhydrous product (carboxylic acid of formula I or ester or salt thereof) can be exposed to a moist atmosphere, e.g. at about +10°C to +40°C.

The starting compounds of formula III can be prepared by thiolation in analogy to the above reaction of the compounds V and VI. The same applies to the preparation of the starting compounds of formula IX. The starting compounds of formula VII can be prepared e.g. from the corresponding 3-acetoxymethyl compounds by thiolation in analogy to the above reaction of the compounds V and VI.

The thiols of formula VI are novel compounds; corresponding carbamoyl derivatives [$R^1$ = a group (a)-(d)] can be prepared e.g. from the corresponding esters, e.g. the 2-(lower alkoxycarbonyl)-s-triazolo[1,5-a]-pyrimidine-7-thiol (Eur. Pat. Publ. 150, 507) or the 3-(lower alkoxycarbonyl)-pyrazolo[1,5-a]pyrimidine-7-thiol (J. Med. Chem. 1981, 24(5), 610-13) or from analogous esters by aminolysis with ammonia or the corresponding amine; or from the corresponding carboxylic acids in the presence of 2-halopyridinium salts, e.g. of 2-chloro-o-2-fluoro-1-methylpyridinium chloride or tosylate, or also in the presence of dicyclohexylcarbodiimide, preferably together with N-hydroxybenztriazole or N-hydroxysuccinimide; or also from the corresponding acid chloride with ammonia or the corresponding amine. The preparation of corresponding 5-thiols is described in Examples 3 and 5. Thereby, a correspondingly substituted 5-amino-s-triazole is converted with an ω,ω,ω-trifluoroacetic acid ester or with diketene, followed by a dehydration agent such as conc. sulphuric acid or polyphosphoric acid into the corresponding 5-hydroxy-s-triazolo(or pyrazolo)[1,5-a]-pyrimidine. Further processing to the desired 5-thiol is effected in analogy to the synthesis of the 7-thiols described in J. Med. Chem. 1981, 24, 610-613.

A syn/anti mixture of a compound of formula I which may be obtained can be separated in to the

corresponding syn and anti forms in the usual manner, for example by recrystallization or by chromatographic methods using a suitable solvent or solvent mixture.

The compounds of formula I as well as the corresponding readily hydrolyzable esters and salts and, respectively, the hydrates of these products have antibiotic, especially bactericidal, activity. They have a broad spectrum of activity against gram-positive and gram-negative microorganisms, including Staphylococci and various cephalosporin-resistant gram-negative bacteria such as e.g. Pseudomonas aeruginosa, Enterobacter cloacae, Escherichia coli, Serratia marcescens, Proteus and Klebsiella species.

The compounds of formula I as well as the corresponding readily hydrolyzable esters and salts and, respectively, the hydrates of these products can be used for the treatment and prophylaxis of infectious diseases. A daily dosage of about 0.1 to about 4 g comes into consideration for adults. The parenteral administration of the compounds in accordance with the invention is especially preferred.

In order to demonstrate the antimicrobial activity of the mentioned products, various end products prepared in accordance with the working Examples hereinafter were tested in vivo for their activity. The minimal inhibitory concentration (µg/ml) was measured. Reference is made to the numbers of the working Examples:

Table

| Organism | End product from Example No. | | | | | | |
|---|---|---|---|---|---|---|---|
| | 1 | 2 | 3a | 3b | 3c | 3d | 3e |
| S. aureus 6538 | 4 | 2 | 4 | 8 | 8 | 16 | 32 |
| E. coli 25922 | - | - | 0.12 | <0.06 | <0.06 | 0.25 | <0.06 |
| E. coli TEM 1 | <0.06 | <0.06 | <0.06 | <0.06 | <0.06 | <0.06 | <0.06 |
| K. pneumoniae 418 | <0.06 | <0.06 | <0.06 | <0.06 | <0.06 | <0.06 | <0.06 |
| K. oxytoca 1082 E | 0.12 | 0.25 | 0.25 | 0.12 | 0.12 | 0.5 | 0.25 |
| E. cloacae P 99 | 2 | 1 | 0.5 | 4 | 4 | 4 | 1 |
| P. vulgaris 1028 | 0.25 | 0.12 | 0.25 | 0.25 | 0.25 | 0.5 | 0.12 |
| P. aeruginosa 143811S | 0.25 | 0.12 | 0.25 | 0.12 | 0.25 | 0.25 | 0.12 |
| P. aeruginosa 143811R | 0.5 | 0.12 | 0.25 | 0.5 | 1 | 4 | 0.5 |
| S. pyogenes D 15 | 0.25 | 0.12 | 0.25 | 0.12 | 0.25 | 1 | 0.5 |
| S. marcescens 69438 | 0.5 | 0.25 | 0.5 | 0.25 | 0.25 | 1 | 0.25 |

Table (continued)

| Organism | End product from Example No. | | | | | | | |
|---|---|---|---|---|---|---|---|---|
| | 4 | 5 | 6 | 7 | 8 | 9 | 10 | 17 |
| S. aureus 6538 | 8 | 4 | 4 | 16 | 4 | 16 | 2 | 8 |
| E. coli 25922 | <0,06 | <0,06 | <0,06 | <0,06 | 0,12 | <0,06 | <0,06 | <0,06 |
| E. coli TEM 1 | <0,06 | <0,06 | <0,06 | <0,06 | <0,06 | <0,06 | <0,06 | <0,06 |
| K. pneumoniae 418 | <0,06 | <0,06 | <0,06 | <0,06 | <0,06 | <0,06 | <0,06 | <0,06 |
| K. oxytoca 1082 E | 0,5 | 0,5 | 0,5 | <0,06 | 1 | 0,12 | 0,12 | 0,12 |
| E. cloacae P 99 | 2 | 4 | 2 | 1 | 8 | 1 | 1 | 1 |
| P. vulgaris 1028 | 0,12 | 0,25 | 0,12 | <0,06 | 0,25 | 0,12 | 0,25 | 0,12 |
| P. aeruginosa 143811S | 0,12 | 0,12 | 0,25 | 0,25 | 0,5 | 0,12 | 0,5 | 0,25 |
| P. aeruginosa 143811R | 0,25 | 0,25 | 0,5 | 0,5 | 1 | 0,12 | 0,5 | 0,5 |
| S. pyogenes B 15 | 0,5 | 0,12 | 0,25 | 2 | 0,25 | 0,25 | <0,06 | 1 |
| S. marcescens 69438 | 0,12 | 0,25 | 0,12 | 0,5 | 0,5 | 0,12 | 0,5 | 0,25 |

The products in accordance with the invention can be used as medicaments, e.g. in the form of pharmaceutical preparations which contain them or their salts in admixture with a pharmaceutical, organic or inorganic inert carrier material which is suitable for enteral or parenteral administration such as e.g. water, gelatine, gum arabic, lactose, starch, magnesium stearate, talc, vegetable oils, polyalkylene glycols, Vaseline, etc. The pharmaceutical preparations can be present in solid form, e.g. as tablets, dragees, suppositories, capsules; or in liquid form, e.g. as solutions, suspensions or emulsions. If desired, they are sterilized and/or contain adjuvants such as preserving, stabilizing, wetting or emulsifying agents, salts for varying the osmotic pressure, anaesthetics or buffers. They can also contain still other therapeutically valuable substances. The compounds of formula I and their salts and, respectively, hydrates preferably come into consideration for parenteral administration and, for this purpose, are preferably prepared as lyophilizates or dry powders for dilution with usual agents such as water or isotonic saline. The readily hydrolyzable esters of the compounds of formula I and their salts and, respectively, hydrates also come into consideration for enteral administration.

In the working Examples hereinafter all temperature details are in degrees Celsius.

## Example 1

0.75 g of (6R,7R)-7-(2-amino-4-thiazoleglyoxylamido)-3-[[(2-carbamoyl-5-methyl-s-triazolo[1,5-a]-pyrimidin-7-yl)thio]methyl-8-oxo-5-thia-1-azabicyclo[4.2.0]oct-2-ene-2-carboxylic acid potassium salt is dissolved in 10 ml of dimethylacetamide, treated with 0.60 g of 1-[2-(aminooxy)-2-methylpropionyl]-2-(3,4-dihydroxybenzoyl)hydrazine hydrochloride and stirred at room temperature for 16 hours. Dimethylacetamide is removed by evaporation in a high vacuum, the residue is taken up with water and stirred at 0° for 30

minutes for the complete precipitation of the product. By filtration and drying in a high vacuum there is obtained (6R,7R)-7-[(Z)-2-(2-amino-4-thiazolyl)-2-[[1-[3-(3,4-dihydroxybenzoyl)carbazoyl]-1-methylethoxy]-imino]acetamido]-3-[[(2-carbamoyl-5-methyl-s-triazolo[1,5-a]pyrimidin-7-yl)thio]methyl]-8-oxo-5-thia-1-azabicyclo[4.2.0]oct-2-ene-2-carboxylic acid as a white powder.

$^1$H NMR: (DMSO-$d_6$): δ 1.46 (s,3H); 1.51 (s,3H) 2.59 (s,3H); 3.48 (d,J = 18 Hz,1H); 3.70 (d,J = 18 Hz,1H); 4.36 (d,J = 14 Hz,1H); 4.62 (d,J = 14 Hz,1H); 5.14 (d,J = 5 Hz,1H); 5.78 (dd,J = 8 Hz and J = 5 Hz,1H); 6.75 (d,J = 10 Hz,1H); 6.86 (s,1H); 7.20 (d,J = 10 Hz,1H), 7.30 (s,1H); 7.8 (s,1H); 8.17 (s,1H); 9.24 (s,H,broad); 8.59 (d,J = 8 Hz,1H); 9.92 (s,1H,broad) ppm.

IR (Kbr) 1771.

The (6R,7R)-7-(2-amino-4-thiazoleglyoxylamido)-3-[[(2-carbamoyl-5-methyl-s-triazolo[1,5-a]pyrimidin-7-yl)thio]methyl]-8-oxo-5-thia-1-azabicyclo[4.2.0]oct-2-ene-2-carboxylic acid used as the starting compound can be prepared as follows:

A suspension of 2.50 g of methyl 7-mercapto-5-methyl-s-triazolo[1,5-a]pyrimidine-2-carboxylate in 25 ml of 25 percent aqueous ammonia is stirred at room temperature for 6 hours. The mixture is filtered and the solid is dried at 50° in a vacuum. There is obtained 7-mercapto-5-methyl-s-triazolo[1,5-a]pyrimidine-2-carboxamide as a 1:1:1 adduct with water and ammonia.

$^1$H NMR (DMSO-$d_6$): δ 2.26 (s,3H); 6.72 (s,1H); 7.17 (s,4H,$NH_4^+$); 7.60 (broad s,1H); 7.84 (broad s,1H) ppm.

A mixture of 5.46 g of (7R)-7-aminocephalosporanic acid and 4.85 g of 7-mercapto-5-methyl-s-triazolo-[1,5-a]pyrimidine-2-carboxamide is treated while stirring well with 50 ml of 20 percent solution of boron trifluoride in acetonitrile. The temperature is held below 40° by means of ice-bath cooling. The reaction mixture is stirred at 20° for 1 hour and subsequently diluted with 200 ml of water. A white precipitate forms, which is collected by filtration. The still moist material is dissolved in 50 ml of 3N HCl and the solution is filtered. A white product crystallizes out from the filtrate after a short period. By filtration, washing with $H_2O$ and acetone and drying in a vacuum there is obtained (6R,7R)-7-amino-3-[[(2-carbomoyl-5-methyl-s-triazolo-[1,5-a]pyrimidin-7-yl)thio]methyl]-8-oxo-5-thia-1-azabicyclo[4.2.0]oct-2]-ene-2-carboxylic acid as the hydrochloride.

$^1$HNMR (DMSO-$d_6$): δ 2.61 (s,3H); 3.74 (d,J = 17.5 Hz,1H); 2.87 (d,J = 17.5 Hz,1H); 4.46 (d,J = 12.5 Hz,1H); 4.54 (d,J = 12.5 Hz,1H); 5.20 (d,J = 5 Hz,1H); 5.25 (d,J = 5 Hz,1H); 7.43 (s,1H); 7.89 (s,1H); 8.19 (s,1H) ppm.

MS: 422 (M + $H^+$).

IR (KBr): 1770.

A suspension of 1.71 g of (6R,7R)-7-amino-3-[[(2-carbamoyl-5-methyl-s-triazolo[1,5-a]pyrimidin-7-yl)-thio]methyl]-8-oxo-5-thia-1-azabicyclo[4.2.0]oct-2-ene-2-carboxylic acid hydrochloride in 20 ml of methylene chloride is treated with 2.74 ml of N,O-bis-(trimethylsilyl)acetamide. After all has passed into solution 1.32 g of 2-amino-4-thiazolethioglyoxylic acid s-(2-benzothiazolyl) ester are added and the mixture is stirred at 20° for 1.5 hours. Undissolved material is separated by filtration and the filtrate is diluted with 40 ml of methylene chloride. Upon the dropwise addition of 2 ml of ethanol there results a yellow precipitate which is collected by filtration and dried in a vacuum. There is obtained (6R,7R)-7-(2-amino-4-thiazoleglyoxylamido)-3-[[(2-carbamoyl-5-methyl-s-triazolo[1,5-a]pyrimidin-7-yl)thio]methyl]-8-oxo-5-thia-1-azabicyclo[4.2.0]oct-2-ene-2-carboxylic acid as a yellow powder.

$^1$H NMR (DMSO-$d_6$): δ 2.60 (s,3H); 3.54 (d,J = 17.5 Hz,1H); 3.74 (d,J = 17.5 Hz,1H); 4.42 (d,J = 14 Hz,1H); 4.56 (d,J = 14 Hz,1H); 5.14 (d,J = 5 Hz,1H); 5.72 (d,J = 5 Hz,1H); 7.41 (s,2H); 7.55 (s,1H) ppm.

The 1-[2-(aminooxy)-2-methylpropionyl]-2-(3,4-dihydroxybenzoyl)hydrazine hydrochloride used as the starting compound can be prepared as follows:

1. Allyl 2-methyl-2-(phthalimidooxy)propionate

230 g of α-bromoisobutyryl bromide are added dropwise while cooling with ice to a solution of 58 g of allyl alcohol and 316 g of pyridine in 1 l of methylene chloride. After 1.5 hours the mixture is treated with 1 l of 3N hydrochloric acid, the organic phase is separated and washed in succession with in each case 1 l of 0.5N hydrochloric acid, 0.5M aqueous sodium bicarbonate and water. Thereafter, the solvent is removed by evaporation. The residue is added to a suspension of 146.8 g of N-hydroxyphthalimide, 165.8 g of potassium carbonate and 20 g of potassium iodide in 1 l of DMSO. The mixture is stirred at 60° for 4 hours. After cooling the mixture is poured into 6 l of water and the product is extracted with 3 l of ethyl acetate. After washing twice with 0.5 l of sodium chloride solution the solvent is removed by evaporation and the residue is recrystallized from 0.6 l of tert.butyl methyl ether and 1.5 l of hexane. The product, m.p. 51-53°, is obtained.

2. 2-Methyl-2-(phthalimidooxy)propionic acid

The allyl ester (187.6 g) obtained in step 1 is dissolved in 1.5 l of acetonitrile. After the addition of 1 g of palladium-(II) acetate, 6.5 ml of triethyl phosphite and 62.4 g of N-methylpyrrolidine the mixture is stirred at room temperature for 2 hours. The solvent is removed by evaporation and the residue is partitioned between 0.5 l of water and 0.5 l of ethyl acetate. The aqueous phase is then adjusted to pH 3-4 with 150 ml of 3N aqueous HCl. After cooling the product is filtered off. M.p. 133-135°.

3. 2-Methyl-2-(phthalimidooxy)propionic acid 2-benzothiazole thiolester

148.2 g of triphenylphosphine and 187.9 g of bis-benzothiazol-2-yl) disulphide are stirred at room temperature for 2 hours in 2 l of methylene chloride. After cooling to 0-5° 98 g of the acid obtained in step 2 are added and the mixture is stirred at 0-5° for a further 3 hours. Undissolved material is filtered off and the filtrate is evaporated. The residue is crystallized using 1 l of ethyl acetate. After filtration it is again suspended in 1 l of isopropanol, filtered off under suction and dried. The product obtained has a m.p. of 170-172°.

4. Methyl 3,4-dihydroxybenzoate

A mixture of 180 g of dihydroxybenzoic acid, 360 ml of dichloroethane, 142 ml of methanol and 18 g of p-toluenesulphonic acid is boiled on a water separator until the distillate is clear. The mixture is evaporated, the residue is taken up with 1.5 l of diethyl ether, washed with 1 l of saturated aqueous sodium bicarbonate solution and twice with 0.4 l of aqueous sodium chloride solution each time. After renewed evaporation the residue is crystallized from 3 l of toluene and there is obtained the product, m.p. 136-137°.

5. 3,4-Dihydroxybenzhydrazide

120 g of methyl 3,4-dihydroxybenzoate are left to react at room temperature for 3 days with 340 ml of methanol and 340 ml of hydrazine hydrate. Thereafter, 340 ml of water are added and the mixture is evaporated in a vacuum. The addition of water and evaporation is repeated twice. Finally, the product is crystallized from 340 ml of water, m.p. 260° with decomposition.

$C_7H_8N_2O_3$:

C: 49.70% calc. 50.00%
H: 4.87% calc. 4.80%
N: 16.40% calc. 16.66%

6. 1-(3,4-Dihydroxybenzoyl)-2-[2-methyl-2-(phthalimidooxy)propionyl]hydrazine

56.1 g of bis-trimethylsilyl-acetamide are added to 23.2 g of 3,4-dihydroxybenzhydrazide and boiled under reflux for 2 hours. After cooling to room temperature 55 g of 2-methyl-2-(phthalimidooxy)propionic acid 2-benzothiazole thiolester are added and the mixture is stirred at room temperature for 24 hours. After removing the solvent by evaporation the residue is boiled for a few minutes in 0.5 l of ethanol and evaporated in a vacuum. Recrystallization from 0.5 l of ethanol gives the product, m.p. 233-235°.

$C_{19}H_{17}N_3O_7$
C 57.11% calc. 57.14%
H 4.41% calc. 4.29%
N 10.49% calc. 10.52%

7. 1-[2-(Aminooxy)-2-methylpropionyl]-2-(3,4-dihydroxybenzoyl)hydrazine hydrochloride

5.85 g of hydrazine hydrate are added to the suspension of 46.7 g of 1-(3,4-dihydroxybenzoyl)-2-[2-methyl-2-(phthalimidooxy)propionyl]hydrazine in 400 ml of ethanol and stirred at room temperature for 1 hour. After filtering off from insoluble material the filtrate is evaporated, the residue is again taken up in 120 ml of ethanol, filtered and treated with 23.4 ml of 5N ethanolic hydrochloric acid and thereafter dropwise with 0.5 l of diethyl ether. For purification, the crystallizate is recrystallized from 140 ml of ethanol. There is obtained the product, m.p. 198-201° (dec.).

$C_{11}H_{15}N_3O_5 \bullet HCl \bullet C_2H_5OH$
C 43.91% calc. 44.39%
H 6.28% calc. 6.30%
N 12.04% calc.11.95%
Cl 10.01% calc. 10.08%

Example 2

2.45 g of (6R,7R)-7-(2-amino-4-thiazoleglyoxylamido)-3-[[(3-carbamoyl-5-methylpyrazolo[1,5-a]pyrimidin-7-yl)thio]-methyl]-8-oxo-5-thia-1-azabicyclo[4.2.0]oct-2-ene-carboxylic acid and 1.95 g of 1-[2-(aminooxy)-2-methylpropionyl]-2-(3,4-dihydroxybenzoyl)hydrazine hydrochloride are dissolved in 10 ml of dimethylacetamide. The reaction mixture is stirred at room temperature for 16 hours. 70 ml of water are added and the resulting suspension is stirred at room temperature for a further 1 hour. By filtration and drying in a high vacuum there is obtained (6R,7R)-7-[(Z)-2-(2-amino-4-thiazolyl)-2-[[1-[3-(3,4-dihydroxybenzoyl)-carbazoyl]-1-methylethoxy]imino]acetamido]-3-[[(3-carbamoyl-5-methylpyrazolo [1,5-a]pyrimidin-7-yl)-thio]methyl]-8-oxo-5-thia-1-azabicyclo[4.2.0]oct-2-ene-2-carboxylic acid as a white powder.
$^1$H NMR (DMSO-$d_6$): δ 1.47 (s,3H); 1.50 (s,3H); 2.60 (s,3H); 3.62 (d,J = 18 Hz,1H), 3.84 (d,J = 18 Hz,1H); 4.32 (d,J = 14 Hz,1H); 4.46 (d,J = 14 Hz,1H); 5.25 (d,J = 5 Hz,1H); 5.91 (dd,J = 8 Hz and J = 5 Hz,1H); 6.76 (d,J = 10 Hz,1H); 6.87 (s,1H); 7.20 (s,1H); 7.24 (d,J = 10 Hz,1H); 7 29 (broad s,Σ4H); 7.49 (s,1H); 7.56 (s,1H); 8.48 (s,1H); 9.20 (s,1H); 9.29 (s,1H); 9.55 (s,1H); 9.66 (d,J = 8 Hz,1H); 10.00 (s,1H) ppm.
IR (KBr) 1772.

The (6R,7R)-7-(2-amino-4-thiazoleglyoxylamido)-3-[[(3-carbamoyl-5-methylpyrazolo[1,5-a]pyrimidin-7-yl)thio]methyl]-8-oxo-5-thia-1-azabicyclo[4.2.0]oct-2-ene-2-carboxylic acid used as the starting compound can be prepared as follows:

Ethyl 7-mercapto-5-methylpyrazolo[1,5-a]pyrimidine-3--carboxylate (J. Med. Chem. 1981, 24(5), 610-13) is heated in 1N aqueous sodium hydroxide solution until all has passed into solution. The reaction mixture is cooled and its pH is adjusted to 1 with aqueous hydrochloric acid. The resulting precipitate is filtered off, dried, and recrystallized from dimethylformamide. There is obtained 7-mercapto-5-methyl-pyrazolo[1,5-a]pyrimidine-3-carboxylic acid as a yellow powder of melting point 161-162° (dec.).

A suspension of 105 g of 7-mercapto-5-methyl-pyrazolo[1,5-a]pyrimidine-3-carboxylic acid in 400 ml of methylene chloride is treated with 6.3 ml of 1-chloro-N,N,2-trimethyl-1-propenamine and treated in an ultrasound bath until the majority has passed into solution. The mixture is suction filtered over a glass fibre filter and ammonia is introduced at 20° into the clear filtrate. There results a crystalline precipitate which is filtered off under suction, dried and subsequently stirred in 100 ml of $H_2O$ for 5 minutes. The undissolved material is filtered off and the filtrate is concentrated in a vacuum to half of the volume. Upon leaving to stand at 0° there is obtained 7-mercapto-5-methylpyrazolo[1,5-a]pyrimidine-3-carboxamide as the ammonium salt in the form of white crystals.
$^1$H NMR (DMSO-$d_6$): δ 2.28 (s,3H); 6.65 (s,1H); 7.00 (broad s,1H); 7.17 (broad s,4H,$NH_4^+$ ); 7.94 broad s,1H) ppm.

A mixture of 2.80 g of 7-mercapto-5-methylpyrazolo[1,5-a]pyrimidine-3-carboxamide ammonium salt

and 3.38 g of (7R)-7-aminocephalosporanic acid is treated with 25 ml of a 20 percent solution of boron trifluoride in acetonitrile and stirred at 20° for 40 minutes. The reaction mixture is treated with 30 ml of water and the pH is adjusted to 3.5 by means of 28 percent aqueous sodium hydroxide solution. The precipitate which is thereby formed is filtered off under suction and the thus-obtained material is chromatographed on OPTI-UP ($C_{12}$) (ANTEC AG, CH-Bennwil) with water as the eluent. There is obtained (6R,7R)-7-amino-3-[[(3-carbamoyl-5-methylpyrazolo[1,5-a]pyrimidin-7-yl)thio]methyl]-8-oxo-5-thia-1-azabicyclo[4.2.0]oct-2-ene-2-carboxylic acid.

$^1$H NMR (DMSO-$d_6$): δ 2.60 (s,3H); 3.56 (d,J = 17.5 Hz,1H); 3.78 (d,J = 17.5 Hz,1H); 4.30 (d,J = 12.5 Hz,1H); 4.43 (d,J = 12.5 Hz),1H); 4.82 (d,J = 5 Hz,1H); 5.04 (d,J = 5 Hz,1H); 7.20 (s,1H); 7.50 (broad s,1H); 7.56 (broad s,1H); 8.48 (s,1H) ppm.

IR (KBr) 1795.

A suspension of 2.05 g of (6R,R)-7-amino-3-[[(3-carbamoyl-5-methylpyrazolo[1,5-a]pyrimidin-7-yl)thio]-methyl]-8-oxo-5-thia-1-azabicyclo[4.2.0]oct-2-ene-2 -carboxylic acid in 15 ml of acetonitrile and 15 ml of water is treated with 0.69 ml of triethylamine, whereupon a clear solution results. 2.28 g of 2-amino-4-thiazolethioglyoxylic acid s-(2-benzothiazolyl) ester are added to this solution. After stirring at 20° for 3 hours the reaction mixture is suction filtered and the filtrate is partitioned between ethyl acetate and water. The aqueous phase is concentrated a little in a vacuum and the pH is adjusted to 2.3 with 3N hydrochloric acid, whereupon a yellow precipitate forms. After filtration, washing with water and drying there is obtained (6R,7R)-7-(2-amino-4-thiazoleglyoxylamido)-3-[[(3-carbamoyl-5-methylpyrazolo[1,5-a]pyrimidin-7-yl)thio]-methyl]-8-oxo-5-thia-1-azabicyclo[4.2.0]oct-2-ene-2-carboxylic acid.

$^1$H NMR (DMSO-$d_6$): 2.61 (s,3H); 3.62 (d,J = 17.5 Hz,1H); 3.82 (d,J = 17.5 Hz,1H); 4.36 (d,J = 12.5 Hz,1H); 4.47 (d,J = 12.5 Hz,1H); 5.22 (d,J = 5 Hz,1H); 5.78 (dd,J = 8 and 5 Hz,1H); 7.22 (s,1H;, 7.49 (broad s,1H); 7.56 (broad s,2H); 7.84 (s,1H); 8.48 (s,1H); 9.82 (d,J = 8 Hz,1H) ppm.

IR (KBr) 1779.

## Example 3

In analogy to Example 1 or 2 there can be manufactured:

a) (6R,7R)-7-[(Z)-2-(2-Amino-4-thiazolyl)-2-,[1-[3-(3,4-dihydroxybenzoyl)carbazoyl]-1-methylethoxy]-imino]acetamido]-3-[[[3-carbamoyl-7-(trifluoromethyl)pyrazolo[1,5-a]pyrimidin-5-yl]thio]methyl]-8-oxo-5-thia-1-azabicyclo[4.2.0]oct-2-ene-2-carboxylic acid

$^1$H NMR (DMSO-$d_6$): δ 1.46 (s,3H); 1.50 (s,3H); 3.60 (d,J = 18 Hz,1H); 3.80 (d,J = 18 Hz,1H); 4.29 (d,J = 14 Hz,1H); 4.60 (d,J = 12 Hz,1H); 5.22 (d,J = 5 Hz,1H); 5.90 (dd,J = 8 Hz and J = 5 Hz,1H); 6.74 (d,J = 10 Hz,1H); 6.85 (s,1H); 7.22 (d,J = 10 Hz,1H); 7.29 (m,4H); 7.50 (broad s,1H ; 7.83 (s,1H); 8.58 (s,1H); 9.18 (s,1H); 9.23 (s,1H). 9.55 (s,1H); 9.64 (d,J = 8 Hz,1H); 10.00 (s,1H) ppm.

IR (KBr) 1772.

MS (70 eV) 880 $(M + H)^+$.

b) (6R,7R)-7-[(Z)-2-(2-Amino-4-thiazolyl)-2-[[1-[3-(3,4-dihydroxybenzoyl)carbazoyl]-1-methylethoxy]-imino]acetamido]-3-[[[5-methyl-2-(methylcarbamoyl)-s-triazolo[1,5-a]pyrimidin-7-yl]thio]methyl]-8-oxo-5-thia-1-azabicyclo[4.2.0]oct-2-ene-2-carboxylic acid.

$^1$H NMR (DMSO-$d_6$): δ 1.47 (s,3H); 1.50 (s,3H); 2.60 (s,3H); 2.80 (d,J = 5 Hz,3H); 3.64 (d,J = 18 Hz,1H); 3.85 (d,J = 18 Hz,1H); 4.43 (m,2H); 5.24 (d,J = 5 Hz,1H); 5.90 (dd,J = 8 Hz and J = 5 Hz, 1H); 6.75 (d,J = 10 Hz,1H); 6.89 (s,1H); 7.23 (d,J = 10 Hz,1H); 7.28 (m,4H); 7.38 (s,1H); 8.83 (qu,J = 5 Hz,1H); 9.18 (s,1H); 9.20 (s,1H); 9.54 (s,1H); 9.67 (d,J = 8 Hz,1H); 10.00 (s,1H) ppm.

IR (KBr) 1777.

c) (6R,7R)-7-[(Z)-2-(2-Amino-4-thiazolyl)-2-[[1-[3-(3,4-dihydroxybenzoyl)carbazoyl]-1-methylethoxy]-imino]acetamido]-3-[[[2-(dimethylcarbamoyl)-5-methyl-s-triazolo[1,5-a]pyrimidin-7-yl]thio]methyl]-8-oxo-5-thia-1-azabicyclo[4.2.0]oct-2-ene-2-carboxylic acid.

$^1$H NMR (DMSO-$d_6$): δ 1.47 (s,3H); 1.50 (s,3H); 2.61 (s,3H); 3.00 (s,3H); 3.04 (s,3H); 3.62 (d,J = 18 Hz, 1H); 3.84 (d,J = 18 Hz,1H); 4.41 (m,2H); 5.24 (d,J = 5 Hz); 5.90 (dd,J = 8 Hz and J = 5 Hz,1H); 6.72 (d,J = 10 Hz,1H); 6.86 (s,1H); 7.22 (d,J = 10 Hz,1H); 7.28 (m,4H); 7.38 (s,1H); 9.18 (s,1H); 9.22 (s,1H); 9.56 (s,1H); 9.66 (d,J = 8 Hz,1H); 1.01 (s,1H) ppm.

IR (KBr) 1776.

MS 885 $(M + H)^+$.

d) (6R,7R)-7-[(Z)-2-(2-Amino-4-thiazolyl)-2-[[1-[3-(3,4-dihydroxybenzoyl)carbazoyl]-1-methylethoxy]-imino]acetamido]-3-[[[3-carbamoyl-5-(trifluoromethyl)pyrazolo [1,5-a]pyrimidin-7-yl]thio]methyl-8-oxo-5-thia-1-azabicyclo[4.2.0]oct-2-ene-2-carboxylic acid.

$^1$H NMR (DMSO-$d_6$) δ 1.47 (s,3H); 1.50 (s,1H); 3.64 (d,J = 18 Hz,1H); 3.84 (d,J = 18 Hz,1H); 4.56 (m,2H); 5.25 (d,J = 5 Hz,1H); 5.92 (dd,J = 8 Hz and J = 5 Hz,1H); 6.75 (d,J = 10 Hz,1H); 6.87 (s,1H); 7.24 (d,J = 10 Hz,1H); 7.30 (m,4H); 7.68 (broad s,1H); 7.71 (s,1H); 9.18 (broad s,1H); 9.22 (s,1H); 9.56 (broad s,1H); 9.67 (d,H = 8 Hz,1H); 10.01 (s,1H) ppm.

IR (KBr) 1773.

MS (70 eV) 880 $(M + H)^+$.

e) (6R,7R)-7-[(Z)-2-(2-Amino-4-thiazolyl)-2-[[1-[3-(3,4-dihydroxybenzoyl)carbazoyl]-1-methylethoxy]-imino]acetamido]-3-[[2-carbamoyl-6,7-dihydro-5H-cyclopenta[d]-s-triazolo[1,5-a]pyrimidin-8-yl)thio]methyl]-8-oxo-5-oxo-5-thia-1-azabicyclo[4.2.0.]oct-2-ene-2-carboxylic acid.

$^1$H NMR (DMSO-$d_6$): δ 1.48 (s,3H); 1.52 (s,3H); 3.00 (m,4H); 3.62 (d,J = 18 Hz,1H); 3.80 (d,J = 18 Hz,1H); 4.32 (m,2H); 5.17 (d,J = 5 Hz): 5.81 (dd,J = 8 Hz and J = 5 Hz,1H); 6.75 (d,J = 10 Hz,1H); 6.87 (s,1H); 7.25 (d,J = 10 Hz,1H); 7.30 (s + m,3H); 7.85 (s,1H); 8.15 (s,1H); 9.18 (s,1H); 9.20 (s,1H); 9.55 (s,1H); 9.64 (d,J = 8 Hz,1H); 10.01 (s,1H); ppm.

IR (KBr) 1775.

The (6R,7R)-7-(2-amino-4-thiazoleglyoxylamido)-3-[[(3-carbamoyl-7-trifluoromethyl)pyrazolo[1,5-a]-pyrimidin-5-yl)thio]methyl]-8-oxo-5-thia-1-azabicyclo[4.2.0]oct-2-ene-2-carboxylic acid used as the starting compound for product a) can be prepared as follows:

A mixture of 11.5 g of methyl 5-aminopyrazole-4-carboxylate, 16 ml of ethyl ω,ω,ω-trifluoroacetate and 150 g of polyphosphoric acid is heated to 100° while stirring for 16 hours. After cooling to 20° cold water is added and the mixture is extracted with ethyl acetate. The organic phase is washed with 1N aqueous hydrochloric acid and aqueous saturated sodium chloride solution and dried over $Na_2SO_4$. The solvent is removed by evaporation in a vacuum and the residue is taken up in ether. The solid product is filtered off under suction and recrystallized from 2-propanol. There is obtained an isomer of the product, namely methyl 7-hydroxy-5-(trifluoromethyl)pyrazolo[1,5-a]pyrimidine-3-carboxylate of m.p. 216-217°. The product enriched in the mother liquors is crystallized from ethyl acetate. There is obtained methyl 5-hydroxy-7-(trifluoromethyl)pyrazolo[1,5-a]pyrimidine-2-carboxylate of m.p. 149-150°.

A mixture of 2.70 g of methyl 5-hydroxy-7-(trifluoromethyl)pyrazolo[1,5-a]pyrimidine-3-carboxylate and 1.26 g of 4-dimethylaminopyridine is heated to 100° for 2.5 hours in 50 ml of phosphorus oxychloride. The resulting solution is concentrated in a vacuum and then partitioned between ethyl acetate and saturated sodium chloride solution. The organic phase is washed in succession with 1N hydrochloric acid and saturated sodium chloride solution dried over sodium sulphate, freed from solvent in a vacuum and the residue is crystallized from ethyl acetate/petroleum ether. There is obtained methyl 5-chloro-7-(trifluoromethyl)pyrazolo[1,5-a]pyrimidine-3-carboxylate as white crystals of m.p. 126-127°.

A mixture of 2.30 g of methyl 5-chloro-7-(trifluoromethyl)pyrazolo[1,5-a]pyrimidine-3-carboxylate and 2.0 g of sodium hydrogen sulphide monohydrate in 6.0 ml of water is stirred at 60° for 1 hour. The reaction mixture is cooled, acidified with hydrochloric acid and extracted with ethyl acetate. The organic phase is washed with saturated sodium chloride solution, dried over sodium sulphate and concentrated in a vacuum up to crystallization. Petroleum ether is added and the solid product is filtered off under suction. There is obtained methyl 5-mercapto-7-(trifluoromethyl)pyrazolo[1,5-a]pyrimidine-3-carboxylate.

$^1$H NMR (DMSO-$d_6$): δ 3.82 (s,3H); 7.30 (s,1H); 8.40 (s,1H) ppm.

A solution of 1.80 g of methyl 5-mercapto-7-(trifluoromethyl)pyrazolo[1,5-a]pyrimidine-3-carboxylate in 800 ml of 25 percent aqueous ammonia is left to stand at 20° for 24 hours. The solution is concentrated in a vacuum to a small volume, made acid with 1N hydrochloric acid and extracted with ethyl acetate. The organic phase is washed with saturated sodium chloride solution, dried over sodium sulphate and evaporated in a vacuum. The residual crystal slurry is treated with petroleum ether and filtered. There is obtained 5-mercapto-7-(trifluoromethyl)pyrazolo[1,5-a]pyrimidine-3-carboxamide.

$^1$H NMR (DMSO-$d_6$): δ 7.28 (s,1H); 7.62 (broad s,1H); 8.08 (broad s,1H); 8.45 (s,1H) ppm.

A mixture of 1.60 g of 5-mercapto-7-(trifluoromethyl)pyrazolo[1,5-a]pyrimidine-3-carboxamide and 1.66 g of (7R)-7-aminocephalosporanic acid in 120 ml of acetonitrile is treated while stirring with 20 ml of a 20 percent solution of boron trifluoride in acetonitrile and stirred at 20° for a further 5 hours. 100 ml of water are added and the mixture is concentrated in a vacuum to a volume of about 80 ml. The pH is adjusted to 2.8 with 25 percent aqueous ammonia. After stirring for 1 hour the precipitate obtained is filtered, washed in succession with water, acetone and ether and dried. There is obtained (6R,7R)-7-amino-3-[[(3-carbamoyl-7-(trifluoromethyl)pyrazolo[1,5-a]pyrimidin-5-yl)thio]methyl]-8-oxo-5-thia-1-azabicyclo[4.2.0]oct-2-ene-2-carboxylic acid.

$^1$H NMR (DMSO-$d_6$): δ 3.53 (d,J = 17.5 Hz,1H); 3.72 (d,J = 17.5 Hz,1H); 4.24 (d,J = 12.5 Hz,1H); 4.58 (d,J = 12.5 Hz,1H); 4.80 (d,J = 5 Hz,1H); 4.98 (d,J = 5 Hz,1H); 7.35 (broad s,1H); 7.52 (broad s,1H); 7.82 (s,1H); 8.57 (s,1H) ppm.

A suspension of 1.425 g of (6R,7R)-7-amino-3-[[(3-carbamoyl-7-(trifluoromethyl)pyrazolo[1,5-a]pyrimidin-5-yl)thio]methyl]-8-oxo-5-thia-1-azabicyclo[4.2.0]oct-2-ene-2-carboxylic acid is treated with 0.475 ml of N,O-bis-(trimethylsilyl)acetamide. After a clear solution has resulted 1.20 g of 2-amino-4-thiazolethioglyoxylic acid s-(2-benzothiazoly) ester are added and the mixture is stirred at 20° for 2.5 hours. The reaction mixture is filtered through a glass fibre filter, the filtrate is concentrated in a vacuum and partitioned between ethyl acetate and 0.25M potassium hydrogen carbonate solution. The aqueous phase is concentrated in a vacuum and then chromatographed on Opti-Up ($C_{12}$), with elution being carried out firstly with water and then with water/acetonitrile mixtures with increasing amounts of acetonitrile. The product fractions are combined, concentrated in a vacuum and adjusted to pH 2 with 3N hydrochloric acid, whereby a yellow precipitate forms. There is obtained (6R,7R)-7-(2-amino-4-thiazoleglyoxylamido)-3-[[(3-carbamoyl-7-(trifluoromethyl)pyrazolo[1,5-a]pyrimidin-5-yl)thio]methyl]-8-oxo-5-thia-1-azabicyclo[4.2.0]oct-2-ene-carboxylic acid.

$^1$H NMR (DMSO-$d_6$): δ 3.60 (d,J = 18 Hz,1H); 3.77 (d,J = 18 Hz,1H); 4.30 (d,J = 12 Hz,1H); 4.62 (d,J = 12 Hz,1H); 5.20 (d,J = 5 Hz,1H); 5.76 (dd,J = 8 Hz and J = 5 Hz,1H); 7.33 (s,1H); 7.40 (s,2H); 7.50 (s,1H); 7.80 (s,1H); 7.83 (s,1H); 8.57 (s,1H); 9.29 (d,J = 8 Hz,1H) ppm.

IR (KBr): 1775

MS: 629 $(M + H)^+$.

The (6R,7R)-7-(2-amino-4-thiazoleglyoxylamido)-3-[[(3-carbamoyl-5-(trifluoromethyl)pyrazolo[1,5-a]-pyrimidin-7 -yl]-thio]methyl]-8-oxo-5-thia-1-azabicyclo[4.2.0]oct-2-ene-2-carboxylic acid used as the starting compound for product d) can be prepared in analogy to Example 3a from the methyl 7-hydroxy-5-(trifluoromethyl)pyrazolo[1,5-a]pyrimidine-3-carboxylate described there.

There are obtained as intermediates:

methyl 7-chloro-5-(trifluoromethyl)pyrazolo[1,5-a]pyrimidine-3-carboxylate, $^1$H-NMR (DMSO-$d_6$): δ 3.87 (s,3H); 8.28 (s,(1H); 8.96 (s,1H) ppm,

methyl 7-mercapto-5-(trifluoromethyl)pyrazolo[1,5-a]pyrimidine-3-carboxylate, MS: 277 (M),

7-mercapto-5-(trifluoromethyl)pyrazolo[1,5-a]pyrimidine-3-carboxamide, $^1$H-NMR (DMSO-$d_6$): δ 6.7 (broad s,1H); 7.05 (s,1H); 7.40 (broad s,1H); 8.38 (s,1H) ppm,

(6R,7R)-7-amino-3-[[[3-carbamoyl-5-(trifluoromethyl)-pyrazolo[1,5-a]pyrimidin-7-yl]thio]methyl]-8-oxo-5-thia-1-azabicyclo[4.2.0]oct-2-ene-2-carboxylic acid, $^1$H-NMR (DMSO-$d_6$): δ 3.58 (d,J = 18 Hz,1H); 3.87 (d,J = 18 Hz,1H), 4.84 (d,J = 5 Hz,1H); 5.04 (d,J = 5 Hz,1H); 7.32 (s,1H); 7.76 (s,1H); 7.72 (s,1H); 8.77 (s,1H) ppm.

IR (KBr): 1778

MS: 475 $(M + H)^+$.

The (6R,7R)-7-(2-amino-4-thiazoleglyoxylamido)-3-[[(3-carbamoyl -5-(trifluoromethyl)pyrazolo[1,5-a]-pyrimidin-7-yl]thio]methyl] -8-oxo-5-thia-1-azabicyclo[4.2.0]oct-2-ene-2-carboxylic acid obtained has the following characteristics:

$^1$H-NMR (DMSO-$d_6$): δ 3.64 (d,J = 18 Hz,1H); 3.80 (d,J = 18 Hz,1H); 4.58 (broad s,2H); 5.26 (d,J = 5 Hz,1H); 5.78 (dd,J = 8 Hz and J = 5 Hz,1H); 7.30 (s,1H); 7.68 (s,1H); 7.74 (s,1H); 7.91 (s,1H); 8.78 (s,1H); 9.84 (d,J = 8 Hz,1H) ppm.

IR (KBr): 1778

MS: 629 $(M + H)^+$.

## Example 4

In analogy to Example 1 there is manufactured:

(6R,7R)-7-[(Z)-2-(2-Amino-4-thiazolyl)-2-[[1-[3-(3,4-dihydroxybenzoyl)carbazoyl]-1-methylethoxy]imino]-acetamido]-3-[[(2-carboxy-7-methyl-s-triazolo[1,5-a]pyrimidin-5-yl)thio]-methyl]-8-oxo-5-thia-1-azabicyclo-[4.2.0]oct-2-ene-2-carboxylic acid disodium salt.

$^1$H-NMR ($D_2O$): δ 1.64 (s,3H), 1.67 (s,3H); 2.69 (s,3H); 3.44 (d,J = 18 Hz,1H); 3.82 (d,J = 18 Hz,1H); 4.09 (d,J = 12 Hz,1H); 4.40 (d,J = 12 Hz,1H); 5.22 (d,J = 5 Hz,1H); 5.81 (d,J = 5 Hz,1H); 6.58 (d,J = 10 Hz,1H); 6.96 (s,1H); 7.10 (s,1H); 7.22 (d,J = 10 Hz,1H); 7.21 (s,1H) ppm.

IR (KBr): 1763.

The (6R,7R)-7-(2-amino-4-thiazoleglyoxylamido)-3-[[(2-carboxy-7-methyl-s-triazolo[1,5-a]pyrimidin-5-yl)-

thio]methyl]-8-oxo-5-thia-1-azabicyclo[4.2.0]oct-2-ene-2-carboxylic acid used here as the starting compound can be prepared as follows:

56 g of methyl 5-amino-4H-s-triazole-3-carboxylate in 150 ml of dimethylformamide are treated with 64 ml of diketene (50% in acetone) and the mixture is stirred at 100° for 3 hours, cooled to room temperature and filtered from a precipitated byproduct. The mother liquor is concentrated in a high vacuum and the residue is treated with water. The crystalline product is filtered off and recrystallized from water. There is obtained methyl 4,5,6,7-tetrahydro-7-hydroxy-7-methyl-5-oxo -s-triazolo[1,5-a]pyrimidine-2-carboxylate as white crystals of melting point 211-214°.

47.8 g of methyl 4,5,6,7-tetrahydro-7-hydroxy-7-methyl-5-oxo-s-triazolo[1,5-a]pyrimidine-2-carboxylate are introduced into 240 ml of sulphuric acid (100%). The temperature rises to 45°. It is stirred at this temperature for 15 minutes and subsequently cooled to 25°. The reaction mixture is poured on to ice and the precipitated product is filtered off, washed with water, acetone and ether and dried in a vacuum. There is obtained methyl 5-hydroxy-7-methyl-s-triazolo[1,5-a]pyrimidine-2-carboxylate as a white powder.
$^1$H-NMR (DMSO-$d_6$): δ 2.50 (s,3H); 3.89 (s,3H); 6.28 (s,1H); 13.1 (s,broad,1H) ppm.

From methyl 5-hydroxy-7-methyl-s-triazolo[1,5-a]pyrimidine-2-carboxylate there is prepared in analogy to Example 3:
Methyl 5-chloro-7-methyl-s-triazolo[1,5-a]pyrimidine-2-carboxylate,
$^1$H-NMR (DMSO-$d_6$): δ 2.92 (s,3H); 4.09 (s,3H); 7.11 (s,1H); 7.27 (s,1H) ppm. MS: 226 (M).
Methyl 5-mercapto-7-methyl-s-triazolo[1,5-a]pyrimidine-2-carboxylate,
$^1$H-NMR (DMSO-$d_6$): δ 2.49 (s,3H); 3.91 (s,3H); 7.12 (s,1H); 14.8 (s,broad,1H) ppm. MS: 224 (M).

2 g of methyl 5-mercapto-7-methyl-s-triazolo[1,5-a]pyrimidine-2-carboxylate are warmed in 20 m of 2N aqueous NaOH until all has dissolved. The reaction mixture is cooled, filtered and the mother liquor is adjusted to pH 2 with 6N aqueous hydrochloric acid. The yellow product is filtered off under suction and dried. There is obtained 5-mercapto-7-methyl-s-triazolo[1,5-ajpycimidine-2-carboxylic acid as a yellow powder.
$^1$H-NMR (DMSO-$d_6$): δ 2.44 (s,3H); 6.91 (s,1H) ppm.
MS: 210 (M).

From 5-mercapto-7-methyl-s-triazolo[1,5-a]pyrimidine-2-carboxylic acid there is prepared in analogy to Example 1:
(6R,7R)-7-Amino-3-[[(2-carboxy-7-methyl-s-triazolo[1,5-a]-pyrimidin-5-yl)thio]methyl]-8-oxo-5-thia-1-azabicyclo[4.2.0.]-oct-2-ene-2-carboxylic acid,
$^1$H-NMR (DMSO-$d_6$): δ 2.69 (s,3H); 3.50 (d,J = 18 Hz,1H); 3.73 (d,J = 18 Hz,1H); 4.12 (d,J = 12 Hz,1H); 4.56 (d,J = 12 Hz,1H); 4.76 (d,J = 5 Hz,1H); 4.96 (d,J = 5 Hz,1H); 7.32 (s,1H) ppm.
IR (KBr): 1797.
(6R,7R)-7-(2-Amino-4-thiazoleglyoxylamido)-3-[[2-carbamoyl-7-methyl-s-triazolo[1,5-a]pyrimidin-5-yl)thio]-methyl]-8-oxo-5-thia-1-azabicyclo[4.2.0]oct-2-ene-2-carboxylic acid,
$^1$H-NMR (DMSO-$d_6$): δ 2.69 (s,3H); 3.58 (d,J = 18 Hz,1H); 3.79 (d,J = 18 Hz,1H); 4.17 (d,J = 12 Hz,1H); 4.60 (d,J = 12 Hz,1H); 5.17 (d,J = 5 Hz,1H); 5.75 (dd,J = 8 Hz and J = 5 Hz,1H); 7.34 (s,1H); 7.40 (s,1H); 7.60 (s,1H); 9.77 (d,J = 8 Hz,1H); 13.8 (s,broad,1H) ppm.
IR (KBr): 1773.

Example 5

In analogy to Example 1 there is manufactured:
(6R,7R)-7-[(Z)-2-(2-Amino-4-thiazolyl)-2-[[1-[3-(3,4-dihydroxybenzoyl)carbazoyl]-1-methylethoxy]imino]-acetamido]-3-[[(2-carbamoyl-7-methyl-s-triazolo[1,5-a]pyrimidin-5-yl)thio]methyl]8-oxo-5-thia-1-azabicyclo-[4.2.0]oct-2-ene-2-carboxylic acid sodium salt.
$^1$H-NMR (DMSO-$d_6$): δ 1.46 (s,3H); 1.48 (s,3H); 2.69 (s,3H); 3.54 (d,J = 18 Hz,1H); 3.80 (d,J = 18 Hz,1H); 4.13 (d,J = 12 Hz,1H); 4.59 (d,J = 12 Hz,1H); 5.20 (d,J = 5 Hz,1H); 5.88 (dd,J = 8 Hz and J = 5 Hz,1H); 6.74 (d,J = 10 Hz,1H); 6.85 (s,1H); 7.23 (d,J = 10 Hz,1H); 7.29 (m,4H); 7.65 (s,1H); 8.20 (s,1H); 9.19 (s,1H); 9.21 (s,1H); 9.55 (s,1H); 9.63 (d,J = 8 Hz,1H); 10.00 (s,1H); 13.5 (s,broad,1H) ppm.
IR (KBr): 1772.
MS: 827 $(M+H)^+$.

The (6R,7R)-7-(2-amino-4-thiazoleglyoxylamido)-3-[[(2-carbamoyl-7-methyls-triazolo[1,5-a]pyrimidin-5-yl)thio]methyl]-8-oxo-5-thia-1-azabicyclo[4.2.0]oct-2-ene-2-carboxylic acid used here as the starting material can be prepared in analogy to Example 1 via the following intermediates:

Starting from methyl 5-mercapto-7-methyl-s triazolo[1,5-a]-pyrimidine-2-carboxylate (Example 5):
5-Mercapto-7-methyl-s-triazolo[1,5-a]pyrimidine-2-carboxamide ammonium salt,
$^1$H-NMR (DMSO-$d_6$): δ 2.38 (s,3H); 6.77 (s,1H); 7.01 (s,broad,4H); 7.55 (s,1H); 7.81 (s,1H) ppm.
MS: 209 (M).
(6R,7R)-7-Amino-3-[[(2-carbamoyl-7-methyl-s-triazolo[1,5-a]pyrimidin-5-yl)thio]methyl]-8-oxo-5-thia-1-azabicyclo-[4.2.0]oct-2-ene-2-carboxylic acid,
$^1$H-NMR (DMSO-$d_6$): δ 2.70 (s,3H); 3.51 (d,J=18 Hz,1H); 3.68 (d,J=18 Hz,1H); 4.10 (d,J=12 Hz,1H); 4.55 (d,J=12 Hz,1H); 4.81 (d,J=5 Hz,1H); 4.99 (d,J=5 Hz,1H); 7.31 (s,1H); 7.85 (s,1H); 8.21 (s,1H) ppm.
IR (KBr): 1782.

The (6R,7R)-7-(2-amino-4-thiazoleglyoxylamido)-3-[[(2-carbamoyl-7-methyl-s-triazolo[1,5-a]pyrimidin-5-yl)thio]methyl]-8-oxo-5-thia-1-azabicyclo[4.2.0]oct-2-ene-2-carboxylic acid obtained has the following characteristics:
$^1$H-NMR (DMSO-$d_6$): δ 2.70 (s,3H); 3.56 (d,J=18 Hz,1H); 3.79 (d,J=18 Hz,1H); 4.16 (d,J=12 Hz,1H); 4.61 (d,J=12 Hz,1H); 5.18 (d,J=5 Hz,1H); 5.76 (dd,J=8 Hz and J=5 Hz,1H); 7.33 (s,1H); 7.44 (s,2H); 7.80 (s,1H); 7.86 (s,1H); 8.22 (s,1H); 9.79 (d,J=8 Hz,1H) ppm.
IR (KBr): 1773.
MS: 570 $(M+H)^+$.

## Example 6

In analogy to Example 1 there is manufactured:
(6R,7R)-7-[(Z)-2-(2-Amino-4-thiazolyl)-2-[[1-[3-(3,4-dihydroxybenzoyl)carbazoyl]-1-methylethoxy]imino]-acetamido]-3-[[(2-methoxycarbonyl-7-methyl-s-triazolo[1,5-a]pyrimidin-5-yl)thio]-methyl]-8-oxo-5-oxo-5-thia-1-azabicyclo[4.2.0]oct-2-ene-2-carboxylic acid.
$^1$H-NMR ($D_2O$): δ 1.46 (s,3H), 1.48 (s,3H); 2.69 (s,3H); 3.55 (d,J=18 Hz,1H); 3.86 (d,J=18 Hz,1H); 3.94 (s,3H); 4.18 (d,J=12 Hz,1H); 4.60 (d,J=12 Hz,1H); 5.21 (d,J=5 Hz,1H); 5.90 (dd,J=8 Hz and J=5 Hz,1H); 6.74 (d,J=10 Hz,1H); 6.86 (s,1H); 7.23 (d,J=10 Hz,1H); 7.28 (s,1H); 7.35 (s,1H); 7.3 (s,broad,2H); 9.20 (s,broad,1H); 9.22 (s,1H); 9.54 (s,broad,1H); 9.64 (d,J=8 Hz,1H); 10.01 (s,1H) ppm.
IR (KBr): 1772.
MS: 842 $(M+H)^+$.

The (6R,7R)-7-(2-amino-4-thiazoleglyoxylamido)-3-[[(2-methoxycarbonyl -7-methyl-s-triazolo[1,5-a]-pyrimidin-5-yl)-thio]methyl]-8-oxo -5-thia-1-azabicyclo[4.2.0]oct-2-ene-2-carboxylic acid used here as the starting material can be prepared in analogy to Example 1 via the following intermediates:
Starting from methyl 5-mercapto-7-methyl-s-triazolo[1,5-a]pyrimidine-2-carboxylate (Example 4):
(6R,7R)-7-Amino-3-[[(2-methoxycarbonyl-7-methyl-s-triazolo[1,5-a]-pyrimidin-5-yl)thio]methyl]-8-oxo-5-thia-1-azabicyclo[4.2.0]oct-2-ene-2-carboxylic acid,
$^1$H-NMR (DMSO-$d_6$): δ 2.69 (s,3H); 3.49 (d,J=18 Hz,1H); 3.76 (d,J=18 Hz,1H); 3.94 (s,3H); 4.13 (d,J=12 Hz,1H); 4.56 (d,J=12 Hz,1H); 7.79 (d,J=5 Hz,1H); 4.98 (d,J=5 Hz,1H); 7.37 (s,1H) ppm.
IR (KBr): 1800.
MS: 437 $(M+H)^+$.

The (6R,7R)-7-(2-amino-4-thiazoleglyoxylamido)-3-[[(2-methoxycarbonyl-7-methyl-s-triazolo[1,5-a]-pyrimidin-5-yl)thio]methyl]-8-oxo-5-thia-1-azabicyclo[4.2.0]oct-2-ene-2-carboxylic acid obtained has the following characteristics:
$^1$H-NMR DMSO-$d_6$): δ 2.70 (s,3H); 3.57 (d,J=18 Hz,1H); 3.80 (d,J=18 Hz,1H); 3.94 (s,3H); 4.18 (d,J=12 Hz,1H); 4.60 (d,J=12 Hz,1H); 5.19 (d,J=5 Hz,1H); 5.76 (dd,J=8 Hz and J=5 Hz,1H); 7.37 (s,1H); 7.41 (s,2H); 7.80 (s,1H); 8.78 (d,J=8 Hz,1H) ppm.
IR (KBr): 170.
MS: 591 $(M+H)^+$.

## Example 7

In analogy to Example 1 there is manufactured:
(6R,7R)-7-[(Z)-2-(2-Amino-4-thiazolyl)-2-[[1-[3-(2,3-dihydroxybenzoyl)carbazoyl]-1-methylethoxy]imino]-acetamido]-3-(2-carbamoyl-5-methyl-s-triazolo[1,5-a]pyrimidin-7-yl)-thio]-methyl]-8-oxo-5-thia-1-azabicyclo-

[4.2.0]oct-2-ene-2-carboxylic acid.
$^1$H-NMR (DMSO-$d_6$): δ 1.49 (s,3H); 1.52 (s,3H); 2.61 (s,3H); 3.63 (d,J = 18 Hz,1H); 3.83 (d,J = 18 Hz,1H); 4.36 (d,J = 14 Hz,1H); 4.48 (d,J = 14 Hz, 1H); 5.24 (d,J = 5 Hz,1H); 5.90 (dd,J = 8 Hz and J = 5 Hz,1H); 6.70 (t,J = 5 Hz,1H); 6.87 (s,1H); 6.95 (d,J = 5 Hz,1H); 7.31 (s,2H); 7.35 (d,J = 5 Hz,1H); 7.39 (s,1H); 7.87 (s,1H); 8.17 (s,1H); 9.35 (s,1H); 9.47 (s,1H); 9.62 (s,1H); 9.65 (d,J = 8 Hz,1H); 10.55 (s,1H) ppm.
IR (KBr): 1774.
MS: (70 eV) 827 $(M + H)^+$.

The 1-[2-(aminooxy)-2-methylpropionyl]-2-(2,3-dihydroxybenzoyl)hydrazine hydrochloride used here as the starting material can be prepared from 2,3-dihydroxybenzoic acid in analogy to Example 1. It melts at 208-211° (dec.).

## Example 8

In analogy to Example 1 there is manufactured:
(6R,7R)-7-[(Z)-2-(2-Amino-4-thiazolyl)-2-[[[1-[3-(3,4-dihydroxybenzoyl)carbazoyl]-1-methylethoxy]imino]-acetamido]-3-[[[2-(cyclopropylcarbamoyl)-7-methyl-s-triazolo[1,5-a]pyrimidin-5-yl]thio]methyl]-8-oxo-5-thia-1-azabicyclo[4.2.0]-oct-2-ene-carboxylic acid.
$^1$H-NMR (DMSO-$d_6$): δ 0.70 (m,4H); 1.46 (s,3H); 1.49 (s,3H); 2.70 (s,3H); 2.90 (m,1H); 3.53 (d,J = 18 Hz,1H); 3.80 (d,J = 18 Hz,1H); 4.12 (d,J = 12 Hz,1H); 4.58 (d,J = 12 Hz,1H); 5.20 (d,J = 5 Hz,1H); 5.88 (dd,J = 8 Hz and J = 5 Hz,1H); 6.75 (d,J = 10 Hz,1H): 6.85 (s,1H); 7.23 (d,J = 10 Hz,1H); 7.30 (m,4H): 8.91 (d,J = 5 Hz,1H); 9.18 (s,1H); 9.21 (s,1H); 9.56 (s,1H); 9.64 (d,J = 8 Hz,1H): 10.0 (s,1H) ppm.
IR (KBr): 1777.
MS: 867 $(M + H)^+$.

The (6R,7R)-7-(2-amino-4-thiazoleglyoxylamino)-3-[[[2-(cyclopropylcarbamoyl)-7-methyl -s-triazolo[1,5-a]-pyrimidin-5-yl]thio]methyl]-8-oxo-5-thia-1-azabicyclo[4.2.0]oct-2-ene-2-carboxylic acid used as the starting compound can be prepared as follows:
1.12 g of methyl 5-mercapto-7-methyl-s-triazolo[1,5-a]pyrimidine-2-carboxylate (Example 4) are suspended in 4 g of cyclopropylamine and 6 ml of water. After 3 hours the reaction mixture is concentrated in a vacuum. The residue is dissolved in water and adjusted to pH 2.0 with aqueous hydrochloric acid. The precipitate obtained is filtered off and dried. There is obtained N-cyclopropyl-5-mercapto-7-methyl-s-triazolo[1,5-a]pyrimidine-2-carboxamide as a slightly yellow powder.
$^1$H-NMR: 0.68 (m,4H); 2.50 (s,3H); 2.88 (m,1H); 6.98 (s,1H); 8.63 (d,J = 5 Hz,1H); 14.7 (s,broad,1H).
MS: 249 (M).

The further processing is effected in analogy to Example 1 via the following intermediate:
(6R,7R)-7-Amino-3-[[[2-(cyclopropylcarbamoyl)-7-methyl-s-triazolo[1,5-a]pyrimidin-5-yl]thio]methyl]-8-oxo-5-thia-1-azabicyclo[4.2.0]oct-2-ene-2-carboxylic acid.
$^1$H-NMR (DMSO-$d_6$): δ 0.70 (m,4H); 2.70 (s,3H); 2.93 (m,1H); 3.45 (d,J = 18 Hz,1H); 3.70 (d,J = 18 Hz,1H); 4.08 (d,J = 12 Hz,1H); 4.53 (d,J = 12 Hz,1H): 4.77 (d,J = 5 Hz,1H); 4.96 (d,J = 5 Hz,1H); 7.31 (s,1H); 8.92 (d,J = 5 Hz,1H) ppm.
IR (KBr): 1799.
MS: 462 $(M + H)^+$.

The (6R,7R)-7-(1-amino-4-thiazoleglyoxylamino)-3-[[[2-(cyclopropylcarbamoyl)-7-methyl-s-triazolo[1,5-a]-pyrimidin-5-yl]thio]methyl]-8-oxo-5-thia-1-azabicyclo[4.2.0]oct-2-ene-2-carboxylic acid obtained has the following characteristics:
$^1$H-NMR (DMSO-$d_6$): δ 0.70 (m,4H); 2.70 (s,3H); 2.93 (m,1H); 3.56 (d,J = 18 Hz,1H); 3.78 (d,J = 18 Hz,1H); 4.14 (d,J = 12 Hz,1H); 4.58 (d,J = 12 Hz,1H); 5.17 (d,J = 5 Hz,1H); 5.75 (dd,J = 8 Hz and J = 5 Hz,1H); 7.32 (s,1H); 7.44 (s,broad,2H); 7.80 (s,1H); 8.92 (d,J = 5 Hz,1H); 9.78 (d,J = 8 Hz.1H) ppm.
IR (KBr): 1777.
MS: 616 $(M + H)^+$.

## Example 9

In analogy to Example 1 there is manufactured:
(6R,7R)-7-[[[(Z)-2-(2-Amino-4-thiazolyl)-2-[1-[3-(3,4-dihydroxybenzoyl)carbazoyl]-1-methylethoxy]imino]-

acetamido]-3-[[[3-carboxy-7-(trifluoromethyl)pyrazolo[1,5-a]pyrimidin-5-yl]thio]methyl]-8-oxo-5-thia-1-azabicyclo[4.2.0]oct-2-ene-2-carboxylic acid.
$^1$H-NMR (DMSO-$d_6$): $\delta$ 1.45 (s,3H); 1.48 (s,3H); 3.71 (d,J=18 Hz,1H): 3.84 (d,J=18 Hz,1H); 4.23 (d,J=12 Hz,1H); 4.62 (d,J=12 Hz,1H); 5.19 ((d,J=5 Hz,1H); 5.87 (dd,J=8 Hz and J=5 Hz,1H); 6.75 (d,J=10 Hz,1H); 6.65 (s,1H); 7.23 (d,J=10 Hz,1H); 7.29 (m,3H); 7.83 (s,1H); 8.59 (s,1H); 9.24 (s,1H); 9.62 (d,J=8 Hz,1H); 10.00 (s,1H) ppm.
IR (KBr): 1771.
MS: 881 $(M+H)^+$.

The (6R,7R)-7-(2-amino-4-thiazoleglyoxylamido)-3-[[(3-carboxy-7-(trifluoromethyl)pyrazolo[1,5-a]-pyrimidin-5-yl]thio]methyl]-8-oxo-5-thia-1-azabicyclo[4.2.0]oct-2-ene-2-carboxylic acid used here as the starting compound is obtainable from methyl 5-mercapto-7-(trifluoromethyl)pyrazolo[1,5-a]pyrimidine-3-carboxylate (Example 3) in analogy to Example 5 or 3. There are obtained as intermediates:
5-Mercapto-7-(trifluoromethyl)pyrazolo[1,5-a]pyrimidine-3-carboxylic acid.
$^+$H-NMR (DMSO-$d_6$): $\delta$ 7.28 (s,1H); 8.33 (s,1H) ppm.
MS: 263 (M).
(6R,7R)-7-Amino-3-[[[3-carboxy-7-(trifluoromethyl)pyrazolo-[1,5-a]pyrimidin-5-yl]thio]methyl]-8-oxo-5-thia-1-azabicyclo-[4.2.0]oct-2-ene-2-carboxylic acid.
$^1$H-NMR (DMSO-$d_6$): $\delta$ 3.72 (m,2H); 4.22 (d,J=12 Hz,1H); 4.58 (d,J= 12 Hz,1H); 4.79 (d,J=5 Hz,1H); 4.98 (d,J=5 Hz,1H); 7.84 (s,1H); 8.59 (s,1H) ppm.
IR (KBr): 1790.
MS: 476 $(M+H)^+$.

The (6R,7R)-7-(2-amino-4-thiazoleglyoxylamido)-3-[[(3-carboxy-7-(trifluoromethyl)pyrazolo[1,5-a]-pyrimidin-5-yl]thio]methyl]-8-oxo-5-thia-1-azabicyclo]4.2.0]oct-2-ene-2-carboxylic acid obtained has the following characteristics:
$^1$H-NMR (DMSO-$d_6$): $\delta$ 3.80 (m,2H); 4.25 (d,J= 12 Hz,1H); 4.59 (d,J=12 Hz,1H); 5.17 (d,J=5 Hz,1H); 5.74 (dd,J=8 Hz and J=5 Hz,1H); 7.40 (s,2H); 7.80 (s,1H); 7.86 (s,1H); 8.59 (s,1H); 9.77 (d,J=8 Hz,1H) ppm.
IR (KBr): 1778.
MS: 630 $(M+H)^1$.

Example 10

In analogy to Example 1 there is manufactured:
(6R,7R)-7-[(Z)-2-(2-Amino-4-thiazolyl)-2-[[1-[3-(3,4-dihydroxybenzoyl)carbazoyl]-1-methylethoxy]imino]-acetamido]-3-[[[3-(methoxycarbonyl)-7-methylpyrazolo[1,5-a]pyrimidin-5-yl]thio]methyl]-8-oxo-5-thia-1-azabicyclo[4.2.0]oct-2-ene-2-carboxylic acid.
$^1$H-NMR (DMSO-$d_6$): $\delta$ 1.46 (s,3H); 1.47 (s,3H); 2.67 (s,3H); 3.82 (s,3H). 3.87 (m,2H); 4.19 (d,J=12 Hz,1H); 4.55 (d,J=12 Hz,1H); 5.22 (d,J=5 Hz,1H); 5.86 (dd,J=8 Hz and J=5 Hz,1H); 6.74 (d,J=10 Hz,1H); 6.86 (s,1H); 7.16 (s,1H); 7.22 (d,J=10 Hz,1H); 7.28 8s,1H); 7.38 (s,broad,2H); 8.53 (s,1H); 9.20 (s,broad,2H); 9.56 (s,broad,1H); 9.62 (d,J=8 Hz,1H); 9.99 (s,1H) ppm.
IR (KBr): 1776.
MS: 841 $(M+H)^+$.

The (6R,7R)-7-(2-amino-4-thiazoleglyoxylamido)-3-[[[3-methoxycarbonyl)-7-methylpyrazolo[1,5-a]-pyrimidin-5-yl]thio]methyl]-8-oxo-5-thia-1-azabicyclo[4.2.0]oct-2-ene-2-carboxylic acid used here as the starting compound is obtainable from methyl 5-aminopyrazole-4-carboxylate in analogy to Example 5. There are obtained as intermediates:
Methyl 4,5,6,7-tetrahydro-7-hydroxy-7-methyl-5-oxo-pyrazolo[1,5-a]pyrimidine-3-carboxylate.
$^1$H-NMR (DMSO-$d_6$): $\delta$ 1.78 (s,3H); 2.74 (d,J=18 Hz,1H); 3.22 (d,J=18 Hz,1H); 3.72 (s,3H); 6.93 (s,1H); 7.77 (s,1H); 10.33 (s,1H) ppm.
MS: 225 (M).
Methyl 5-hydroxy-7-methyl-pyrazolo[1,5-a]pyrimidine-3-carboxylate.
$^1$H-NMR (DMSO-$d_6$): $\delta$ 2.50 (s,3H); 3.77 (s,3H); 6.13 (s,1H); 8.18 (s,1H) ppm.
MS: 207 (M).
Methyl 5-chloro-7-methyl-pyrazolo[1,5-a]pyrimidine-3-carboxylate.
$^1$H-NMR (DMSO-$d_6$): $\delta$ 2.76 (s,3H); 3.83 8s,3H); 7.43 (s,1H); 8.67 (s,1H) ppm.
MS: 225, 227 (M).
Methyl 5-mercapto-7-methyl-pyrazolo[1,5-a]pyrimidine-3-carboxylate.

$^1$H-NMR (DMSO-$d_6$): δ 2.50 (s,3H); 3.82 (s,3H); 6.93 (s,1H); 8.31 (s,1H) ppm.
MS: 223 (M).
(6R,7R)-7-Amino-3-[[[3-(methoxycarbonyl)-7-methylpyrazolo-[1,5-a]pyrimidin-5-yl]thio]methyl]-8-oxo-5-thia-1-azabicyclo[4.2.0]oct-2-ene-2-carboxylate.
$^1$H-NMR (DMSO-$d_6$): δ 2.67 (s,3H); 3.77 (m,2H); 3.83 (s,3H); 4.15 (d,J = 12 Hz,1H); 4.53 (d,J = 12 Hz,1H); 4.76 (d,J = 5 Hz,1H); 4.99 (d,J = 5 Hz,1H); 7.17 (s,1H); 8.52 (s,1H) ppm.
IR (KBr): 1781.

The (6R,7R)-7-(2-amino-4-thiazoleglyoxylamino)-3-[[[3-(methoxycarbonyl)-7-methylpyrazolo[1,5-a]-pyrimidin-5-yl]thio]methyl]-8-oxo-5-thia-1-azabicyclo-[4.2.0]oct-2-ene-carboxylic acid obtained has the following characteristics:
$^1$H-NMR (DMSO-$d_6$): δ 2.67 (s,3H); 3.82 (s,3H); 3.86 (m,2H); 4.21 (d,J = 12 Hz,1H); 4.56 (d,J = 12 Hz,1H); 5.18 (d,J = 5 Hz,1H); 5.74 (dd,J = 8 Hz and J = 5 Hz,1H); 7.18 (s,1H); 7.40 (s,2H); 7.80 (s,1H); 8.53 (s,1H); 9.76 (d,J = 8 Hz,1H) ppm.
IR: 1777.
MS: 590 $(M + H)^1$.

Example 11

In analogy to Example 1 there is manufactured:
(6R,7R)-7-[(Z)-2-(2-Amino-4-thiazolyl)-2-[[1-[3-(3,4-dihydroxybenzoyl)carbazoyl]-1-methylethoxyimino]-acetamido]-3-[[[2-carbamoyl-5-(trifluoromethyl)-s-triazolo[1,5-a]pyrimidin-7-yl]thio]methyl]-8-oxo-5-thia-1-azabicyclo[4.2.0]oct-2-ene-2-carboxylic acid.
$^1$H-NMR: (DMSO-$d_6$): δ 1.46 (s,3H); 1.49 (s, 3H); 3.65 (d,J = 18 Hz, 1H); 3.86 (d,J = 18 H, 1H); 4.59 (s,broad, 2H); 5.24 (d,J = 5Hz, 1H); 5.93 (d,d,J = 8 Hz and J = 5 Hz, 1H); 6.75 (d,J = 10 Hz, 1H); 6.87 (s,1H); 7.15 (d,J = 10 Hz, 1H); 7.28 (m,4H); 7.92 (s,1H); 8.03 (s,1H); 8.36 (s,1H); 9.22 (s,2H); 9.55 (s,1H); 9.68 (d,J = 8 Hz, 1H); 10.02 (s,1H) ppm.
IR (KBr): 1774
MS: $(M + H)^+$ 881.

The (6R,7R)-7-(2-amino-4-thiazoleglyoxylamido)-3-[[[2-carbamoyl-5-(trifluoromethyl)-s-triazolo[1,5-a]-pyrimidin-7-yl]thio]methyl]-8-oxo-5-thia-1-azabicyclo[4.2.0]oct-2-ene-2-carboxylic acid used here as the starting compound can be prepared as follows:

14.2 g of methyl 5-amino-4H-s-triazole-3-carboxylate are added at 60° to a mixture of 14.6 ml of ethyl trifluoroacetate in 50 g of polyphosphoric acid. The mixture is heated to 130° within one hour, whereby some ethanol is distilled off. The reaction mixture is stirred at 130° for 2 hours, cooled to 90° and treated with ice and ethyl acetate. The phases are separated and the organic phase is washed in succession in each case 2 times with 1N aqueous hydrochloric acid and saturated aqueous sodium chloride solution, dried over magnesium sulphate and concentrated. The residue is dissolved in aqueous bicarbonate solution and washed with ethyl acetate. The aqueous phase is acidified with aqueous hydrochloric acid and extracted with ethyl acetate. The ethyl acetate phase is washed with saturated aqueous sodium chloride solution, dried with magnesium sulphate and concentrated. The residue is treated with ether and cooled to 0°. The separated solid product is filtered off under suction and recrystallized from isopropanol. There is obtained methyl 5-hydroxy-7-(trifluoromethyl)-s-triazolo[1,5-a]pyrimidine-2-carboxylate as white crystals of melting point 232-234°. The desired isomeric methyl 7-hydroxy-5-(trifluoromethyl)-s-triazolo[1,5-a]-pyrimidine-2-carboxylate is present in the mother liquors and can be purified by crystallization from ethyl acetate. There are obtained white crystals of melting point 227-228°.

As further intermediates there are obtained in analogy to Example 3a:
Methyl 7-chloro-5-(trifluoromethyl)-s-triazolo[1,5-a]pyrimidine -2-carboxylate; white crystals of melting point 183-185°;
Methyl 7-mercapto-5-(trifluoromethyl)-s-triazolo[1,5-a]pyrimidine -2-carboxylate.
$^1$H NMR (DMSO-$d_6$): δ 3.93 (s,3H); 7.12 (s,1H) ppm;
7-Mercapto-5-(trifluoromethyl)-s-triazolo[1,5-a]pyrimidine-2-carboxamide;
$^1$H NMR (DMSO-$d_6$): δ 5.73 (s, 4H, broad), 7.25 (s,1H); 8.00 (s,1H, broad); 8.32 (s,1H, broad) ppm.
MS: (M) 263.
(6R,7R)-7-Amino-3-[[(2-carbamoyl -5-(trifluoromethyl)-s-triazolo[1,5-a]pyrimidin -7-yl)thio]methyl]-8-oxo-5-thia-1-azabicyclo[4.2.0]oct -2-ene-2-carboxylic acid.
$^1$H NMR (DMSO-$d_6$): δ 3.57 (d,J = 18 Hz, 1H); 3.78 (d,J = 18 Hz, 1H); 4.52 (d,J = 12 Hz, 1H); 4.62 (d,J = 12

Hz, 1H); 4.90 (d,J=5 Hz, 1H); 5.05 (d,J=5 Hz, 1H); 7.94 (s,1H); 8.04 (s,1H); 8.40 (s,1H) ppm.
IR (KBr): 1777
MS: $(M + H)^+$ 476.

The (6R,7R)-7-(2-amino-4-thiazoleglyoxylamido)-3-[[[2-carbamoyl -5-(trifluoromethyl)-s-thiazolo[1,5-a]-pyrimidin-7-yl]thio]methyl]-8-oxo-5-thia-1-azabicyclo[4.2.0]oct-2-ene-2-carboxylic acid obtained has the following characteristics:
$^1$H NMR (DMSO-$d_6$): δ 3.63 (d,J=18 Hz, 1H); 3.80 (d,J=18 Hz, 1H); 4.61 (m, 2H); 5.20 (d,J=5H, 1H); 5.80 (d,d,J=8 Hz and J=5 Hz, 1H); 7.41 (s,2H, broad); 7.81 (s,1H); 7.95 (s,1H); 8.03 (s,1H); 8.36 (s,1H); 9.82 (d,J=8 Hz, 1H) ppm.
IR (KBr): 1779
MS: $(M + H)^+$ 630.

## Example 12

In analogy to Example 1 there is manufactured:
(6R,7R)-7-[(Z)-2-[2-Amino-4-thiazolyl)-2-[[1-[3-(3,4-dihydroxybenzoyl)carbazoyl]-1-methylethoxy]imino]-acetamido]-3-[[[2-carbamoyl-7-(trifluoromethyl)-s-triazolo[1,5-a]pyrimidin-5-yl]thio]methyl]-8-oxo-5-thia-1-azabicyclo[4.2.0]oct-2-ene-2-carboxylic acid.
$^1$H NMR (DMSO-$d_6$): δ 3.55 (d,J=18 Hz, 1H); 3.81 (d,J=18 Hz, 1H); 4.18 (d J=12 Hz, 1H); 4.63 (d,J=12 Hz, 1H); 5.20 (d,J=5 Hz, 1H); 5.91 (dd, J=8 Hz and J=5 Hz, 1H); 6.75 (d,J=10 Hz, 1H); 6.85 (s,1H); 7.22 (d,J=10 Hz, 1H); 7.25 (m, 3H); 7.96 (s, 1H); 8.07 (s, 1H); 8.31 (s,1H); 9.19 (s,1H); 9.23 (s,1H); 9.55 (s, 1H); 9.64 (d,J=8 Hz, 1H); 10.02 (s,1H) ppm.
IR (KBr): 1775
MS: $(M + H)^+$ 881

The (6R,7R)-7-(2-amino-4-thiazoleglyoxylamido)-3-[[(2-carbamoyl-7-(trifluoromethyl]-s-triazolo[1,5-a]-pyrimidin-5-yl]thio]methyl]-8-oxo-5-thia -1-azabicyclo[4.2.0]oct-2-ene-2-carboyxlic acid used here as the starting compound can be prepared from methyl 5-hydroxy-7-(trifluoromethyl)-s-triazolo[1,5-a]pyrimidine-2-carboxylate in analogy to Example 3.

As further |ntermediates there are obtained:
Methyl 5-chloro-7-(trifluoromethyl)-s-triazolo-[1,5-a]pyrimidine-2-carboxylate; white crystals of melting point 163-164°.
$^1$H NMR (DMSO-$d_6$): δ 3.99 (s,3H); 8.42 (s,1H) ppm.
Methyl 5-mercapto-7-(trifluoromethyl) -s-triazolo[1,5-a]pyrimidine-2-carboxylate.
$^1$H NMR (DMSO-$d_6$): δ 3.92 (s,3H); 7.43 (s,1H); 7.47 (s,1H) ppm.
MS: (M) 278
5-Mercapto-7-(trifluoromethyl) -s-triazolo[1,5-a]pyr imidin-2-carboxamide.
$^1$H NMR (DMSO-$d_6$): δ 7.40 (s,1H); 7.93 (s,2H broad), ppm.
MS: (M) 263.
(6R,7R)-7-Amino-3-[[[2-carbamoyl -7-(trifluoromethyl)-s-triazolo[1,5-a]pyrimidin -5-yl]thio]methyl]-8-oxo-5-thia-1-azabicyclo[4.2.0]oct-2-ene-2-carboxylic acid.
$^1$H-NMR (DMSO-$d_6$): δ 3.49 (d,J=18 Hz, 1H); 3.72 (d,J=18 Hz, 1H); 4.14 (d,J=12 Hz, 1H); 4.58 (d,J=12 Hz, 1H): 4.79 (d,J=5 Hz, 1H); 4.97 (d,J=5 Hz, 1H); 7.97 (s,1H); 8.07 (s,1H); 8.31 (s,1H) ppm.
IR (KBr): 1779
MS: $(M + H)^+$ 476

The (6R,7R)-7-(2-amino-4-thiazoleglyoxylamido)-3-[[(2-carbamoyl-7-(trifluoromethyl]-s-triazolo[1,5-a]-pyrimidin-5-yl]thio]methyl]-8-oxo-5-thia -1-azabicyclo[4.2.0]oct-2-ene-2-carboxylic acid obtained has the following characteristics:
$^1$H NMR (DMSO-$d_6$): δ 3.58 (d,J=18 Hz, 1H); 3.70 (d,J=18 Hz, 1H); 4.19 (d,J=12 Hz, 1H); 4.63 (d,J=12 Hz, 1H); 5.16 (d,J=5 Hz, 1H); 5.77 (dd,J=8 Hz and J=5 Hz, 1H); 7.40 (s,2H); 7.80 (s,1H); 7.97 (s,1H); 8.09 (s,1H); 8.32 (s,1H); 9.67 (d,J=8 Hz, 1H) ppm.
IR (KBr): 1775
MS: $(M + H)^+$ 630.

Example 13

In analogy to Example 3a there is manufactured:

(6R,7R)-7-[(Z)-2-(2-Amino-4-thiazolyl)-2-[[1-(3-(3,4-dihydroxybenzoyl)carbazoyl]-1-methylethoxy]imino]-acetamido]-3-[[[2-carboxy-7,8-dihydro-6H-cyclopenta[e][1,2,4]triazolo-[1,5-a]pyrimidin-5-yl]thio]methyl]-8-oxo-5-thia-1-azabicyclo[4.2.0]oct-2-ene-2-carboxylic acid.

$^1$H NMR (DMSO-$d_6$): δ 1.46 (s,3H); 1.48 (s, 3H); 2.30 (m,2H); 3.30 (m overlapping from $H_2O$); 3.56 (d,J=18 Hz, 1H); 3.85 (d,J=18 Hz, 1H); 4.20 (d,J=12 Hz, 1H); 4.65 (d,J=12 Hz, 1H); 5.21 (d,J=5 Hz, 1H); 5.89 (dd,J=5 Hz and J=8 Hz, 1H); 6.73 (d,J=10 Hz, 1H); 6.85 (s,1H); 7.22 (d,J=10 Hz, 1H); 7.28 (m,3H); 9.20 (s,2H, broad), 9.56 (s,1H); 9.64 (d,J=8 Hz, 1H); 10.00 (s,1H) ppm.

The (6R,7R)-7-(2-amino-4-thiazoleglyoxylamido)-3-[[[2-carboxyl-7,8-dihydro-6H-cyclopenta[e][1,2,4]-triazolo[1,5-a]pyrimidin-5-yl]thio]methyl]-8-oxo -5-thia-1-azabicyclo[4.2.0]oct-2-ene-2-carboxylic acid used as the starting compound can be prepared as follows:

2.80 g of methyl 5-amino-4H-s-triazole-3-carboxylate are suspended in 80 ml of dimethylformamide and heated at 80° for 12 hours with 8.70 ml of ethyl cyclopentanone-2-carboxylate. The dimethylformamide is removed by evaporation in a vacuum and the residue is partitioned between ethyl acetate and 1N aqueous hydrochloric acid. The organic phase is washed with saturated aqueous sodium chloride solution and concentrated. For crystallization, the residual oil is mixed with ether. There is obtained methyl 5-(5-oxocyclopentanecarboxamido)-1H-1,2,4-triazole-3-carboxylate as crystals of melting point 163-164° (dec.).

$^1$H NMR (DMSO-$d_6$): δ 1.4-1.9 (m, 3H); 2.1-2.4 cm, 2H); 2.5-2.6 (m, 1H); 3.04 (t,J=10 Hz, 1H); 3.84 (s, 3H); 7.52 (s, 1H); 11.87 (s, 1H) ppm.

The cyclization of this product to methyl 7,8-dihydro-5-hydroxy-6H-cyclopenta[e][1,2,4]triazolo[1,5-a]-pyrimidine-2-carboxylate is effected in analogy to Example 4. There are obtained white crystals of melting point 252-253° (dec.).

$^1$H NMR (eDMSO-$d_6$): δ 2.17 (m, J=10 Hz, 1H); 2.50 (t,J=10 Hz, 2H); 3.16 (t,J=10 Hz, 2H); 3.88 (s,3H); 13.14 (s,1H, broad) ppm.

MS: (M) 234.

As further intermediates there are obtained in analogy to Example 3a:

Methyl 5-chloro-7,8-dihydro-6H-cyclopenta[e][1,2,4]triazolo[1,5-a]pyrimidine-2-carboxylate.

$^1$H NMR (DMSO-$d_6$): 2.34 (m,J=10 Hz, 1H); 3.10 (t,J=10 Hz, 2H); 3.46 (t,J=10 Hz, 2H); 3.96 (s,3H) ppm.

MS: (M) 252, 254

Methyl 7,8-dihydro-5-mercapto-6H-cyclopenta[e][1,2,4]triazolo[1,5-a]pyrimidine-2-carboxylate.

$^1$H NMR (DMSO-$d_6$): 2.17 (m,J=Hz, 2H); 2.83 (t,J=10 Hz, 1H); 3.23 (t, J=10 Hz, 2H); 3.91 (s,3H), 14.76 (s,1H, broad) ppm.

MS: (M) 250

7,8-Dihydro-5-mercapto-6H-cyclopenta[e][1,2,4]triazolo[1,5-a]pyrimidin-2-carboxylic acid.

$^1$H NMR (DMSO-$d_6$): δ 2.16 (m,J=10 Hz, 2H); 2.83 (t,J=10 Hz,2H); 3.23 (t,J=10 Hz, 2H).

MS: (M) 236

Therefrom there is obtained in analogy to Example 1:

(6R,7R)-7-Amino-3-[[[2-carboxy-7,8-dihydro -6H-cyclopenta[e][1,2,4]triazolo[1,5-a]pyrimidin -5-yl]thio]-methyl]-8-oxo-5-thia-1-azabicyclo[4.2.0]oct-2-ene-2-carboxylic acid.

$^1$H NMR ($D_2O$): δ 2.37 (m,J=10 Hz, 2H); 2.94 (t,J=10 Hz, 2H); 3.34 (t,J=10 Hz,2H); 3.48 (d,J=18 Hz, 1H); 3.78 (d,J=18 Hz, 1H); 4.13 (d,J=12 H, 1H); 4.59 (d,J=12 Hz, 1H); 4.77 (d,J=5 Hz, 1H); 5.05 (d,J=5 Hz, 1H) ppm.

IR (KBr): 1790

MS: $(M + H)^+$ 493

Therefrom there is obtained in analogy to Example 4:

(6R,7R)-7-(2-Amino-4-thiazoleglyoxylamido) -3-[[[2-carboxy-7,8-dihydro-6H-cyclopenta[e][1,2,4,triazolo[1,5-a]pyrimidin-5-yl]thio]methyl]-8-oxo -5-thia-1-azabicyclo[4.2.0]oct-2-ene-2-carboxylic acid.

$^1$H NMR (DMSO-$d_6$): δ 2.30 (m,J=10 Hz, 2H); 2.89 (t,J=10 Hz, 2H); 3.35 (t,J=10 Hz, 1H); 3.57 (d,J=18 Hz, 1H); 3.83 (d,J=18 Hz, 1H); 4.22 (d,J=12 Hz, 1H); 4.68 (d,J=12 Hz, 1H); 5.18 (d,J=5 Hz, 1H); 5.77 (dd,J=8 Hz and J=5 Hz, 1H); 7.40 (s,2H); 7.80 (s,1H); 9.77 (d,J=8 H,1H) ppm.

IR (KBr): 1774

MS: $(M + H)^+$ 603

Example 14

In analogy to Example 1 there is manufactured: (6R,7R)-7-[(Z)-2-(2-Amino-4-thiazolyl)-2-[[1-[3-(3,4-dihydroxybenzoyl)carbazoyl]-1-methylethoxy]imino-[acetamide]-3-[[[2-carbamoyl-7,8-dihydro-6H-cyclopenta[e][1,2,4]triazolo[1,5-a]pyrimidin-5-yl]thio]methyl]-8-oxo-5-thia-1-azabicyclo-[4.2.0]oct-2-ene-2-carboxylic acid.

$^1$HNMR (DMSO-$d_6$): $\delta$ 1.46 (s,3H); 1.48 (s,3H); 2.30 (m, 2H); 2.35 (m, 2H); 2.85 (m, 2H); 3.55 (d,J=18Hz, 1H); 3.83 (d,J=18 Hz, 1H); 4.17 (d,J=12 Hz, 1H); 4.66 (d,J=12 Hz, 1H); 5.21 (d,J=5 Hz, 1H); 5.89 (d,d,J=8 Hz and J=5 Hz, 1H); 6.24 (d,J=10 Hz, 1H); 6.85 (s, 1H); 7.22 (d,J=10 Hz, 1H); 7.28 (m, 3H); 7.83 (s,1H); 8.22 (s,1H), 9.19 (s,2H); 9.56 (s,1H); 9.78 (d,J=8 Hz, 1H); 10.00 (s,1H) ppm.

IR (KBr): 1772

MS: $(M + H)^+$ 853

The (6R,7R)-7-(2-amino-4-thiazoleglyoxylamido)-3-[[[2-carbamoyl -7,8-dihydro-6H-cyclopenta[e][1,2,4]-triazolo[1,5-a]pyrimidin -5-yl]thio]methyl]-8-oxo-5-thia-1-azabicyclo[4.2.0]oct-2-ene-2-carboxylic acid used as the starting material can be prepared as follows:

1.70 g of methyl 7,8-dihydro-5-mercapto-6H-cyclopenta[e][1,2,4]triazolo[1,5-a]pyrimidine-2-carboxylate (Example 13) are dissolved in 300 ml of 25% aqueous ammonia while stirring and left to stand at room temperature for 24 hours. The mixture is concentrated and acidified with aqueous hydrochloric acid. The separated solid is filtered off under suction, washed in succession with water and acetone and dried. There is obtained 7,8-dihydro-5-mercapto-6H-cyclopenta[e][1,2,4]triazolo[1,5-a]pyrimidine-2-carboxamide.

$^1$H NMR (DMSO-$d_6$): 2.16 (m,J=10 Hz, 2H); 2.84 (t,J=10 Hz, 2H); 3.24 (t,J=10 Hz, 2H); 7.81 (s,1H); 7.93 (s,1H); 14.73 (s,1H, broad) ppm.

MS: (M) 235

As further intermediates there are obtained in analogy to Example 1:

(6R,7R)-7-Amino-3-[[(2-carbamoyl-7,8-dihydro-6H-cyclopenta[e][1.2,4]triazolo[1,5-a]pyrimidin -5-yl)thio]-methyl]-8-oxo-5-thia-1-azabicyclo [4.2.0]oct-2-ene-2-carboxylic acid.

$^1$H NMR (DMSO-$d_6$): $\delta$ 2.34 (m, 2H); 2.88 (m, 2H); 3.31 (m, 2H overlapping from $H_2O$); 3.47 (d,J=18 Hz, 1H); 3.74 (d,J=18 Hz, 1H); 4.14 (d,J=12 Hz, 1H); 4.62 (d,J=12 Hz, 1H); 4.78 (d,J=5 Hz, 1H); 4.97 (d,J=5 Hz, 1H); 7.84 (s, 1H); 8.22 (s, 1H) ppm.

IR (KBr): 1776

MS: $(M + H)^+$ 448

(6R,7R)-7-(2-Amino-4-thiazoleglyoxylamido) -3-[[[2-carbamoyl-7,8-dihydro-6H-cyclopenta[e][1,2,4]triazolo-[1,5-a]pyrimidin -5-yl]thio]methyl]-8-oxo-5-thia-1-azabicyclo [4.2.0]oct-2-ene-2-carboxylic acid.

$^1$H NMR (DMSO-$d_6$): $\delta$ 2.33 (m, 2H); 2.91 (m, 2H); 3.34 (m, 2H overlapping from $H_2O$); 3.54 (d,J= 18 Hz, 1H); 3.81 (d,J=18 Hz, 1H); 4.19 (d,J=12 Hz, 1H); 4.68 (d,J=12 Hz, 1H); 5.19 (d,J=5 Hz, 1H); 5.77 (d,d,J=8 Hz and J=5 Hz, 1H); 7.43 (s,2H); 7.81 (s,1H); 7.85 (s,1H); 8.21 (s,1H); 9.80 (d,J,8 Hz, 1H); 13.75 (s,1H broad) ppm.

IR (KBr): 1777

MS: $(M + H)^+$ 602

Example 15

In analogy to Example 1 there is manufactured: (6R,7R)-7-[(Z)-2-(2-Amino-4-thiazolyl)-2-[[1-(3-[3,4-dihydroxybenzoyl)carbazoyl]-1-methylethoxy]imino]-acetamido]-3-[[[2-carboxy-7-(trifluoromethyl)-s-triazolo[1,5-a]pyrimidin-5-yl]thio]methyl]-8-oxo-5-thia-1-azabicyclo[4.2.0]oct-2-ene-2-carboxylic acid.

$^1$H NMR (DMSO-$d_6$): $\delta$ 1.46 (s,3H); 1.50 (s,3H); 3.55 (d,J=18 Hz, 1H); 3.84 (d,J=18 Hz, 1H); 4.20 (d,J=12 Hz, 1H); 4.56 (d,J=12 Hz, 1H); 5.21 (d,J=5 Hz, 1H); 5.80 (d.d,J=8 Hz and J=5 Hz, 1H); 6.74 (d,J=10 Hz, 1H); 6.88 (s, 1H); 7.23 (d,J=10 Hz, 1H); 7.31 (m,3H); 8.11 (s, 1H); 8.20 (s, 1H, broad); 9.24 (s, 1H); 9.54 (s, 1H, broad); 9.63 (d,J=8 Hz, 1H): 10.00 (s, 1H) ppm.

IR (KBr): 1774

MS: (70eV): 882 $(M + H)^+$

The (6R,7R)-7-(2-amino-4-thiazoleglyoxylamido)-3-[[[2-carboxy -7-(trifluoromethyl)-s-triazolo[1,5-a]-pyrimidin -5-yl]thio]methyl]-8-oxo-5-thia-1-azabicyclo[4.2.0]oct -2-ene-2-carboxylic acid can be prepared in analogy to Example 4 from methyl 5-mercapto-7-(trifluoromethyl)-s-triazolo[1,5-a]pyrimidine-2-carboxylate

(Example 12).

As further intermediates there are obtained:
5-Mercapto-7-(trifluoromethyl)-s-triazolo[1,5-a]pyrimidine-2-carboxylic acid.
$^1$H NMR (DMSO-$d_6$): δ 7.15 (s,1H) ppm.
IR (KBr) 1653
MS: (M + H)$^+$ 264.
(6R,7R)-7-Amino-3-[[[2-carboxy -7-(trifluoromethyl)-s-triazolo[1,5-a]pyrimidin -5-yl]thio]methyl] -8-oxo-5-thia-1-azabicyclo[4.2.0]oct -2-ene-2-carboxylic acid.
$^1$H NMR (DMSO-$d_6$): δ 3.52 (d,J=18 Hz, 1H); , 3.78 (d,J=18 Hz, 1H); 4.18 (d,J=12 Hz, 1H); 4.61 (d,J=12 Hz, 1H); 4.84 (d,J=5 Hz, 1H); 5.00 (d,J=5 Hz, 1H); 8.10 (s, 1H) ppm.
IR (KBr): 1787
MS: 477 (M + H)$^+$.

The (6R,7R)-7-(2-amino-4-thiazoleglyoxylamido)-3-[[[2-carboxy-7-(trifluoromethyl)-s-triazolo[1,5-a]-pyrimidin-5-yl[thio]methyl]-8-oxo-5-thia-1-azabicyclo[4.2.0]oct-2-ene-2-carboyxlic acid obtained has the following characteristics:
$^1$H NMR (DMSO-$d_6$): δ 3.58 (d,J=18 Hz, 1H); 3.80 (d,J=18 Hz, 1H); 4.21 (d,J=12 Hz, 1H); 4.64 (d,J=12 Hz, 1H); 5.16 (d,J=5 Hz, 1H); 5.78 (d,d,J=8 Hz and J=5 Hz, 1H); 7.39 (s, 2H); 7.78 (s, 1H); 8.12 (s, 1H); 9.78 (d,J=8 Hz, 1H) ppm.
IR (KBr): 1778
MS: 631 (M + H)$^+$

Example 16

In analogy to Example 2 there can be manufactured:

a) (6R,7R)-7-[(Z)-2-(2-Amino-4-thiazolyl)-2-[[1-[3-(3,4-diacetoxybenzoyl)carbazoyl]-1-methylethoxy]-imino]acetamido]-3-[[(3-carbamoyl-6,7-dihydro-5H-cyclopenta[d]pyrazolo-[1,5-a]pyrimidin-8-yl)thio]methyl]-8-oxo-5-thia-1-azabicyclo[4.2.0]oct-2-ene-2-carboxylic acid.
$^1$H NMR (DMSO-$d_6$): δ 1.65 (s, 6H); 2.0-2.44 (m, 8H); 2.86-3.30 (m, 4H); 3.86 (s, 2H); 4.48 (bs, 2H), 5.26 (d,J=5 Hz, 1H); 5.84-6.18 (m, 1H): 6.85-7.66 (m, 4H); 8.66 (s, 1H).

b) (6R,7R)-7-[(Z)-2-(2-Amino-4-thiazolyl)-2-[[1-[3-(3,4-dihydroxybenzoyl)carbazoyl]-1-methylethoxy]-imino]acetamido]-3-[[(3-carbamoyl-6,7-dihydro-5H-cyclopenta[d]pyrazolo[1,5-a]pyrimidin-8-yl)thio]methyl]-8-oxo-5-thia-1-azabicyclo[4.2.0]oct-2-ene-2-carboxylic acid hydrochloride.
$^1$H NMR (DMSO-$d_6$): δ 1.64 (s, 6H); 2.04-2.42 (m, 2H); 2.86-3.33 (m, 4H); 3.83 (s, 2H); 4.50 (bs, 2H), 5.28 (d,J=5 Hz, 1H); 5.84-6.15 (m, 1H); 6.88-7.67 (m, 4H); 8.67 (s, 1H).

c) (6R,7R)-7-[(Z)-2-(2-Amino-4-thiazolyl)-2-[[1-[3-(3,4-diacetoxybenzoyl)carbazoyl]-1-methylethoxy]-imino]acetamido]-3-[[(3-carbamoyl-5-methyl-pyrazolo[l 5-a]pyrimidin-7-yl)-thio]methyl]-8-oxo-5-thia-1-azabicyclo[4.2.0]oct-2-ene-2-carboxylic acid.
$^1$H NMR (DMSO-$d_6$): 1.65 (s, 6H); 2.33 (s, 6H); 2.69 (s, H); 3.84 (s, 2H); 4.55 (bs, 2H); 5.44 (d, J=5 Hz, 1H); 5.92-6.26 (m, 1H); 6.94-7.66 (m, 5H); 8.69 (s, 1H).

d) (6R,7R)-7-[(Z)-2-(2-Amino-4-thiazolyl)-2-[[1-[3-(3,4-diacetoxybenzoyl)carbazoyl]-1-methylethoxy]-imino]acetamido]-3-[[[3-(dimethylcarbamoyl)-5-methyl-pyrazolo[1,5-a]-pyrimidin-7-yl]thio]methyl]-8-oxo-5-thia-1-azabicyclo[4.2.0]-oct-2-ene-2-carboxylic acid.
$^1$H NMR (DMSO-$d_6$): 1.63 (s, 6H); 2.35 (s, 6H); 2.66 (s, 3H); 3.17 (s, 6H); 3.87 (s, 2H); 4.58 (bs, 2H); 5.46 (d,J=5 Hz, 1H); 5.90-6.30 (m, 1H); 6.92-7.66 (m, 5H): 8.60 (s, 1H).

e) (6R,7R)-7-[(Z)-2-(2-Amino-4-thiazolyl)-2-[1-[3-(3,4-dihydroxybenzoyl)carbazoyl]-1-methylethoxy]-imino]acetamido]-3-[[[3-(dimethylcarbamoyl)-5-methyl-pyrazolo[1,5-a]-pyrimidin-7-yl]thio]methyl]-8-oxo-5-thia-1-azabicyclo[4.2.0]-oct-2-ene-2-carboxylic acid hydrochloride.
$^1$H NMR (DMSO-$d_6$): 1.64 (s, 6H); 2.68 (s, 3H), 3.17 (s, 6H); 3.88 (s, 2H); 4.58 (bs, 2H): 5.48 (d,J=5 Hz, 1H); 5.92-6.28 (m, 1H); 6.94-7.67 (m, 5H); 8.59 (s, 1H).

Example 17

In analogy to Example 1 there is manufactured:
(6R,7R)-7-[(Z)-2-(2-amino-4-thiazolyl)-2-[[l-[3-(3chloro-4,5-dihydroxybenzoyl)carbazoyl]-1-methylethoxy]-

imino]acetamido]-3-[[(2-carbamoyl-5-methyl-s-triazolo[1,5-a]pyrimidin-7-yl)thio]methyl]-8-oxo-5-thia-1-azabicyclo[4.2.0]oct-2-ene-2-carboxylic acid sodium salt.
$^1H$ NMR (DMSO-$d_6$): $\delta$ 2,58 (s, 3H); 3,44 (d. J=18 Hz, 1H); 3,65 (d, J=18 Hz, 1H); 4.40 (d, J=14 Hz, 1H); 4.74 (d, J=14 Hz, 1H); 5.09 (d, J.5 Hz, 1H); 5.73 (m, 1H); 6.84 (s, 1H); 7.26 (d, J=2 Hz, 1H); 7.32 (d, J=2 z, 1H); 7.75 (s, 1H); 7.86 (s, 2H, broad); 8.17 (s, 1H, broad); 9.30 (s, 1H, broad); 9.54 (d, J=8 Hz, 1H) ppm.
IR (KBr): 1765.

The 1-(2-aminooxy-2-methylpropionyl)-2-(3-chlor-4,5-dihydroxybenzoyl)hydrazine-hydrochloride employed as starting material can be prepared starting from -4,5-dihydroxybenzoic acid in analogy to the disclosure given in Example 1.

Example 18

Manufacture of dry ampoules for intramuscular administration:

A lyophilizate of 1 g of the sodium salt of (6R,7R)-7-[(Z)-2-(2-amino-4-thiazolyl-2-[[1-[3-(3,4-dihydroxybenzoyl)carbazoyl]-1-methylethoxy]imino]acetamido]-3-[[(2-carbamoyl-5-methyl-s-triazolo[1,5-a]pyrimidin-7-yl)thio]methyl]-8-oxo-5-thia-1-azabicyclo[4.2.0]oct-2-ene-2-carboxylic acid is prepared in the usual manner and filled into an ampoule. Prior to the administration the lyophilizate is treated with 2.5 ml of a 2% aqueous lidocaine hydrochloride solution.

## Claims

1. Acyl derivatives of the general formula

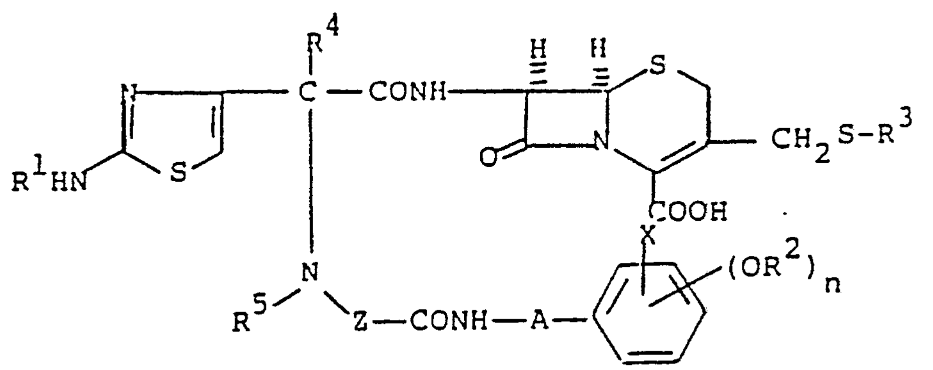

I

in which A signifies a group of the general formulae

-NHCO-, -NHCONHCO-, -NHCOCH=CH-,

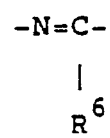

wherein $R^6$ represents hydrogen or lower alkyl; $R^1$ and $R^2$ in each case signify hydrogen or a protecting group, X signifies hydrogen, halogen, lower alkoxy, nitro or an additional group -O$R^2$, n signifies the number 1 or 2, $R^4$ and $R^5$ both signify hydrogen or together signify an additional bond and Z signifies a direct bond or carbonyl (where $R^4$ and $R^5$ both represent hydrogen) or a group of the formula -O-B-(where $R^4$ and $R^5$ together represent an additional bond) in which B signifies straight-chain, branched or cyclic lower alkylene; and wherein further $R^3$ signifies a substituted bicyclic group of the general formulae

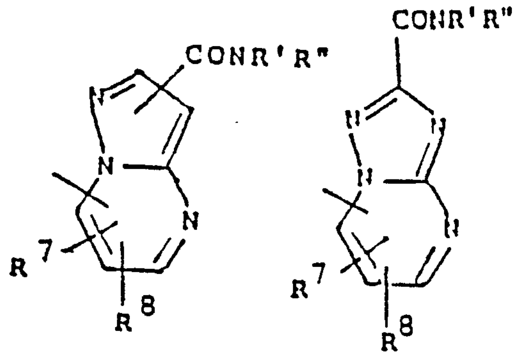

(a) (b)

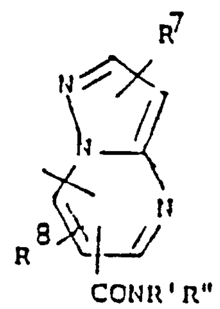

(c)

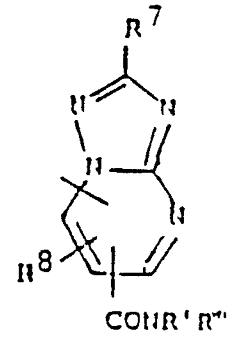

(d)

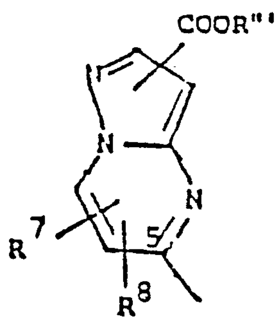

(e)

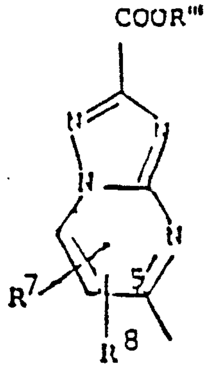

(f)

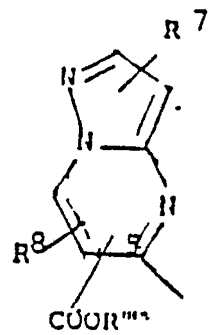

(g)

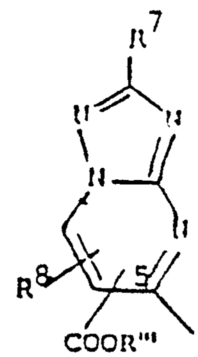

(h)

in which $R^7$ and $R^8$ each individually represent hydrogen, lower alkyl or trifluoromethyl or (in formulae (a), (b), (e) and (f)) together represent alkylene with 3 or 4 carbon atoms and R′, R″ and R‴ each individually represent hydrogen, lower alkyl or lower cycloalkyl; whereby compounds of formula I in which $R^4$ and $R^5$ together represent an additional bond are present in the syn-isomeric form or as a mixture with the anti-isomeric form in which the syn-isomeric form predominates,

as well as readily hydrolyzable esters and salts of these compounds and hydrates of the compounds of formula I or of their esters and salts.

2. Acyl derivatives according to claim 1 of the general formula

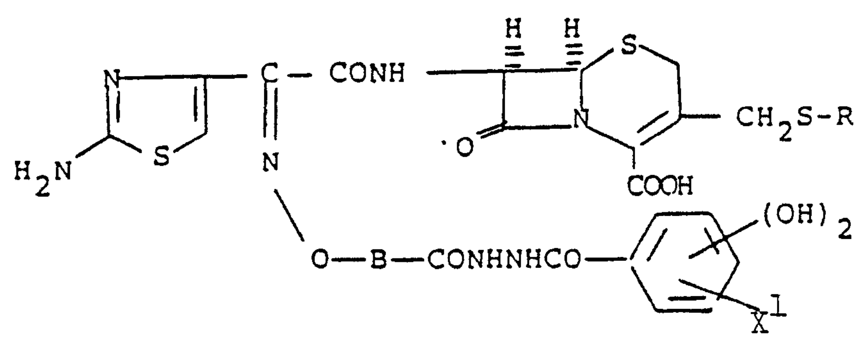

Ia

in which $R^3$ and B have the significance given in claim 1, and $X^1$ represents hydrogen or chlorine,

as well as readily hydrolyzable esters and salts of these compounds and hydrates of the compounds of formula Ia or of their esters and salts.

3. Compounds according to claim 1 or 2, wherein B represents the group $-C(CH_3)_2-$ or $-CH_2-$.

4. Compounds according to any one of claims 1-3, wherein the groups $-(OR^2)_2$ and $-(OH)_2$, respectively, are present in the 3,4-or 2,3-position.

5. Compounds according to any one of claims 1-4, wherein $R^3$ represents one of the groups

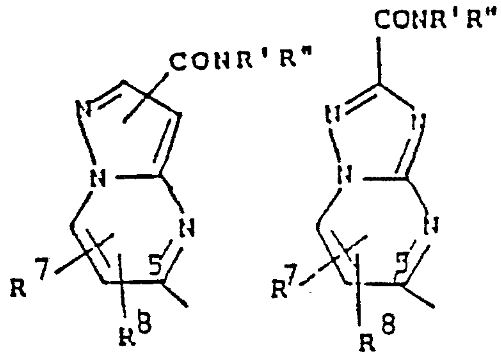
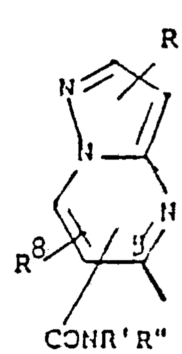
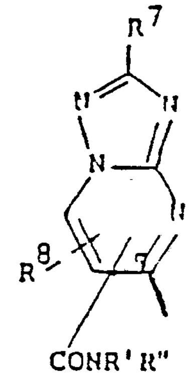

(a$^1$) (b$^1$) (c$^1$) (d$^1$)

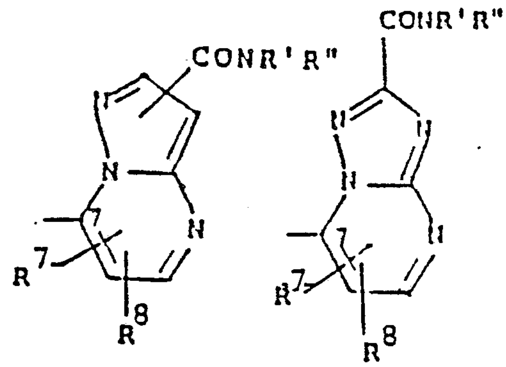
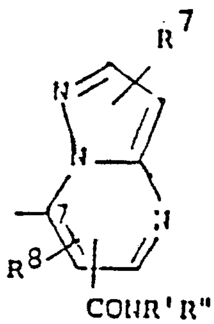
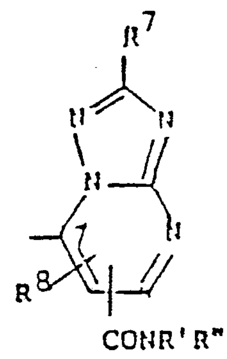

(a$^2$) (b$^2$) (c$^2$) (d$^2$)

wherein R', R", $R^7$ and $R^8$ have the significance given in claim 1.

6. Compounds according to any one of claims 1-4, wherein $R^3$ represents one of the groups

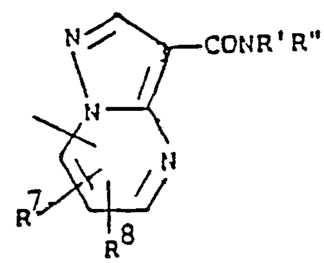
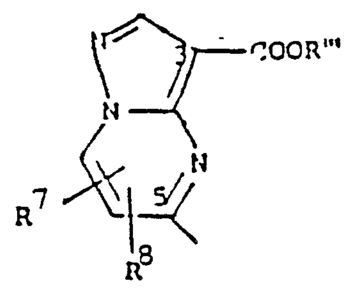

(a$^3$) (e$^3$)

wherein R', R", $R^7$ and $R^8$ have the significance given in claim 1.

7. Compounds according to any one of claims 1-4, wherein $R^3$ signifies a carbamoyl-substituted bicyclic group of the general formulae

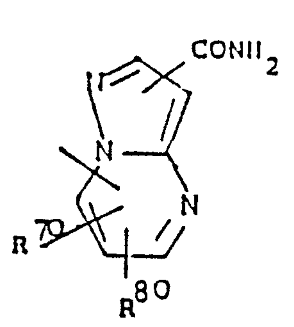

(a⁴)

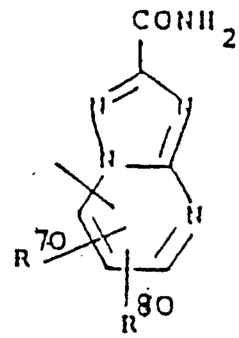

(b⁴)

wherein $R^{70}$ and $R^{80}$ each individually represent hydrogen, methyl or trifluoromethyl or together represent alkylene with 3 or 4 carbon atoms.

8. Compounds according to any one of claims 1-4, wherein $R^3$ signifies a substituted bicyclic group of the general formulae

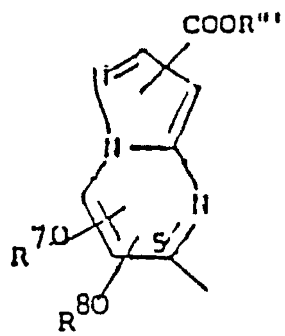

(e⁴)

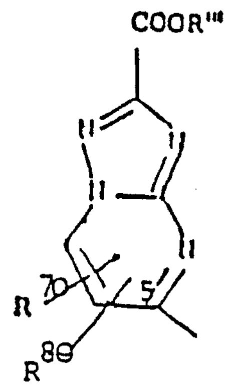

(f⁴)

wherein $R^{70}$ and $R^{80}$ each individually represent hydrogen, methyl or trifluoromethyl or together represent alkylene with 3 or 4 carbon atoms and $R'''$ signifies hydrogen or methyl.

9. Compounds according to any one of claims 1-4, wherein $R^3$ represents one of the groups

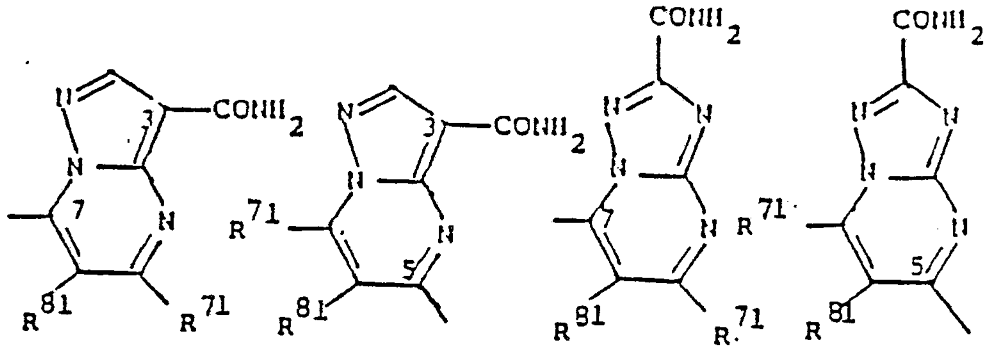

(a⁵) (a⁶) (b⁵) (b⁶)

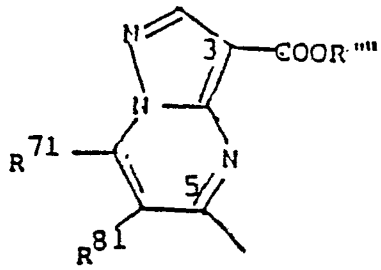

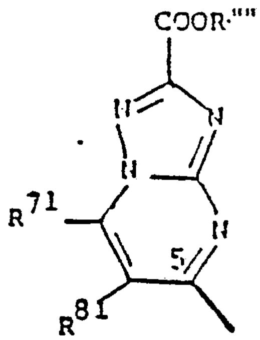

(e⁵) (f⁵)

wherein $R^{71}$ represents methyl or trifluoromethyl and $R^{81}$ represents hydrogen or $R^{71}$ and $R^{81}$ together represent tetramethylene and R'''' signifies hydrogen or methyl.

10. Compounds according to claim 7, wherein $R^3$ represents group ($a^4$) which is linked via position 5:

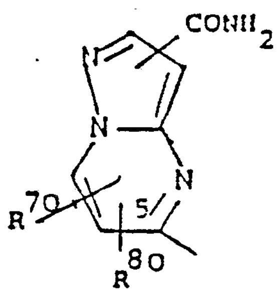

11. Compounds according to claim 7, wherein $R^3$ represents group ($a^4$) which is linked via position 7:

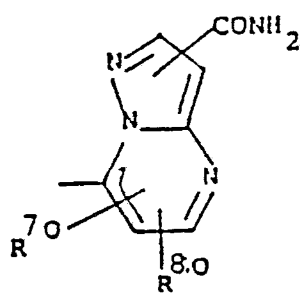

12. Compounds according to any one of claims 7, 10 and 11, wherein $R^3$ represents group ($a^4$) in which the carbamoyl group is present in position 3:

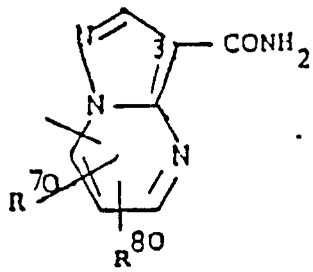

13. Compounds according to claim 11 or 12, wherein $R^3$ represents the group

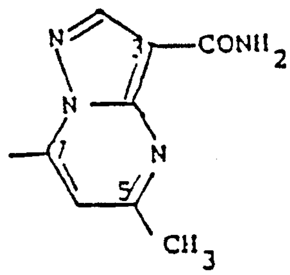

(a[7])

14. Compounds according to claim 7, wherein $R^3$ represents group ($b^4$) which is linked via position 5:

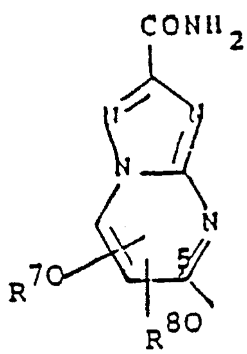

15. Compounds according to claim 7, wherein $R^3$ represents group ($b^4$) which is linked via position 7:

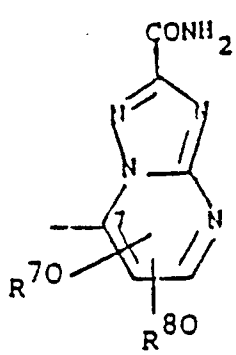

16. Compounds according to claim 14 or 15, wherein $R^3$ represents the group

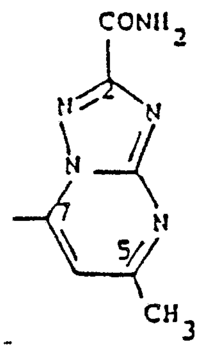

($b^7$)

17. (6R,7R)-7-[(Z)-2-(2-Amino-4-thiazoly])-2-[[1-[3-(3,4-dihydroxybenzoyl)carbazoyl]-1-methylethoxy]-imino]acetamido]-3-[[(2-carbamoyl-5-methyl-s-triazolo[1,5-a]pyrimidin-7-yl)thio]methyl]-8-oxo-5-thia-1-azabicyclo[4.2.0]oct-2-ene-2-carboxylic acid and its salts.

18. (6R,7R)-7-[(Z)-2-(2-Amino-4-thiazolyl)-2-[[1-[3-(3,4-dihydroxybenzoyl)carbazoyl]-1-methylethoxy]-imino]acetamido]-3-[[(3-carbamoyl-5-methylpyrazolo[1,5-a]pyrimidin-7-yl)thio]methyl]-8-oxo-5-thia-1-azabicyclo[4.2.0]oct-2-ene-2-carboxylic acid and its salts.

19. (6R,7R)-7-[(Z)-2-(2-Amino-4-thiazolyl)-2-[[1-[3-(3,4-dihydroxybenzoyl)carbazoyl]-1-methylethoxy]-imino] acetamido]-3-[[(2-carbamoyl-6.7-dihydro-5H-cyclopenta[d]-s-triazolo[1,5-a]pyrimidin-8-yl)thio]methyl]-8-oxo-5-thia-1-azabicyclo[4.2.0]oct-2-ene-2-carboxylic acid and its salts.

20. (6R,7R)-7-[(Z)-2-(2-Amino-4-thiazolyl)-2-[[1-[3-(3,4-dihydroxybenzoyl)carbazoyl]-1-methylethoxy]-imino]acetamido]-3-[[(2-carbamoyl-7-methyl-s-triazolo[1,5-a]pyrimidin-5-yl)thio]methyl]-8-oxo-5-thia-1-azabicyclo[4.2.0]oct-2-ene-2-carboxylic acid and its salts.

21. (6R,7R)-7-[(Z)-2-(2-Amino-4-thiazolyl)-2-[[1-[3-(3,4-dihydroxybenzoyl)carbazoyl]-1-methylethoxy]-imino]acetamido-3-[[[3-carbamoyl-7-(trifluoromethyl)pyrazolo[1,5-a]pyrimidin-5-yl]thio]methyl]-8-oxo-5-thia-1-azabicyclo[4.2.0]oct-2-ene-2-carboxylic acid and its salts.

22. (6R,7R)-7-[(Z)-2-(2-Amino-4-thiazolyl)-2-[[1-[3-(3,4-dihydroxybenzoyl)carbazoyl]-1-methylethoxy]-imino]acetamido]-3-[[[3-carbamoyl-5-(trifluoromethyl)pyrazolo[1,5-a]pyrimidin-7-yl]thio]methyl]-8-oxo-5-thia-1-azabicyclo[4.2.0]oct-2-ene-2-carboxylic acid and its salts.

23. (6R,7R)-7-[(Z)-2-(2-Amino-4-thiazolyl)-2-[[1-[3-(2,3-dihydroxybenzoyl)carbazoyl]-1-methylethoxy]-imino]acetamido]-3-[[(2-carbamoyl-5-methyl-s-triazolo[1,5-a]pyrimidin-7-yl)-thio]methyl]-8-oxo-5-thia-1-azabicyclo[4.2.0]oct-2-ene-2-carboxylic acid and its salts.

24. (6R,7R)-7-[(Z)-2-(2-Amino-4-thiazolyl)-2-[[1-[3-(3,4-dihydroxybenzoyl)carbazoyl]-1-methylethoxy]-imino]acetamido]-3-[[(2-carboxy-7-methyl-s-triazolo[1,5-a]pyrimidin-5-yl)thio]-methyl]-8-oxo-5-thia-1-azabicyclo[4.2.0]oct-2-ene-2-carboxylic acid.

(6R,7R)-7-[(Z)-2-(2-amino-4-thiazoalyl)-2-[[1-[3-(3,4-dihydroxybenzoyl)carbazoyl]-1-methylethoxy]imino] acetamido]-3-[[(2-methoxycarbonyl-7-methyl-s-triazolo[1,5-a]pyrimidin-5-yl)thio]-methyl]-8-oxo-5-thia-1-azabicyclo-[4.2.0]oct-2-ene-2-carboxylic acid.

(6R,7R)-7-[(Z)-2-(2-amino-4-thiazolyl)-2-[[1-[3-(3,4-dihydroxybenzoyl)carbazoyl]-1-methylethoxy]imino]-acetamido]-3-[[[2-(cyclopropylcarbamoyl)-7-methyl-s-triazolo[1,5-a]-pyrimidin-5-yl]thio]methyl]-8-oxo-5-thia-1-azabicyclo[4.2.0]-oct-2-ene-2-carboxylic acid,

(6R,7R)-7-[(Z)-2-(2-amino-4-thiazolyl)-2-[[1-[3-(3,4-dihydroxybenzoyl)carbazoyl]-1-methylethoxy]imino]-acetamido]-3-[[[3-(methoxycarbonyl)-7-methylpyrazolo[1,5-a]pyrimidin-5-yl]thio]methyl]-8-oxo-5-thia-1-azabicyclo[4.2.0]oct-2-ene-2-carboxylic acid,

(6R,7R)-7-[[[(Z)-2-(2-amino-4-thiazolyl)-2-[1-[3-(3,4-dihydroxybenzoyl)carbazoyl]-1-methylethoxy]imino]-acetamido]-3-[[[3-carboxy-7-(trifluoromethyl)pyrazolo[1,5-a]-pyrimidin-5-yl]thio]methyl]-8-oxo-5-thia-1-azabicyclo[4.2.0]-oct-2-ene-2-carboxylic acid and salts of any of these compounds.

25. (6R,7R)-7-[(Z)-2-(2-Amino-4-thiazolyl)-2-[[1-[3-(3,4-dihydroxybenzoyl)carbazoyl]-1-methylethoxy]-imino]acetamido]-3-[[[5-methyl-2-(methylcarbamoyl)-s-triazolo[1,5-a]pyrimidin-7-yl]thio]methyl]-8-oxo-5-thia-1-azabicyclo[4.2.0]oct-2-ene-2-carboxylic acid and its salts.

26. (6R,7R)-7-[(Z)-2-(2-Amino-4-thiazolyl)-2-[[1-[3-(3,4-dihydroxybenzoyl)carbazoyl]-1-methylethoxy]-imino]acetamido]-3-[[[2-(dimethylcarbamoyl)-5-methyl-s-triazolo[1,5-a]-pyrimidin-7-yl]thio]methyl]-8-oxo-5-thia-1-azabicyclo[4.2.0]-oct-2-ene-2-carboxylic acid and its salts.

27. (6R,7R)-7-[(Z)-2-(2-Amino-4-thiazolyl)-2-[[1-[3-(3,4-dihydroxybenzoyl)carbazoyl]-1-methylethoxy]-imino]acetamido]-3-[[[2-carbamoyl-5-(trifluoromethyl)-s-triazolo[1,5-a]pyrimidin-7-yl]thio]methyl]-8-oxo-5-thia-1-azabicyclo[4.2.0]oct-2-ene-2-carboxylic acid and its salts.

28. (6R,7R)-7-[(Z)-2-[2-Amino-4-thiazolyl)-2-[[1-[3-(3,4-dihydroxybenzoyl)carbazoyl]-1-methylethoxy]-imino]acetamido]-3-[[[2-carbamoyl-7-(trifluoromethyl)-s-triazolo[1,5-a]pyrimidin-5-yl]thio]methyl]-8-oxo-5-thia-1-azabicyclo[4.2.0]oct-2-ene-2-carboxylic acid and its salts.

29. (6R,7R)-7-[(Z)-2-(2-Amino-4-thiazolyl)-2-[[1-(3-(3,4-dihydroxybenzoyl)carbazoyl]-1-methylethoxy]-imino]acetamido]-3-[[[2-carboxy-7,8-dihydro-6H-cyclopenta[e][1,2,4]triazolo[1,5-a]pyrimidin-5-yl]thio]methyl]-8-oxo-5-thia-1-azabicyclo[4.2.0]oct-2-ene-2-carboxylic acid,

(6R,7R)-7-[(Z)-2-(2-amino-4-thiazolyl)-2-[[1-[3-(3,4-dihydroxybenzoyl)carbazoyl]-1-methylethoxy]imino[-acetamido]-3-[[[2-carbamoyl-7,8-dihydro-6H-cyclopenta[e][1,2,4]triazolo[1,5-a]pyrimidin-5-yl]thio]methyl]-8-oxo-5-thia-1-azabicyclo[4.2.0]oct-2-ene-2-carboxylic acid,

(6R,7R)-7-[(Z)-2-(2-amino-4-thiazolyl)-2-[[1-(3-[3,4-dihydroxybenzoyl)carbazoyl]-1-methylethoxy]imino]-acetamido]-3-[[[2-carboxy-7-(trifluormethyl)-s-triazolo[1,5-a]pyrimidin-5-yl]thio]methyl]-8-oxo-5-thia-1-azabicyclo[4.2.0]oct-2-ene-2-carboxylic acid,

(6R,7R)-7-[(Z)-2-(2-amino-4-thiazolyl)-2-[[1-[3-(3,4-diacetoxybenzoyl)carbazoyl]-1-methylethoxy]imino]-acetamido]-3-[[(3-carbamoyl-6,7-dihydro-5H-cyclopenta[d]pyrazolo[1,5-a]pyrimidin-8-yl)thio]methyl]-8-oxo-5-thia-1-azabicyclo[4.2.0]oct-2-ene-2-carboxylic acid,

(6R,7R)-7-[(Z)-2-(2-amino-4-thiazolyl)-2-[[1-[3-(3,4-dihydroxybenzoyl)carbazoyl]-1-methylethoxy]imino]-acetamido]-3-[[(3-carbamoyl-6,7-dihydro-5H-cyclopenta[d] pyrazolo[1,5-a]pyrimidin-8-yl)thio]methyl]-8-oxo-5-thia-1-azabicyclo[4.2.0]oct-2-ene-2-carboxylic acid.

(6R,7R)-7-[(Z)-2-(2-amino-4-thiazolyl)-2-[[1-[3-(3,4-diacetoxybenzoyl)carbazoyl]-1-methylethoxy]imino]-acetamido]-3-[[(3-carbamoyl-5-methyl-pyrazolo[1,5-a]pyrimidin-7-yl)thio]methyl]-8-oxo-5-thia-1-azabicyclo-[4.2.0]oct-2-ene-2-carboxylic acid,

(6R,7R)-7-[(Z)-2-(2-amino-4-thiazolyl)-2-[[1-[3-(3,4-diacetoxybenzoyl)carbazoyl]-1-methylethoxy]imino]-acetamido]-3-[[[3-(dimethylcarbamoyl)-5-methyl-pyrazolo[1,5-a]pyrimidin-7-yl]thio]methyl]-8-oxo-5-thia-1-azabicyclo[4.2.0]-oct-2-ene-2-carboxylic acid,

(6R,7R)-7-[(Z)-2-(2-amino-4-thiazolyl)-2-[[1-[3-(3,4-dihydroxybenzoyl)carbazoyl]-1-methylethoxy]imino]-acetamido]-3-[[[3-(dimethylcarbamoyl)-5-methyl-pyrazolo[1,5-a]pyrimidin-7-yl]thio]methyl]-8-oxo-5-thia-1-azabicyclo[4.2.0]-oct-2-ene-2-carboxylic acid and salts of any of these compounds.

30. (6R,7R)-7-[(Z)-2-(2-amino-4-thiazolyl)-2-[[1-[3(3-chloro-4,5-dihydroxybenzoyl)carbazoyl]-1-methylethoxy]imino]acetamido]-3-[[(2-carbamoyl-5-methyl-s-triazolo[1,5-a]pyrimidin-7-yl)thio]methyl]-8-oxo-5-thia-1-azabicyclo[4.2.0]oct-2-ene-2-carboxylic acid and its salts.

31. Compounds according to any one of claims 1-30 as pharmaceutically active substances.

32. Compounds according to any one of claims 1-30 as pharmaceutically active substances for the treatment and prophylaxis of infectious diseases.

33. A process for the manufacture of the compounds in accordance with any one of claims 1-30, which process comprises

(a) reacting a compound of the general formula

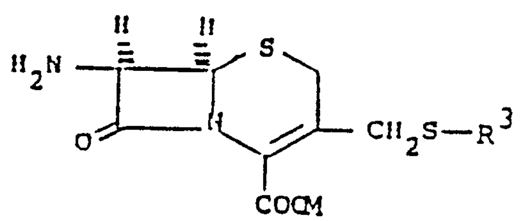

III

in which $R^3$ has the significance given in claim 1 and M represents hydrogen or an ester protecting group, with a carboxylic acid of the general formula

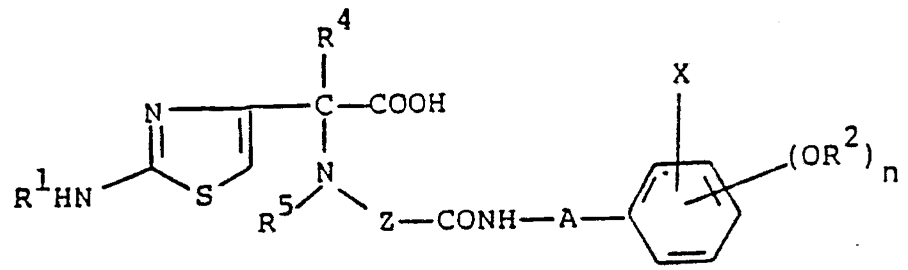

IV

in which $R^1$, $R^2$, $R^4$, $R^5$, X, Z, A and n have the significance given in claim 1,

or one of its reactive derivatives and, if desired, cleaving off any protecting groups, or

(b) reacting a compound of the general formula

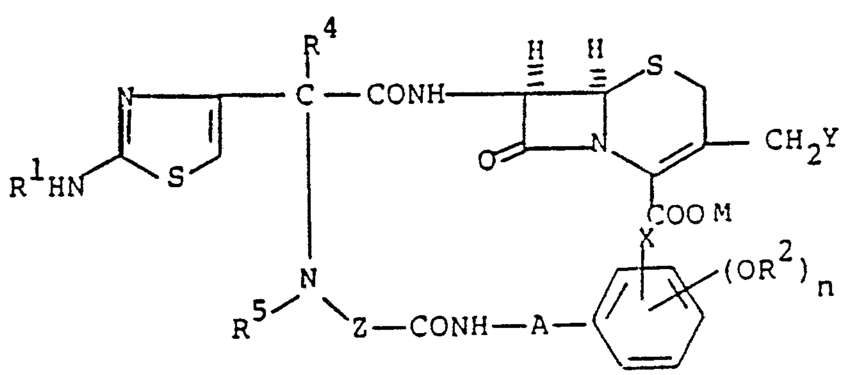

V

in which $R^1$, $R^2$, $R^4$, $R^5$, X, Z, A and n have the significance given in claim 1, M has the significance given above and Y represents a leaving group,

with a compound of the general formula

$HSR^3$ VI

in which $R^3$ has the significance given in claim 1,

and if desired. cleaving off any protecting groups, or

(c) reacting a compound of the general formula

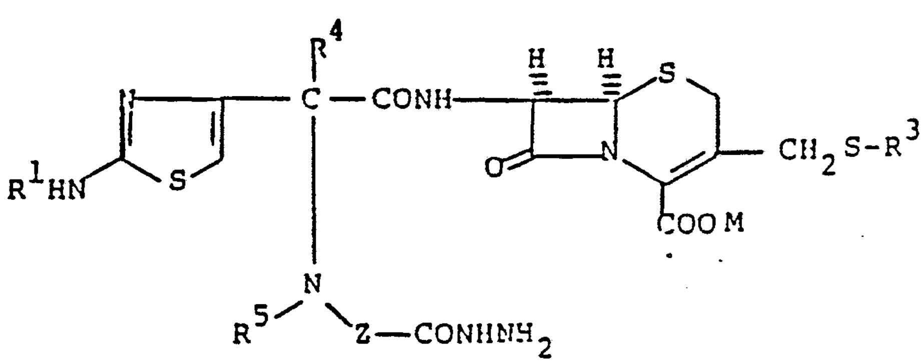

VII

in which $R^1$, $R^3$, $R^4$, $R^5$ and Z have the significance given in claim 1 and M has the above significance,

with a compound of the general formulae

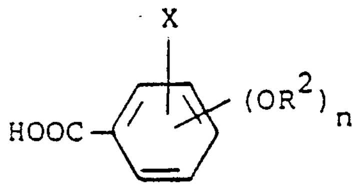

VIIIa

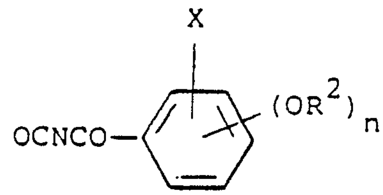

VIIIb

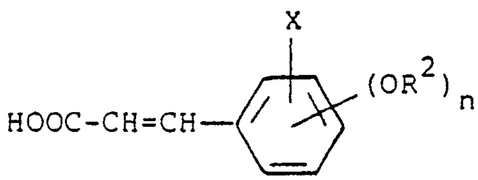

VIIIc

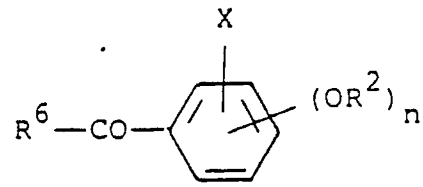

VIIId

in which $R^2$, $R^6$, X and n have the significance given in claim 1,

or a reactive derivative of one of the carboxylic acids VIIIa and VIIIc and, if desired, cleaving off any protecting groups, or

(d) for the manufacture of compounds of formula I in which $R^4$ and $R^5$ together represent an additional bond, reacting a compound of the general formula

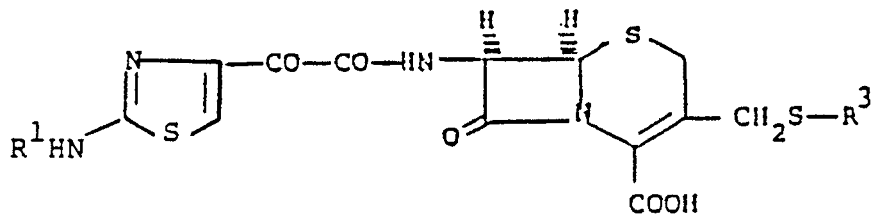

IX

wherein $R^1$ and $R^3$ have the significance given in claim 1,

with a salt of a compound of the general formula

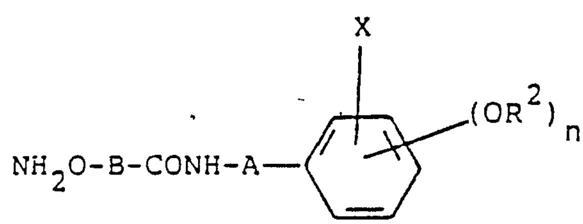

X

in which $R^2$, A, B, X and n have the significance given in claim 1,

or

(e) for the manufacture of a readily hydrolyzable ester of a compound of formula I, subjecting a carboxylic acid of formula I to an appropriate esterification, or

(f) for the manufacture of salts and hydrates of a compound of formula I or of hydrates of these salts, converting a compound of formula I into a salt or hydrate or into a hydrate of this salt.

34. Pharmaceutical preparations, which contain a compound in accordance with any one of claims 1-30.

35. Pharmaceutical preparations for the treatment and prophylaxis of infectious diseases, said preparations containing a compound in accordance with any one of claims 1-30.

36. The use of compounds in accordance with any one of claims 1-30 in the treatment or prophylaxis of illnesses.

37. The use of compounds in accordance with any one of claims 1-30 in the treatment or prophylaxis of infectious diseases.

38. The use of compounds in accordance with any one of claims 1-30 in the manufacture of medicaments for the treatment or prophylaxis of infectious diseases.

Claims for the following Contracting States: GR, ES

1. Process for the manufacture of acyl derivatives of the general formula

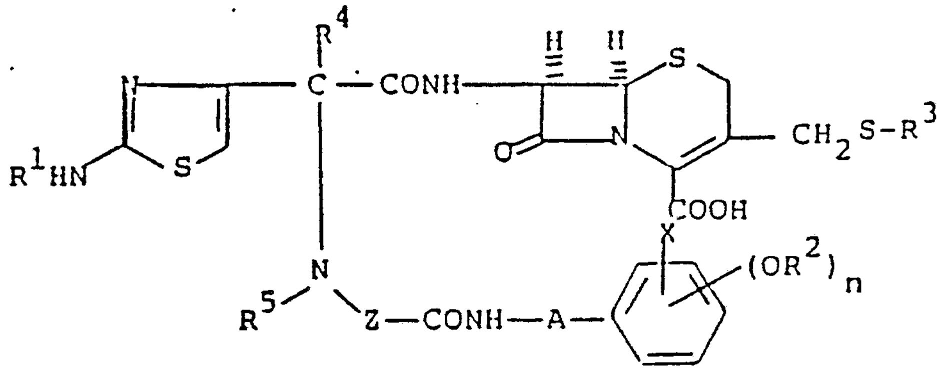

I

in which A signifies a group of the general formulae

-NHCO-, -NHCONHCO-, -NHCOCH = CH-,

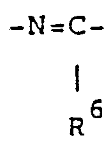

wherein $R^6$ represents hydrogen or lower alkyl; $R^1$ and $R^2$ in each case signify hydrogen or a protecting group, X signifies hydrogen, halogen, lower alkoxy, nitro or an additional group $-OR^2$, n signifies the number 1 or 2, $R^4$ and $R^5$ both signify hydrogen or together signify an additional bond and Z signifies a direct bond or carbonyl (where $R^4$ and $R^5$ both represent hydrogen) or a group of the formula -O-B-(where $R^4$ and $R^5$ together represent an additional bond) in which B signifies straight-chain, branched or cyclic lower alkylene; and wherein further $R^3$ signifies a substituted bicyclic group of the general formulae

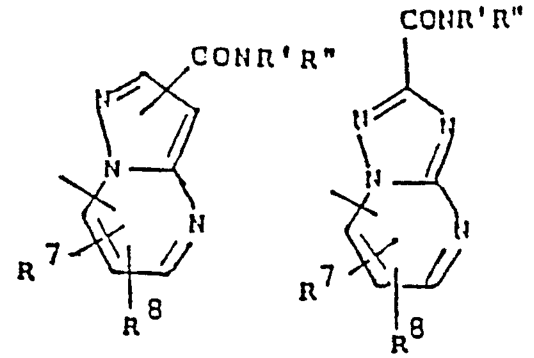

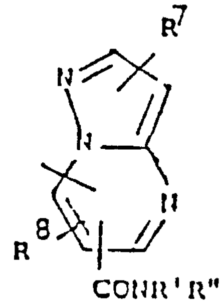

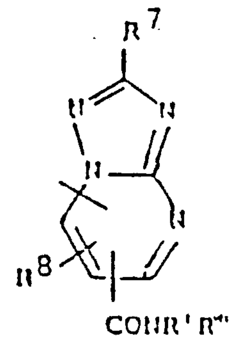

(a) (b) (c) (d)

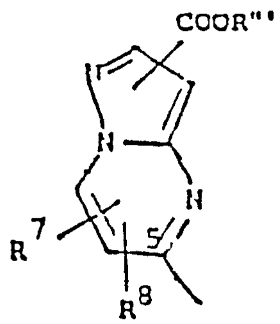

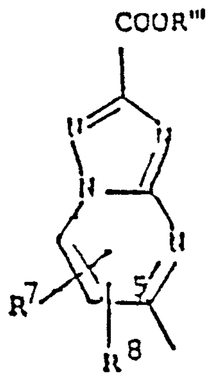

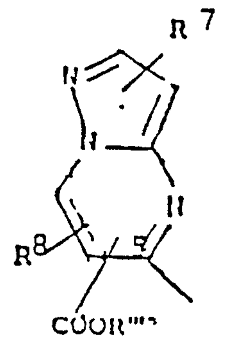

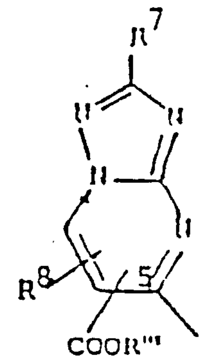

(e) (f) (g) (h)

in which $R^7$ and $R^8$ each individually represent hydrogen, lower alkyl or trifluoromethyl or (in formulae (a), (b), (e) and (f)) together represent alkylene with 3 or 4 carbon atoms and R′, R″ and R‴ each individually represent hydrogen, lower alkyl or lower cycloalkyl; whereby compounds of formula I in which $R^4$ and $R^5$ together represent an additional bond are present in the syn-isomeric form or as a mixture with the anti-isomeric form in which the syn-isomeric form predominates,

as well as readily hydrolyzable esters and salts of these compounds and hydrates of the compounds of formula I or of their esters and salts, which process comprises

(a) reacting a compound of the general formula

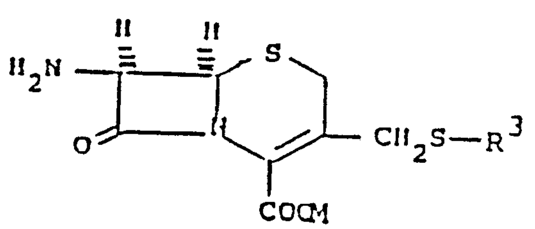

III

in which $R^3$ has the significance given above and M represents hydrogen or an ester protecting group,

with a carboxylic acid of the general formula

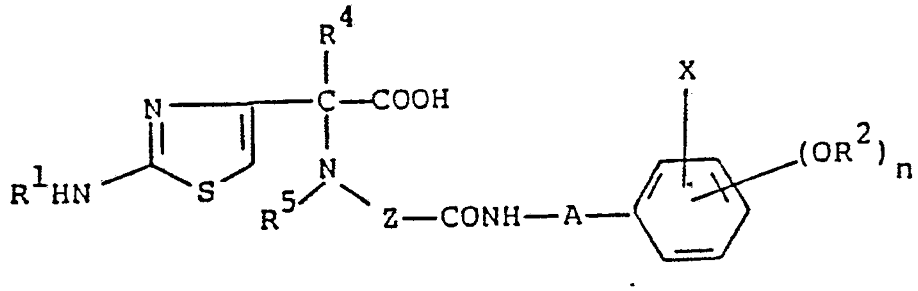

IV

in which $R^1$, $R^2$, $R^4$, $R^5$, X, Z, A and n have the significance given above,

or one of its reactive derivatives and, if desired, cleaving off any protecting groups, or
(b) reacting a compound of the general formula

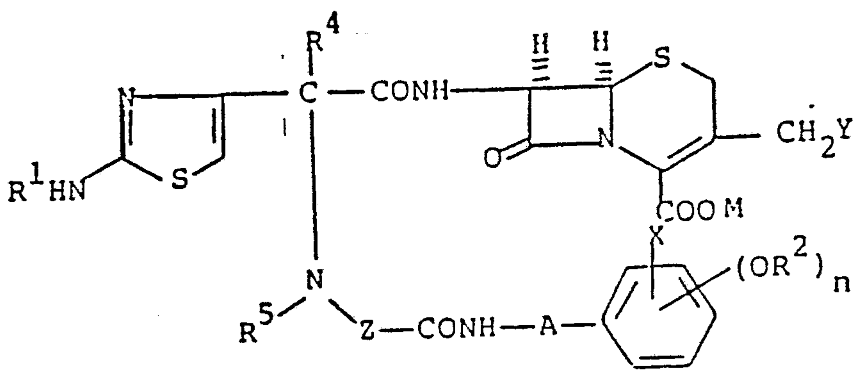

V

in which $R^1$, $R^2$, $R^4$, $R^5$, X, Z, A and n have the significance given above, M has the significance given above and Y represents a leaving group,

with a compound of the general formula

$HSR^3$ VI

in which $R^3$ has the significance given above,

and, if desired, cleaving off any protecting groups, or
(c) reacting a compound of the general formula

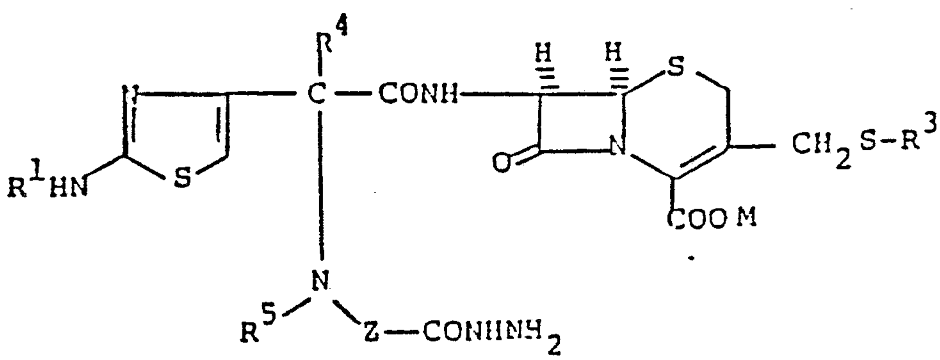

VII

in which $R^1$, $R^3$, $R^4$, $R^5$ and Z have the significance given above and M has the above significance,

with a compound of the general formulae

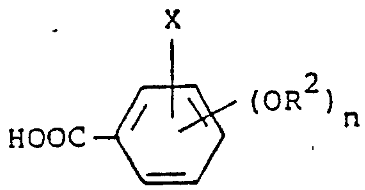

VIIIa

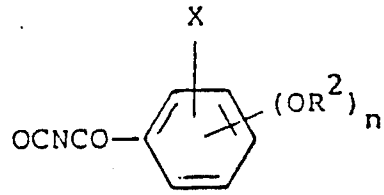

VIIIb

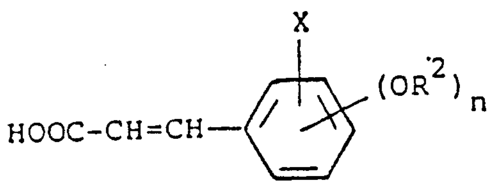

VIIIc

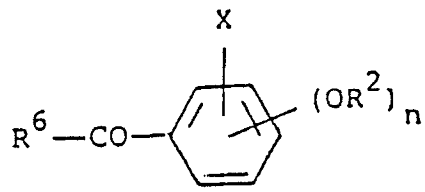

VIIId

in which $R^2$, $R^6$, X and n have the significance given above,
or a reactive derivative of one of the carboxylic acids VIIIa and VIIIc and, if desired, cleaving off any protecting groups, or

(d) for the manufacture of compounds of formula I in which $R^4$ and $R^5$ together represent an additional bond, reacting a compound of the general formula

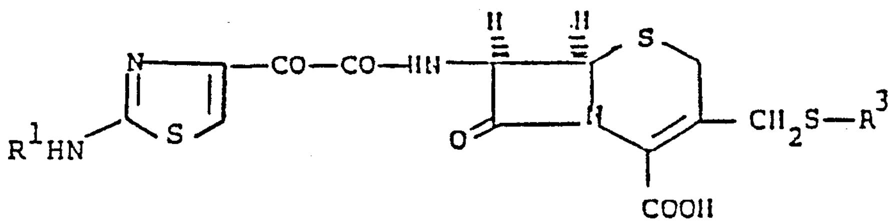

IX

wherein $R^1$ and $R^3$ have the significance given above,

with a salt of a compound of the general formula

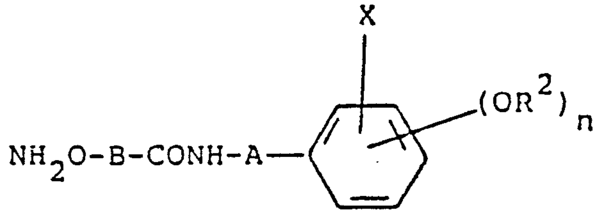

X

in which $R^2$, A, B, X and n have the significance given above,

or

(e) for the manufacture of a readily hydrolyzable ester of a compound of formula I, subjecting a carboxylic acid of formula I to an appropriate esterification, or

(f) for the manufacture of salts and hydrates of a compound of formula I or of hydrates of these salts, converting a compound of formula I into a salt or hydrate or into a hydrate of this salt.

2. Process for the manufacture of acyl derivatives according to claim 1 of the general formula

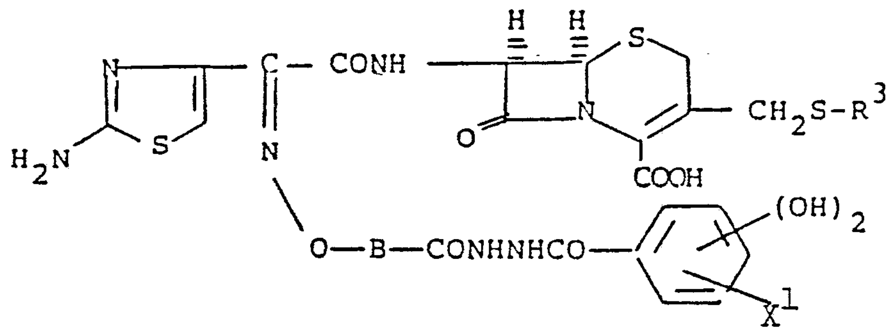

Ia

in which $R^3$ and B have the significance given in claim 1, and $X^1$ represents hydrogen or chlorine,

as well as readily hydrolyzable esters and salts of these compounds and hydrates of the compounds of formula Ia or of their esters and salts which process is characterized in that correspondingly substituted starting materials are employed.

3. Process for the manufacture of compounds according to claim 1 or 2, wherein B represents the group $-C(CH_3)_2$-or $-CH_2$-which process is characterized in that correspondingly substituted starting materials are employed.

4. Process for the manufacture of compounds according to any one of claims 1-3, wherein the groups --$(OR^2)_2$ and $-(OH)_2$, respectively, are present in the 3,4-or 2,3-position which process is characterized in that correspondingly substituted starting materials are employed.

5. Process for the manufacture of compounds according to any one of claims 1-4 which process is characterized in that correspondingly substituted starting materials are employed and, moreover, that one of the process alternatives (a), (b), (d), (e) and (f) is carried out.

6. Process for the manufacture of compounds according to claim 5, wherein $R^3$ represents one of the groups

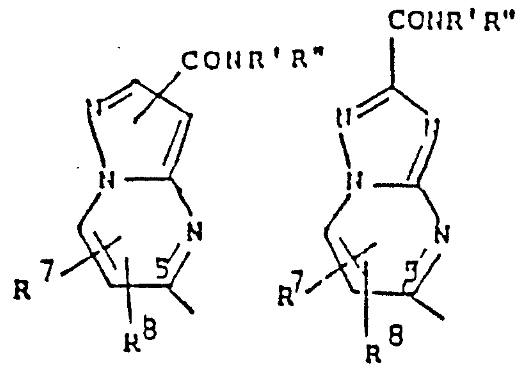
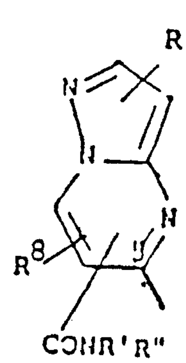
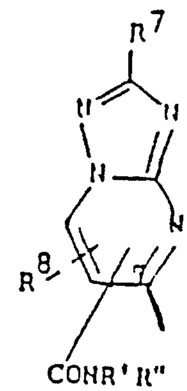

(a1) (b1) (c1) (d1)

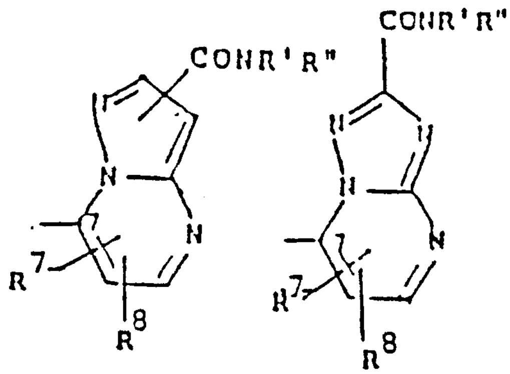
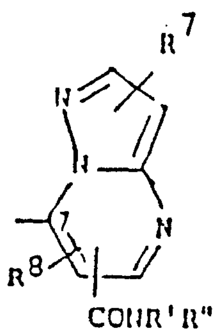
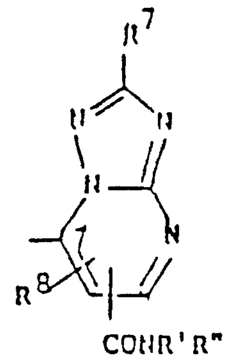

(a2) (b2) (c2) (d2)

wherein R', R", $R^7$ and $R^8$ have the significance given in claim 1,

which process is characterized in that correspondingly substituted starting materials are employed.

7. Process for the manufacture of compounds according to claim 5, wherein $R^3$ represents one of the groups

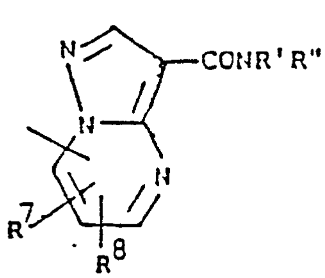

(a3)

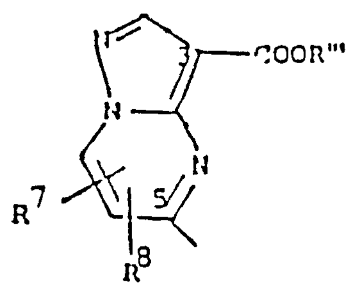

(e3)

wherein R', R", $R^7$ and $R^8$ have the significance given in claim 1,

which process is characterized in that correspondingly substituted starting materials are employed.

8. Process for the manufacture of compounds according to claim 5, wherein $R^3$ signifies a carbamoyl-substituted bicyclic group of the general formulae

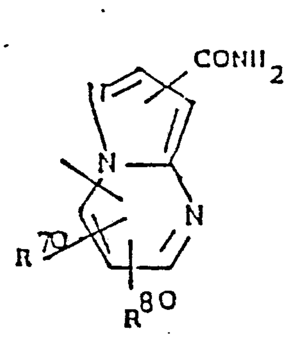

(a⁴)

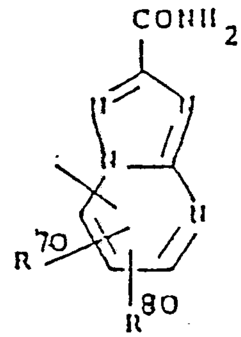

(b⁴)

wherein $R^{70}$ and $R^{80}$ each individually represent hydrogen, methyl or trifluoromethyl or together represent alkylene with 3 or 4 carbon atoms,

which process is characterized in that correspondingly substituted starting materials are employed.

9. Process for the manufacture of compounds according to claim 5, wherein $R^3$ signifies a substituted bicyclic group of the general formulae

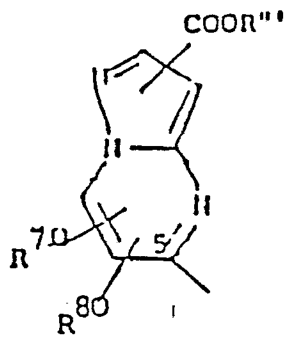

(e⁴)

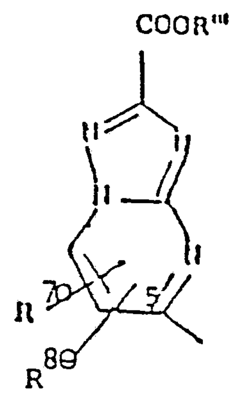

(f⁴)

wherein $R^{70}$ and $R^{80}$ each individually represent hydrogen, methyl or trifluoromethyl or together represent alkylene with 3 or 4 carbon atoms and R‴ signifies hydrogen or methyl,

which process is characterized in that correspondingly substituted starting materials are employed.

10. Process for the manufacture of compounds according to claim 5, wherein $R^3$ represents one of the groups

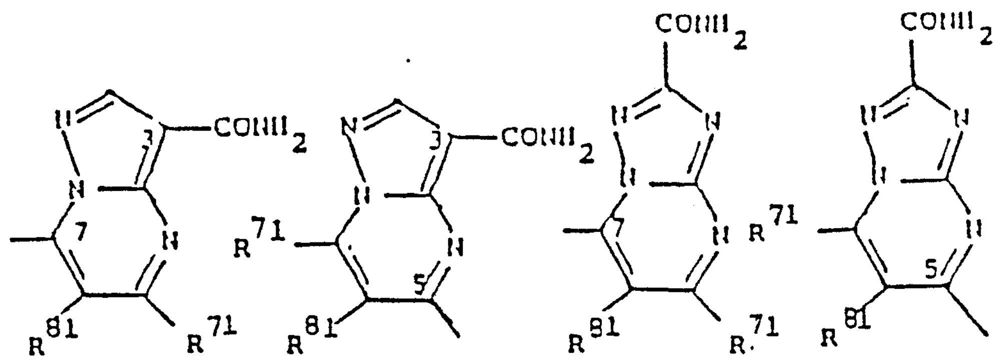

(a⁵) (a⁶) (b⁵) (b⁶)

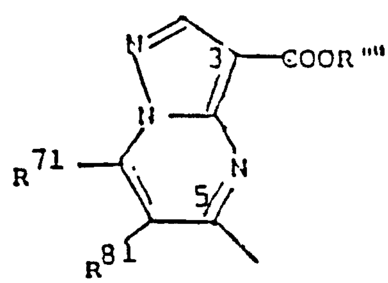

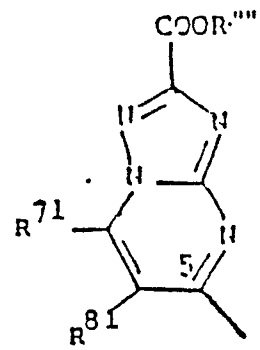

(e⁵) (f⁵)

wherein $R^{71}$ represents methyl or trifluoromethyl and $R^{81}$ represents hydrogen or $R^{71}$ and $R^{81}$ together represent tetramethylene and R'''' signifies hydrogen or methyl,

which process is characterized in that correspondingly substituted starting materials are employed.

11. Process for the manufacture of compounds according to claim 8, wherein $R^3$ represents group ($a^4$) which is linked via position 5:

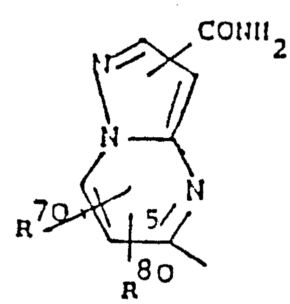

which process is characterized in that correspondingly substituted starting materials are employed.

12. Process for the manufacture of compounds according to claim 8, wherein $R^3$ represents group ($a^4$) which is linked via position 7:

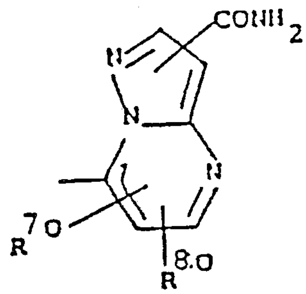

which process is characterized in that correspondingly substituted starting materials are employed.

13. Process for the manufacture of compounds according to any one of claims 8, 11 and 12, wherein $R^3$ represents group ($a^4$) in which the carbamoyl group is present in position 3:

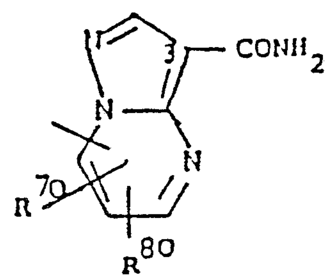

which process is characterized in that correspondingly substituted starting materials are employed.

14. Process for the manufacture of compounds according to claim 12 or 13, wherein $R^3$ represents the group

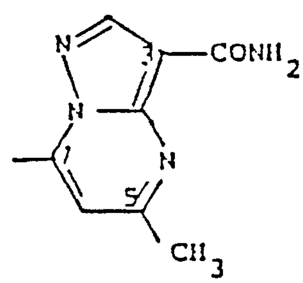

($a^7$)

which process is characterized in that correspondingly substituted starting materials are employed.

15. Process for the manufacture of compounds according to claim 8, wherein $R^3$ represents group ($b^4$) which is linked via position 5:

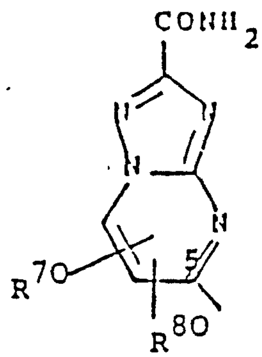

which process is characterized in that correspondingly substituted starting materials are employed.

16. Process for the manufacture of compounds according to claim 8, wherein $R^3$ represents group ($b^4$) which is linked via position 7:

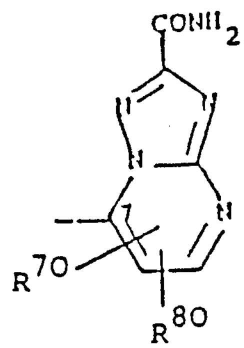

which process is characterized in that correspondingly substituted starting materials are employed.

17. Process for the manufacture of compounds according to claim 15 or 16, wherein $R^3$ represents the group

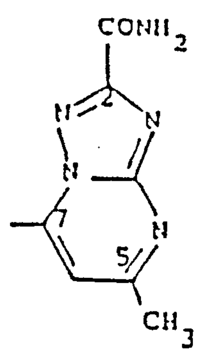

($b^7$)

which process is characterized in that correspondingly substituted starting materials are employed.

18. Process according to claim 5 for the manufacture of (6R,7R)-7-[(Z)-2-(2-amino-4-thiazolyl)-2-[[1-[3-(3,4-dihydrobenzoyl)carbazoyl]-1-methylethoxy]imino]acetamido]-3-[[(2-carbamoyl-5-methyl-s-triazolo[1,5-a]-pyrimidin-7-yl)thio]methyl]-8-oxo-5-thia-1-azabicyclo[4.2.0]oct-2-ene-2-carboxylic acid and its salts, which process is characterized in that correspondingly substituted starting materials are employed.

19. Process according to claim 5 for the manufacture of (6R,7R)-7-[(Z)-2-(2-amino-4-thiazolyl)-2-[[1-[3-(3,4-dihydroxybenzoyl)carbazoyl]-1-methylethoxy]imino]acetamido]-3-[[(3-carbamoyl-5-methylpyrazolo[1,5-a]pyrimidin-7-yl)thio]methyl]-8-oxo-5-thia-1-azabicyclo[4.2.0]oct-2-ene-2-carboxylic acid and its salts, which process is characterized in that correspondingly substituted starting materials are employed.

20. Process according to claim 5 for the manufacture of (6R,7R)-7-[(Z)-2-(2-amino-4-thiazolyl)-2-[[1-[3-(3,4-dihydroxybenzoyl)carbazoyl]-1-methylethoxy]imino]acetamido]-3-[[(2-carbamoyl-6,7-dihydro-5H-cyclopenta[d]-s-triazolo[1,5-a]pyrimidin-8-yl)thio]methyl]-8-oxo-5-thia-1-azabicyclo[4.2.0]oct-2-ene-2-carboxylic acid and its salts, which process is characterized in that correspondingly substituted starting materials are employed.

21. Process according to claim 5 for the manufacture of (6R,7R)-7-[(Z)-2-(2-amino-4-thiazolyl)-2-[[1-[3-(3,4-dihydroxybenzoyl)carbazoyl]-1-methylethoxy]imino]acetamido]-3-[[(2-carbamoyl-7-methyl-s-triazolo[1,5-a]pyrimidin-5-yl)thio]methyl]-8-oxo-5-thia-1-azabicyclo[4.2.0]oct-2-ene-2-carboxylic acid and its salts, which process is characterized in that correspondingly substituted starting materials are employed.

22. Process according to claim 5 for the manufacture of (6R,7R)-7-[(Z)-2-(2-amino-4-thiazolyl)-2-[[1-[3-(3,4-dihydroxybenzoyl)carbazoyl]-1-methylethoxy]imino]acetamido]-3-[[[3-carbamoyl-7-(trifluoromethyl)-pyrazolo[1,5-a]pyrimidin-5-yl]thio]methyl]-8-oxo-5-thia-1-azabicyclo[4.2.0]oct-2-ene-2-carboxylic acid and its salts, which process is characterized in that correspondingly substituted starting materials are employed.

23. Process according to claim 5 for the manufacture of (6R,7R)-7-[(Z)-2-(2-amino-4-thiazolyl)-2-[[1-[3-(3,4-dihydroxybenzoyl)carbazoyl]-1-methylethoxy]imino]acetamido]-3-[[[3-carbamoyl-5-(trifluoromethyl)-pyrazolo[1,5-a]pyrimidin-7-yl]thio]methyl]-8-oxo-5-thia-1-azabicyclo[4.2.0]oct-2-ene-2-carboxylic acid and its salts, which process is characterized in that correspondingly substituted starting materials are employed.

24. Process according to claim 5 for the manufacture of (6R,7R)-7-[(Z)-2-(2-amino-4-thiazolyl)-2-[[1-[3-(2,3-dihydroxybenzoyl)carbazoyl]-1-methylethoxy]imino]acetamido]-3-[[(2-carbamoyl-5-methyl-s-triazolo[1,5-a]pyrimidin-7-yl)-thio]methyl]-8-oxo-5-thia-1-azabicyclo[4.2.0]oct-2-ene-2-carboxylic acid and its salts, which process is characterized in that correspondingly substituted starting materials are employed.

25. Process according to claim 5 for the manufacture of

(6R,7R)-7-[(Z)-2-(2-amino-4-thiazolyl)-2-[[1-[3-(3,4-dihydroxybenzoyl)carbazoyl]-1-methylethoxy]imino]-acetamido]-3-[[(2-carboxy-7-methyl-s-triazolo[1,5-a]pyrimidin-5-yl)thio]-methyl]-8-oxo-5-thia-1-azabicyclo-[4.2.0]oct-2-ene-2-carboxylic acid,

(6R,7R)-7-[(Z)-2-(2-amino-4-thiazolyl)-2-[[1-[3-(3,4-dihydroxybenzoyl)carbazoyl]-1-methylethoxy]imino]-acetamido]-3-[[(2-methoxycarbonyl-7-methyl-s-triazolo[1,5-a]pyrimidin-5-yl)thio]-methyl]-8-oxo-5-thia-1-azabicyclo-[4.2.0]oct-2-ene-2-carboxylic acid,

(6R,7R)-7-[(Z)-2-(2-amino-4-thiazolyl)-2-[[1-[3-(3,4-dihydroxybenzoyl)carbazoyl]-1-methylethoxy]imino]-acetamido]-3-[[[2-(cyclopropylcarbamoyl)-7-methyl-s-triazolo[1,5-a]-pyrimidin-5-yl]thio]methyl]-8-oxo-5-thia-1-azabicyclo[4.2.0]-oct-2-ene-2-carboxylic acid,

(6R,7R)-7-[(Z)-2-(2-amino-4-thiazolyl)-2-[[1-[3-(3,4-dihydroxybenzoyl)carbazoyl]-1-methylethoxy]imino]-acetamido]-3-[[[3-(methoxycarbonyl)-7-methylpyrazolo[1,5-a]pyrimidin-5-yl]thio]methyl]-8-oxo-5-thia-1-azabicyclo[4.2.0]oct-2-ene-2-carboxylic acid,

(6R,7R)-7-[[[(Z)-2-(2-amino-4-thiazolyl)-2-[1-[3-(3,4-dihydroxybenzoyl)carbazoyl]-1-methylethoxy]imino]-acetamido]-3-[[[3-carboxy-7-(trifluoromethyl)pyrazolo[1,5-a]-pyrimidin-5-yl]thio]methyl]-8-oxo-5-thia-1-azabicyclo[4.2.0]-oct-2-ene-2-carboxylic acid

and salts of any of these compounds, which process is characterized in that correspondingly substituted starting materials are employed.

26. Process according to claim 5 for the manufacture of (6R,7R)-7-[(Z)-2-(2-amino-4-thiazolyl)-2-[[1-[3-(3,4-dihydroxybenzoyl)carbazoyl]-1-methylethoxy]imino]acetamido]-3-[[[5-methyl-2-(methylcarbamoyl)-s-triazolo[1,5-a]pyrimidin-7-yl]thio]methyl]-8-oxo-5-thia-1-azabicyclo[4.2.0]oct-2-ene-2-carboxylic acid and its salts, which process is characterized in that correspondingly substituted starting materials are employed.

27. Process according to claim 5 for the manufacture of (6R,7R)-7-[(Z)-2-(2-amino-4-thiazolyl)-2-[[1-[3-(3,4-dihydroxybenzoyl)carbazoyl]-1-methylethoxy]imino]acetamido]-3-[[[2-(dimethylcarbamoyl)-5-methyl-s-triazolo[1,5-a]-pyrimidin-7-yl]thio]methyl]-8-oxo-5-thia-1-azabicyclo[4.2.0]-oct-2-ene-2-carboxylic acid and its salts, which process is characterized in that correspondingly substituted starting materials are employed.

28. Process according to claim 5 for the manufacture of (6R,7R)-7-[(Z)-2-(2-amino-4-thiazolyl)-2-[[1-[3-(3,4-dihydroxybenzoyl)carbazoyl]-1-methylethoxy]imino]acetamido]-3-[[[2-carbamoyl-5-(trifluoromethyl)-s-triazolo[1,5-a]pyrimidin-7-yl]thio]methyl]-8-oxo-5-thia-1-azabicyclo[4.2.0]oct-2-ene-2-carboxylic acid and its salts, which process is characterized in that correspondingly substituted starting materials are employed.

29. Process according to claim 5 for the manufacture of (6R,7R)-7-[(Z)-2-[2-amino-4-thiazolyl)-2-[[1-[3-(3,4-dihydroxybenzoyl)carbazoyl]-1-methylethoxy]imino]acetamido]-3-[[[2-carbamoyl-7-(trifluoromethyl)-s-triazolo[1,5-a]pyrimidin-5-yl]thio]methyl]-8-oxo-5-thia-1-azabicyclo[4.2.0]oct-2-ene-2-carboxylic acid and its salts, which process is characterized in that correspondingly substituted starting materials are employed.

30. Process according to any one of claims 1-4 for the manufacture of

(6R,7R)-7-[(Z)-2-(2-amino-4-thiazolyl)-2-[[1-(3-(3,4-dihydroxybenzoyl)carbazoyl]-1-methylethoxy]imino]-acetamido]-3-[[[2-carboxy-7,8-dihydro-6H-cyclopenta[e][1,2,4]triazolo[1,5-a]pyrimidin-5-yl]thio]methyl]-8-oxo-

5-thia-1-azabicyclo[4.2.0]oct-2-ene-2-carboxylic acid and its salts.

(6R,7R)-7-[(Z)-2-(2-amino-4-thiazolyl)-2-[[1-[3-(3,4-dihydroxybenzoyl)carbazoyl]-1-methylethoxy]imino[-acetamido]-3-[[[2-carbamoyl-7,8-dihydro-6H-cyclopenta[e][1,2,4]triazolo[1,5-a]pyrimidin-5-yl]thio]methyl]-8-oxo-5-thia-1-azabicyclo[4.2.0]oct-2-ene-2-carboxylic acid.

(6R,7R)-7-[(Z)-2-(2-amino-4-thiazolyl)-2-[[1-(3-[3,4-dihydroxybenzoyl)carbazoyl]-1-methylethoxy]imino]-acetamido]-3-[[[2-carboxy-7-(trifluormethyl)-s-triazolo[1,5-a]pyrimidin-5-yl]thio]methyl]-8-oxo-5-thia-1-azabicyclo[4.2.0]oct-2-ene-2-carboxylic acid,

(6R,7R)-7-[(Z)-2-(2-amino-4-thiazolyl)-2-[[1-[3-(3,4-diacetoxybenzoyl)carbazoyl]-1-methylethoxy]imino]-acetamido]-3-[[(3-carbamoyl-6,7-dihydro-5H-cyclopenta[d]pyrazolo[1,5-a]pyrimidin-8-yl)thio]methyl]-8-oxo-5-thia-1-azabicyclo[4.2.0]oct-2-ene-2-carboxylic acid,

(6R,7R)-7-[(Z)-2-(2-amino-4-thiazolyl)-2-[[1-[3-(3,4-dihydroxybenzoyl)carbazoyl]-1-methylethoxy]imino]-acetamido]-3-[[(3-carbamoyl-6,7-dihydro-5H-cyclopenta[d]pyrazolo[1,5-a]pyrimidin-8-yl)thio]methyl]-8-oxo-5-thia-1-azabicyclo[4.2.0]oct-2-ene-2-carboxylic acid,

(6R,7R)-7-[(Z)-2-(2-amino-4-thiazolyl)-2-[[1-[3-(3,4-diacetoxybenzoyl)carbazoyl]-1-methylethoxy]imino]-acetamido]-3-[[(3-carbamoyl-5-methyl-pyrazolo[1,5-a]pyrimidin-7-yl)thio]methyl]-8-oxo-5-thia-1-azabicyclo-[4.2.0]oct-2-ene-2-carboxylic acid,

(6R,7R)-7-[(Z)-2-(2-amino-4-thiazolyl)-2-[[1-[3-(3,4-diacetoxybenzoyl)carbazoyl]-1-methylethoxy]imino] acetamido]-3-[[[3-(dimethylcarbamoyl)-5-methyl-pyrazolo[1,5-a]pyrimidin-7-yl]thio]methyl-8-oxo-5-thia-1-azabicyclo[4.2.0]-oct-2-ene-2-carboxylic acid,

(6R,7R)-7-[(Z)-2-(2-amino-4-thiazolyl)-2-[[1-[3-(3,4-dihydroxybenzoyl)carbazoyl]-1-methylethoxy]imino]-acetamido]-3-[[[3-(dimethylcarbamoyl)-5-methyl-pyrazolo[1,5-a]pyrimidin-7-yl]thio]methyl]-8-oxo-5-thia-1-azabicyclo[4.2.0]-oct-2-ene-2-carboxylic acid
and salts of any of these compounds, which process is characterized in that correspondingly substituted starting materials are employed.

31. Process according to any one of claims 1-4 for the manufacture of (6R,7R)-7-[(Z)-2-(2-amino-4-thiazolyl)-2-[[1-[3(3-chloro-4,5-dihydroxybenzoyl)carbazoyl]-1-methylethoxy]imino]acetamido]-3-[[(2-carbamoyl-5-methyl-s-triazolo[1,5-a]pyrimidin-7-yl)thio]methyl]-8-oxo-5-thia-1-azabicyclo[4.2.0]oct-2-ene-2-carboxylic acid and its salts, which process is characterized in that correspondingly substituted starting materials are employed.

32. A process for the manufacture of pharmaceutical preparations. which process comprises mixing an end product as defined in claim 1 as active ingredient with non-toxic, inert, therapeutically compatible solid or liquid carriers and/or excipients commonly used in such preparations.

33. A pharmaceutical preparation containing an end product according to any one of claims 1-31.

34. A pharmaceutical preparation for the treatment and prophylaxis of infectious diseases containing an end product according to any one of claims 1-31.

35. The use of the end products according to any one of claims 1-31 in the treatment and prophylaxis of illnesses.

36. The use of the end products according to any one of claims 1-31 in the treatment and prophylaxis of infectious diseases.

37. The use of the end products according to any one of claims 1-31 for the manufacture of medicaments for the treatment and prophylaxis of infectious diseases.

38. Compounds according to any one of claims 1-31 whenever prepared according to the process claimed in any one of claims 1-31 or by an obvious chemical equivalent thereof.